(19) **Europäisches Patentamt European Patent Office Office européen des brevets**

(11) **EP 4 744 486 A1**

(12) **EUROPEAN PATENT APPLICATION**

(43) Date of publication:
20.05.2026 Bulletin 2026/21

(21) Application number: 24461635.5

(22) Date of filing: 18.11.2024

(51) International Patent Classification (IPC):
*A01N 1/122* (2025.01)    *A01N 1/126* (2025.01)
*A01P 1/00* (2006.01)    *A61K 9/00* (2006.01)
*A61K 9/107* (2006.01)    *A61K 47/26* (2006.01)
*A61K 49/00* (2006.01)    *C12N 5/00* (2006.01)

(52) Cooperative Patent Classification (CPC):
(C-Sets available)
**A01N 1/126; A01P 1/00; C12N 5/522;** A61K 9/0019;
A61K 9/1075                                (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **NanoSanguis S.A.**
**02-532 Warszawa (PL)**

(72) Inventors:
• **GRAFFSTEIN, Joanna**
**04-780 Warszawa (PL)**
• **FRACKOWIAK, Wojciech**
**03-982 Warszawa (PL)**
• **CZARNOCKA-SNIADALA, Sylwia**
**05-816 Michalowice (PL)**
• **ZUK, Pawel**
**01-318 Warszawa (PL)**
• **ZAKRZEWSKA, Julia**
**05-530 Krepa (PL)**
• **KOPEC , Daniel**
**21-400  Luków (PL)**
• **PRYSTUPIUK, Kornel**
**02-972 Warszawa (PL)**

• **SUTKOWSKI, Marek**
**05-807 Podkowa Lesna (PL)**
• **SZELAG, Daria**
**01-452 Warszawa (PL)**
• **STRUGA, Marta**
**05-822 Milanówek (PL)**
• **SKRZYCKI, Michal**
**05-600 Grójec (PL)**
• **KOSIERADZKI, Maciej**
**03-287 Warszawa (PL)**
• **DOMAGALA, Piotr**
**00-870 Warszawa (PL)**
• **PODGÓRSKI, Rafa**
**05-075 Warszawa (PL)**
• **PIETRZAK, Rafa**
**04-859 Warszawa (PL)**
• **CIACH, Tomasz**
**02-765 Warszawa (PL)**
• **STEFANEK, Agata**
**05-420 Józefów (PL)**

(74) Representative: **Augustyniak, Magdalena Anna et al**
**Polservice**
**Kancelaria Rzecznikow**
**Patentowych sp. z o.o.**
**Bobrowiecka 8**
**00-728 Warszawa (PL)**

(54) **PERFUSION FLUID, METHODS OF PRODUCING PERFUSION FLUID, AND ITS USES.**

(57)    The subject of the present invention is a perfusion fluid in the form of an oil-in-water PFC nonoemulsion, methods of producing this perfusion fluid, and its use in perfusion of organs.

EP 4 744 486 A1

(52)  Cooperative Patent Classification (CPC): (Cont.)

C-Sets
**A01N 1/126, A01N 25/04, A01N 25/30**

## Description

### Field of invention

The subject of the present invention is a perfusion fluid in the form of an oil-in-water PFC nanoemulsions, methods of production of this perfusion fluid, and its applications in organ perfusion.

### State of the art

In Poland in 2023, 977 kidney transplants, 523 liver transplants and 178 heart transplants were performed, and the waiting list for a vascularised organ transplant averages 1,903 patients per year in Poland (Statistics 2023. https://www.poltransplant.org.pl/statystyka_2023.html). It is estimated that up to 25 per cent of patients die each year while waiting for a vascular organ transplant, while a second as many are taken off the list due to a disqualifying medical condition (Committee on A Fairer and More Equitable, Cost-Effective, and Transparent System of Donor Organ Procurement, Allocation, and Distribution. Realizing the Promise of Equity in the Organ Transplantation System. 26364 (National Academies Press, Washington, D.C., 2022). doi:10.17226/26364), and patients waiting for a kidney transplant through the use of dialysis systems account for up to 7% of the total cost of the entire health care budget (Himmelfarb, J., Vanholder, R., Mehrotra, R. & Tonelli, M. The current and future landscape of dialysis. Nat. Rev. Nephrol. 16, 573-585 (2020)).

The growing problem of too few donors can be addressed by allowing organs from currently excluded donors after irreversible cardiac arrest, i.e. donation after circulatory death (DCD) and donors qualified as expanded criteria donors (ECD), to be transplanted. The ECD category includes donors aged 60 years and older and those aged 50-59 years with at least two of the following risk factors: hypertension, death due to stroke, blood creatinine level at the moment of death above 1.5 mg/dL.

The risk of rejection of a kidney transplant taken from an ECD-qualified donor is 70% higher than that from a healthy, non-ECD and DCD donor (Rao, P. S. & Ojo, A. The Alphabet Soup of Kidney Transplantation: SCD, DCD, ECD-Fundamentals for the Practicing Nephrologist. Clin. J. Am. Soc. Nephrol. 4, 1827 (2009)).

DCD kidney recipients due to ischaemic damage require dialysis much more frequently and for a longer time after kidney transplantation due to delayed graft function and have a markedly reduced distant organ survival time.

The solution to increase the availability of organs from ECD and DCD donors is to use methods that allow organ procurement and storage, which would result in an extended shelf life. Storage is a critical period, the only one in which interventions are possible to reduce the impact of ischaemic damage that has already been initiated.

The first methods of storing kidneys involved simple 'dry' storage - i.e. placing them in polystyrene containers surrounded by dry ice. Hypothermia, by lowering the rate of chemical reactions taking place, only slows down the organ's death process. This method is insufficient to maintain adequate blood supply to the organ.

The solution to this problem is to maintain fluid circulation to provide nutrients and protect the organ from further degradation. The first such attempts at organ preservation using the static cold storage (SCS) method, using a dedicated fluid, a Collins formulation based on electrolytes with a high potassium and low sodium content, took place in 1969 (Wesson, L. G., Colberg, J. E., deGuzman, A., Elsasser, W. & Dunn, S. Extracellular fluid of the kidney preserved by the Collins technique. Transplantation 27, 380-383 (1979)).

Continuing to maintain, or even improve, the condition of the organ for transplantation as a result of the activation of repair processes requires raising the temperature of the organ with the simultaneous supply of oxygen and other substrates for active metabolism. Normothermic perfusion with blood at 37°C has been shown to improve transplantation outcomes compared to simple hypothermia in a large animal model (pig). Already when kidneys were stored at 20°C (subnormothermia) after reperfusion with blood, improvements in flow, urine excretion and creatinine clearance were observed.

In relation to this rationale, increasing the pool of potential organs eligible for transplantation, through the use of mechanical kidney perfusion methods under subnormothermic and normothermic conditions, which could realistically increase the number of transplants performed, reduce the duration of dialysis use and reduce the number of deaths of patients awaiting transplantation, seems clear.

A solution to this problem may be to conduct the perfusion process using an acellular fluid containing synthetic oxygen nanocarriers consisting of a perfluoroarbon nanoemulsions. This fluid should provide adequate oxygenation and essential nutrients to the organ awaiting transplantation. Perfluorocarrier-based perfusion fluids will also have significant advantages over products based on blood morphotic elements, as they avoid phenomena such as the release of leukotrienes from them, as well as other inflammatory and vasoconstrictive substances.

Perfluorinated compounds (PFCs) are substances in which the hydrogen atoms are completely substituted with fluorine atoms and sometimes with other additional halogens (e.g. bromine). This substitution radically changes the physical properties of these organic compounds. The carbon-fluorine bond is very strong (~484 kJ/mol) and extremely polar (almost ionic), which gives these compounds very high thermal and chemical stability. However, this does not result in water solubility, as the internal symmetry removes the polarity of each C-F bond and the entire PFC molecule becomes non-polar. These compounds also have twice the density and about 50 times the gas solubility of water. For example: perfluorooctyl bromide (PFOB) can dissolve 527 mLO$_2$/L PFOB at a pressure of 1 atm. Carbon dioxide, on the other hand, can dissolve up to 4 times more than oxygen. For comparison, the solubility of O$_2$ in water is about 9-10 mLO$_2$/L water, and in blood about 200 mLO$_2$/L blood. The presence of fluorine atoms affects the shape and size of the PFC molecule, which needs more space between the H$_2$O molecules, increasing the hydration energy. This effect reduces the solubility of the PFC in water compared to the corresponding hydrocarbon derivative. On the other hand, this extreme polarity inhibits the formation of induced dipoles, which would lead to the van der Waals forces necessary to obtain solubility in lipids. Therefore, PFCs are both hydrophobic and lipophobic compounds.

Due to their unique physico-chemical properties, perfluorinated compounds and their derivatives have a wide range of industrial applications. In medicine and pharmaceutics, research is being conducted into their use mainly as PFC-based oxygen carriers (PFOCs) in blood substitutes and organ perfusion fluids, contrast agents and as excipients in anticancer therapies. The extreme hydrophobicity of PFCs makes it necessary to use emulsifiers such as lipids, partially fluorinated compounds, proteins or other surfactants to combine with aqueous media, e.g. blood, organ perfusion fluids. These preparations are usually in the form of an emulsion, i.e. a mixture of two immiscible phases (in this case water and perfluorinated compounds). The formation of such kinetically stable systems is made possible by the use of surface tension-reducing compounds and high-pressure emulsification methods. The oxygenation of PFC preparations results in the dissolving of large amounts of O$_2$ in the perfluorinated core of the emulsion particles. The emulsifier present in the envelope does not interfere with this process. If such a formulation, is then exposed to a lower oxygen environment (e.g. body tissues), oxygen diffuses out of the mixture.

WO2024046999A1 discloses compositions for the production of stable synthetic oxygen carriers based on perfluorocarbons, methods for their manufacture and use as synthetic blood-oxygen carriers and as volume substitutes. The manufacture of perfluorocarbon-based synthetic oxygen carriers (PFOCs) disclosed herein comprises the following steps: (a) preparation of a suitable aqueous albumin solution and mixing it with at least one suitable perfluorocarbon-based artificial oxygen carrier, (b) suitable addition of lecithin, (c) pre-emulsification by rapid mixing with suitable cooling, (d) emulsification of the pre-emulsion from step (c) under pressure using a high-pressure homogenizer with suitable cooling, and storage for at least 30 minutes, also with suitable cooling. Albumin used was from among the mammalian albumin group, human serum albumin (HSA) and bovine serum albumin (BSA), or suitable derivatives such as pegylated albumin. Albumin was prepared in: a suitable aqueous buffer, water or balanced electrolyte solution for infusion therapy, such as Sterofundin® ISO, Ringerfundin®, Ringer solution, STEEN solution, OCS solution and hydroxyethyl starch solution. The addition of lecithin as a stabiliser was used in the preparation of emulsions.

WO200877641 A1 discloses nanoemulsions comprising at least one aqueous component and a carrier, the carrier comprising at least one lipophilic component, at least one surfactant and at least one alcohol. The method of preparing an emulsion comprises the steps of (a) preparing an aqueous component, (b) preparing a carrier comprising at least one lipophilic component, at least one surfactant and at least one alcohol, the at least one alcohol having at least three carbon atoms, and (c) mixing the aqueous component of step (a) with the carrier of step (b). The document also discloses a composition comprising mentioned nanoemulsion and mentioned active substance. In particular, the composition is in the form of a gel and the active substance is 5-aminolevulinic acid (ALA), a derivative, precursor and/or metabolite thereof. The invention also relates to the preparation of mentioned nanoemulsion and/or composition and to their use in the treatment of dermatological diseases, viral diseases, and diseases associated with cell proliferation, in particular cancer and/or psoriasis, and to the use of mentioned nanoemulsion in cosmetics.

Document US11406722 B2 discloses a method of making a liquid perfluorocarbon (PFC) nanodroplet composition, the method comprising the following steps: combining the PFC liquid with a surfactant and a cosurfactant to produce a liquid composition; and emulsifying mentioned PFC liquid with mentioned surfactant and mentioned cosurfactant within mentioned liquid composition by direct microfluidization at a pressure of at least 3,000 psi to reduce the size of the PFC droplets within mentioned liquid composition to form a nanodroplet composition with an average particle diameter of less than 300 nm, the PFC having a boiling point of less than about 0°C. Mentioned surfactant comprises an amphiphilic phospholipid-based compound, wherein mentioned cosurfactant comprises a semi-fluorinated alkane and wherein emulsification is carried out at a temperature below 0°C. The document discloses compositions having an average particle diameter of less than about 250 nm, for example less than about 200 nm or less than about 150 nm. The composition disclosed in document US11406722 B2 shows less than 10 % change in average particle diameter over a period of 1 week at 4°C, or less than 10 % change in average particle diameter over a period of 1 hour at 37°C. The invention has applications in the field of medical diagnostics and therapeutics, where the stabilised nanodroplets are used in ultrasound imaging and potentially in therapeutics, e.g. by forming microbubbles under ultrasound.

In document KR20230124148, there is disclosed a composition for inhibiting fat accumulation using a nanoemulsion containing a perfluorocarbon, a surfactant and an auxiliary surfactant dispersed in a continuous phase. The document discloses the effective delivery of perfluorocarbon to cells, tissues or body tissue causing an inhibitory effect on fat accumulation. The paper describes studies on nanoemulsion particle size under different conditions. Compositions containing EggPC or Span80 with Tween80 as an auxiliary surfactant were found to form stable emulsions with small particles (100-200 nm). However, the addition of Poloxamer188 as an auxiliary surfactant negatively affected the stability of the emulsions, causing an increase in particle size above 1000 nm and instability with changes in temperature and time.

In WO2020209752 A1, there is disclosed an emulsion comprising the perfluorocarbon compound perfluorodecalin, a perfluorocarbon additive and phospholipids in the form of liposomes (vesicles) prepared by homogenization under pressure in an aqueous saline medium. The perfluorocarbon additive is a mixture of perfluorinated products: perfluorooctyl bromide and perfluorotripropylamine, and the liposomes (vesicles) contain natural oils as an excipient. The method of making the emulsion comprises producing the liposomes (vesicles) by homogenization at a pressure of 60-120 atm in an aqueous saline medium, followed by hot sterilization, homogenizing the mentioned perfluorocarbon compounds under pressure into a liposomal form (vesicles) of phospholipids and performing hot sterilization of the prepared emulsion. The emulsion is intended to improve blood oxygen supply during the treatment of hypoxic conditions and to preserve isolated perfused organs and tissues. The stability of the emulsion is increased while the particle structure is preserved, thus improving the quality of the emulsion and maintaining biocompatibility with the biological medium (blood, plasma, serum, blood substitutes). The shelf life of the emulsion when thawed at +4°C is a minimum of 23 months, while maintaining the emulsion structure.

Document WO201356246 A1, discloses an oxygen therapeutic composition comprising a perfluorocarbon material, a viscosity modifier, a buffer, the buffer stabilising the pH of the composition at from about 6.5 to about 7.5, the composition having a viscosity of from about 2.0 to about 3.5 mPa·s and the fluorocarbon having a boiling point of from about 4°C to about 60°C. The paper indicates that DDFPe absorbs more oxygen at room temperature than other agents including perfluorodecalin and perfluorooctylbromide and is even more effective at physiological temperature (above the boiling point of DDFP). The paper discloses that DDFPe forms an emulsion in water consisting of submicron-sized droplets at room temperature and converts to a gas at 29°C. In order to maintain the stability of the DDFPe emulsion, a viscosity modifying material was used which prevents particle settling and agglomeration. The paper indicates that sucrose is the preferred viscosity modifying material.

Document CN106176601A discloses a perfluorocarbon emulsion and a method of making and using it, in particular to improve oxygen content in tumours. The perfluorocarbon emulsion is prepared from perfluorocarbons and proteins preferably bovine serum albumin or human albumin. Such an emulsion has a particle size of 180-200 nm and has a high oxygen carrying capacity and can rapidly release the contained oxygen under ultrasound. In the method of producing the emulsion, the protein is dissolved in phosphate buffer, then perfluorocarbon liquid is added, and the resulting solution is sonicated by ultrasound, the bottom layer obtained is centrifuged, then dissolved in phosphate buffer. The experiment showed that the oxygen carried by the perfluorocarbon emulsion could be rapidly released under the mechanical action of ultrasound, and the oxygen content at the tumour site increased steadily. With this method, it is possible to effectively alleviate oxygen deficiency at the tumour site, which translates into improved treatment effects with photodynamic therapy and radiotherapy.

Document WO2010/121082 discloses an emulsion comprising a perfluorocarbon dispersed as particles in a liquid phase, the dispersed particles having a monomodal particle size distribution. According to the disclosure, the perfluorocarbon may be perfluoro (tert-butylhexane), perfluorodecalin, perfluoroisopropyldecalin, perfluorotripropylamine, perfluorotributylamine, perfluoromethylcyclohexylpiperidine, perfluorooctylbromide, perfluorodecylbromide, perfluorodichlorohexane, perfluorohexane, dodekafluoropentane or mixtures thereof. The emulsion additionally contains an emulsifier, which is a phospholipid from egg yolk. In addition, the emulsion contains an aqueous carrier buffered to pH 6.8-7.4. The method of making the emulsion comprises the following steps: mixing the emulsifying agent with the aqueous medium (step a), adding a perfluorocarbon to this mixture (step b), mixing this mixture to form a coarse emulsion (step c), obtaining a sample of the emulsion and determining the particle size distribution (step d), if the particle size distribution is monomodal, homogenization of the emulsion is carried out (step e), obtaining the final emulsion (step f). The document indicates the usefulness of the emulsion in the treatment of sickle cell anaemia, decompression sickness, air embolism or carbon monoxide poisoning in a patient by administering the described emulsion in an amount effective to treat these conditions. The document also discloses the use of the emulsion in a method of preserving an organ prior to transplantation, which involves contacting the organ with the described emulsion to increase the organ's survival time. In one variant, the organ is also perfused with the emulsion.

Document WO2015/147705 discloses a method for producing a stable, sterile nanoemulsion of perfluoroorganic compounds (PFOC) with an average particle size of 150 nm or less, preferentially 30-100 nm, most preferably 30 to 80 nm. The manufacturing method comprises filling the circuit in the installation for producing the PFOC nanoemulsion with an aqueous solution of a stabilizing additive, preferably a poloxamer, adding the PFOC mixture to the aqueous solution of the stabilizing additive and then homogenizing the obtained PFOC mixture in the aqueous solution of the stabilizing additive to obtain the PFOC pre-emulsion of the required particle size, under thermostatic control of the operation of the homogenizer working chamber. The PFOC pre-emulsion is then added to an aqueous salt solution to produce a PFOC nanoemulsion with the required concentration of PFOC, stabilising additive and salt. The PFOC nanoemulsions are stored at 2-10°C, with optional pre-packaging of the finished product into sterile consumer packaging and freezing of the finished product.

According to the disclosure in the document, two types of perfluoroorganic compounds are typically used for PFOC emulsions. One is chosen from the C8-C10 group, comprising, for example, perfluorodecalin (PFD) or perfluorooctylbromide (PFOB), while the other is chosen from the C11-C12 group, comprising, for example, perfluorotripropylamine (PFTPA), perfluoromethylcyclohexylpiperidine (PFMCP) and perfluorotributylamine (PFTBA). Compounds of the first type are rapidly (within a month) removed from the body, but do not provide sufficient emulsion stability. Compounds of the second type, on the other hand, have a high emulsion stability that allows them to be stored without freezing, but can persist in the body for long periods of time (8 months to 2 years). It is also possible to use mixtures of two PFOC compounds, such as FPD/PFTBA, PFD/PFMCP, PFOB/PFTBA, PFOB/PFMCP, or three compounds, for example PFOB/PFD/PFMCP, PFOB/PFD/PFTBA, or even four, such as FPD/PFOB/PFMCP/PFTBA. Special stabilisers are added to the emulsion to reduce the average particle diameter of the emulsion, increase dispersion and stability for long-term storage. Non-toxic high-molecular-weight non-ionic surfactants (non-ionic surfactants), in particular poloxamers (proxanols, Pluronics, Kolliphor), are preferred as stabilisers. Their amount in PFOC emulsions is kept to a minimum, resulting only from the need to ensure satisfactory emulsification and homogenization of PFOC. Minimising the amount of surfactants is important because they affect the toxicity and reactivity of PFOC emulsions.

Document WO2007/139827 A2 discloses a storage-stable fluorocarbon emulsion for in vivo oxygen delivery in patients, comprising a continuous aqueous phase and a discontinuous fluorocarbon phase, wherein the discontinuous fluorocarbon phase comprises perfluorooctylbromide and perfluorodecylbromide, wherein perfluorooctylbromide is present in the fluorocarbon emulsion in an amount of about 57-60 % w/v of the total emulsion and perfluorodecylbromide is present in the emulsion in an amount of about 2-3% w/v of the total emulsion. The emulsion additionally contains an emulsifying agent comprising egg yolk phospholipid in an amount of about 3-4 % w/v. The manufacturing method comprises a) preparing an aqueous solution of sodium salts in hot water (preferably at temp. 65°C to 80°C) in the first tank and saturation with nitrogen; b) addition of perfluorooctyl bromide and perfluorodecyl bromide to the second tank and saturation with nitrogen; c) addition of emulsifying agent to the third tank and addition of the contents of the first tank to the third tank; (d) adding perfluorooctylbromide and perfluorodecylbromide to the third tank and mixing the contents to emulsify the perfluorooctylbromide and perfluorodecylbromide; (e) diverting the contents of the third tank into one or more homogenizing tanks. Although not limited to the following, some other therapeutic applications of the emulsion include its use in improving post-operative organ function (bowel, liver, heart, brain, kidney), treating sickle cell anaemia, organ preservation, organ transplantation or enhancing chemotherapy treatment.

In WO2007/105978, there is disclosed a composition formed from a mixture of fast (C8-C10) and/or slow (C11-C12) excreted types of perfluorocarbons, such as perfluorodecalin (PFD)/perfluoromethylcyclohexylpyridine (PFMCP), or PFD/perfluorotributylamine (PFTBA), or perfluorooctylbromide (PFOB)/PFMCP, or PFOB/PFTBA in a ratio of 1/1 to 10/10, respectively; or mixtures of three types of perfluorocarbons: two rapidly excreted (C8-C10) and one slowly excreted (C11-C12), or one rapidly excreted (C8-C10) and two slowly excreted (C11-C12) perfluorocarbon types such as PFOB/PFD/PFMCP, or PFOB/PFD/PFTBA, or PFOB/PFMCP/PFTBA, or PFD/PFMCP/PFTBA, or PFD/PFMCP/PFTBA in a ratio of 1/1/1 to 10/10/10, respectively; or a mixture of four perfluorocarbon types: two rapidly excreted (C8-C10) and two slowly excreted (C11-C12) types of perfluorocarbons, such as PFOB/PFD/PFMCP/PFTBA in a ratio of 1/1/1/1 to 10/10/10/10, respectively. A mixture of perfluorocarbons in an emulsion of 1% (0.5% by volume) to 100% (50% by volume) and an average particle size of 30-80 nm is emulsified with proxanol-268 in an amount of 0.2% to 20% with a molar mass of 6-12 Da and contains an acceptable electrolyte solution. The method of producing the perfluorocarbon emulsion comprises: producing the emulsion by mixing the total perfluorocarbons with an emulsifier, repeated homogenization of the resulting mixture in a high-pressure homogenizer, the entire emulsion preparation process being carried out in at least two high-pressure extrusion units by sequentially passing the emulsion through a primary and secondary extrusion unit (extruders), passing the emulsion through a buffer volume located between the two extruders to equalise the pressure between the devices, passing the emulsion through the primary extruder, increasing the homogenization pressure by 2-3 times compared to the pressure in the secondary extruder, passing the emulsion through the buffer volume and introducing (supplying) carbon dioxide gas. The disclosed emulsion can be used as a synthetic perfluorocarbon blood substitute for the treatment of various diseases or as a synthetic perfusion agent.

In CN1286081 A, there is disclosed a high concentration, ultra-fine particle perfluorocarbon emulsion for injection which contains a perfluorocarbon, an emulsifier, an isotonic buffer solution, an additive and water. The emulsion is prepared by sterilizing all equipment and containers and producing the emulsion under aseptic conditions. Its advantages are that the product does not need to be sterilized at high temperatures, has a long shelf life and has a long duration of action in the circulatory system. In the disclosed method of manufacture, prior to emulsification, all parts in direct contact with the materials, the product packaging containers and all materials are sterilized and pyrogens are removed prior to emulsification. The material is then weighed in a clean environment, all ingredients except perfluorocarbon are added to water to form an aqueous solution and then sterilized using high-pressure steam. The next step is to heat the aqueous solution to a temperature 2-10°C higher than the turbidity point of the emulsifier, stir to make the aqueous solution transparent, then add the perfluorocarbon, stir evenly, pass through the homogenizer circuit; the first pass through the homogenizer has a pressure of 100-200 kg/cm$^2$, the second pass has a low pressure of 100-200 kg/cm$^2$ and a high

pressure of 500-700 kg/cm$^2$. The circulation pump pumps the emulsion 5-30 times to achieve an emulsion particle size of 40-80 nm. The disclosed perfluorocarbon emulsion, with high concentration and ultrafine particles for injection, contains 20.0-115.0 g of perfluorocarbon per 100 ml of emulsion; 0.5-10.0 g of emulsifier; 0.3-1.0 g of isotonic buffer, 0.5-10.0 g of additive and the remaining amount of water, with an average particle size in the emulsion in the range of 40-80 nm. The perfluorocarbon is selected from the group including perfluorooctylbromide, bis(perfluorobutyl)ethylene, perfluoroalkyl ethylene, perfluorocarbon ether or perfluorodecalin. The emulsifier is selected from the group comprising egg yolk lecithin, partially hydrogenated egg yolk lecithin or polyether F-68.

Document US5684050 discloses stable emulsions of highly fluorinated organic compounds for use as oxygen transport agents, "artificial blood" or red blood cell substitutes and as contrast agents for biological imaging. These emulsions contain a high-fluorinated organic compound, an oil that is not significantly surface active and not significantly soluble in water, a surfactant and water. The high-fluorinated organic compound is selected from the group comprising perfluorodecalin, perfluorodimethyladamantanes, perfluorooctylbromide, perfluoro-4-methyl-octa-hydroquinolidine, perfluoro-N-methyl-decahydroquinoline, F-methyl-1-oxa-decalin, perfluoro-bicyclo[5.3.0]decane, perfluoro-octa-hydroquinolidine, perfluoro-5,6-dihydro-5-decene and perfluoro-4,5-dihydro-4-octene. The oil is selected from the group consisting of liquid fatty oils, C12-C18 fatty acid diglycerides with one unsaturation, silicone oils and mineral oil, in addition the emulsion may further comprise at least one compound from the group consisting of isotonic agents, osmotic pressure controlling agents and antioxidants. The disclosed emulsion is prepared by mixing the respective components in a Fisher brand blender and then passing them through a microfluidizer for 30 minutes at 60 psi.

Document US4987154 A, discloses a perfluorocarbon emulsion with a concentration of up to 125% for use in animals and their organs, maintains emulsion stability during standard sterilization procedures due to selective osmotic and buffering agents, maintains the emulsion at a specified osmolality level and, when desired, is free from excessive calcium precipitation, reduces damage to red blood cells in vivo and in vitro, reduces the adverse effects of anaemia, reduces viscosity and slows the rate of oxidation, and seeks to equilibrate its distribution in major body organs, thereby reducing toxicity. Osmotic agents can act as buffers and provide nutrients in the form of sugars. Osmotic and buffering agents can selectively include hexahydric alcohols such as mannitol and sorbitol; certain sugars such as glucose, mannose and fructose; and buffering agents that will affect osmolality, including imidazole, tris(hydroxymethyl)aminomethane, sodium chloride, sodium bicarbonate, monobasic potassium phosphate, dibasic potassium phosphate, calcium chloride, magnesium sulphate, monobasic sodium phosphate, dibasic sodium phosphate or combinations thereof. The emulsion may contain tocopherol. The method for emulsifying a perfluorocarbon comprises forced flows under pressure after mixing the perfluorocarbon in the dispersed phase. The perfluorocarbon emulsion can be used to deliver drugs and therapeutic substances that are soluble in the emulsion or transported through the emulsion.

In JP94062402 B2, there is disclosed a sterilizable brominated perfluorocarbon emulsion suitable for intravenous injection, excellent in transporting oxygen to the cellular tissue in the animal body and in maintaining the emulsion, comprising a brominated perfluorocarbon, cholesterol, etc., and a specific compound. The emulsion disclosed comprises an aqueous phase, a small amount of emulsifying agent (e.g. phospholipid or anionic surfactant) and a compound selected from the group consisting of brominated perfluorocarbon, cholesterol, steroid hormone, tocopherol and combinations thereof in an amount to ensure the presence of the compound in the body. Steroid hormone, cholesterol, tocopherol and a mixture thereof are used as the compound. The method of producing a perfluorocarbon emulsion, comprises the steps of: (a) adding a compound selected from the group consisting of steroid hormones, cholesterol, tocopherol and combinations thereof in an in vivo compatible amount to a buffered aqueous solution of glycerol; (b) dispersing an emulsifying agent comprising a phospholipid, brominated perfluorocarbon at a concentration of 50% to 125% by weight/volume in the liquid prepared in step (a); and (c) dividing the resulting mixture into multiple streams and diverting the divided streams at a pressure of at least 281.2 kg/cm$^2$ (a method of emulsion production involving a step of diverting the divided streams at a pressure of 4000 psi).

Paper by Johannes Jägers et al, 18 April 2022, Artificial oxygen carriers in organ preservation: Dose dependency in a rat model of ex-vivo normothermic kidney perfusion, Artificial Organs. 2022;46:1783-1793. (https://onlinelibrary.wiley.com/doi/full/10.1111/aor.14264 ) describes the preservation of organs by ex-vivo normothermic perfusion (EVNP) using albumin-derived perfluorocarbon-based artificial oxygen carriers (A-AOCs), consisting of of albumin-derived perfluorodecalin-filled nanocapsules, prior to transplantation, would be a promising approach to avoid hypoxic tissue damage during organ preservation. According to the Gibbs-Helmholtz equation, the biggest obstacle to emulsion is the formation of a huge phase interface. The large surface tension difference that prevails between perfluorocarbon and albumin is due to the lack of hydrogen bond acceptors, which increases the internal energy of the system. Emulsifiers can adhere to the boundary surface and act as hydrogen bond acceptors to minimise the unwanted increase in internal energy during the manufacturing process. An important property of the albumin emulsifier used in this system is its anisotropy. It has a hydrophilic side and a hydrophobic side that try to escape from the aqueous phase. In this way, albumin acts as an excellent hydrogen bond acceptor on one side and covers the hydrophilic surface on the other side. In the method for making the emulsion, the indicated document reveals to emulsify 4 ml of perfluorodecalin (Fluorochem Chemicals, Derbyshire, UK) in 20 ml of a 5% bovine serum albumin solution (BSA, AppliChem GmbH, Darmstadt, Germany) in a Ringer-saline solution, both cooled liquids are pre-emulsified for 20 s using Ultra-Turrax® (IKA®-Werke GmbH & Co. KG, Staufen, Germany). The emulsion is then further emulsified using microfluidization technology (Microfluidics®, Westwood, MA) at 20000 PSI in single pass mode. The emulsion is then diluted with a Ringer-Saline solution containing 5% BSA to 2%, 4% and 8% A-AOCs, respectively.

Hosgood, Sarah A.; Nicholson, Michael L., 27 May 2010, The Role of Perfluorocarbon in Organ Preservation, Transplantation 89(10):p 1169-1175, 2010, DOI: 10.1097/TP.0b013e3181da6064(https://journals.lww.com/transplant journal/fulltext/2010/0527 0/the_role_of_perfluorocarbon_in_organ_preservation.1.aspx) discloses perfluorocarbon (PFC) emulsions used in organ preservation, dividing them into first generation (e.g. FX-80, FC-43, Fluosol-DA, Oxypherol, Perftoran) and second generation (e.g. Perftoran 1989-1996, Oxygent, Pher O2), with different components and emulsions such as Pluronic F-68, Proxanol-268, phospholipids, perfluorodecalin and perfluorotributylamine. The paper discloses that the most successful application of PFCs is TLM (perfusion therapy) for pancreatic preservation, which has significant evidence of its benefits in clinical practice. However, the paper points out that questions remain about how effectively oxygen can diffuse through tissue under these conditions. Additionally, the usefulness of this technique for organs other than the pancreas has not been thoroughly investigated. Evidence suggests that perfusion or flushing techniques using PFC emulsions are beneficial for hypothermic storage and appear to be superior to static techniques. However, most of these mainly historical, isolated trials have not been introduced into clinical practice, probably due to the instability and adverse reactions associated with first-generation emulsions at the time. Recent advances and the trend towards machine perfusion and the development of improved second-generation PFCs suggest that PFCs may have a significant role in organ preservation techniques in the future. However, further experimental evidence in clinically relevant models is needed to establish this.

**Technical problem**

With the need to increase the pool of potential organs eligible for transplantation, the use of mechanical kidney perfusion methods under subnormothermic and normothermic conditions could realistically increase the number of transplants performed, reduce the duration of dialysis use and reduce the number of deaths of patients awaiting transplantation.

The solution to this problem is to conduct the perfusion process using an acellular fluid containing synthetic oxygen nano-carriers consisting of a nanoemulsion of perfluorinated compounds, the subject of the above invention.

Unexpectedly, it turned out that careful development of the preparation method and optimisation of the composition lead to a perfluorocarbon emulsion giving the possibility of perfusing organs, i.e. kidney, liver, heart, or using it as a potential blood substitute.

The fluid ensures adequate oxygenation and the necessary nutrients for the organ awaiting transplantation. Perfluorocarbon-based perfusion fluid according to the invention also has a significant advantage over products based on blood morphotic elements, as it avoids phenomena such as the release of leukotrienes from them, as well as other inflammatory and vasoconstrictive substances.

**Object of the invention**

Thus, the subject matter of the present invention is a perfusion fluid in the form of an oil-in-water PFC nanoemulsion including:

the disperse phase, which includes:

- an oxygen carrier, preferably in an amount of 100.00-800.00 g/L, more preferably in an amount of 100-400 g/L, which is one or more compounds selected from:
perfluorooctyl bromide, perfluorodecyl bromide; perfluoropentane, perfluorohexane, perfluoroheptane, perfluorotributylamine, perfluorodecalin perfluorooctane, perfluoromethylcyclohexylpiperidine, perfluorodichlorooctane, perfluorobutylcyclohexane, perfluoro-15-crown ether (PFCE), perfluorocyclohexane, preferably perfluorooctyl bromide, perfluorodecyl bromide; perfluoropentane, perfluorohexane, perfluoroheptane, perfluorotributylamine, perfluorodecalin

the water phase, which includes:

- a surfactant which is either the main surfactant or at least one main surfactant and at least one cosurfactant, wherein:

a main surfactant, preferably in an amount of 23.80-40.08 g/L, more preferably in an amount of 23.80-34.00 g/L, which is Pluronic F68 (Kolliphor P188) or Pluronic F108 or bovine serum albumin (BSA) or human serum albumin (HSA) or recombinant human serum albumin or derivatives thereof, diblock/triblock/multiblock pluronics, such as, for example pluronics (condensation polymers of polypropylene glycols with polyoxyethylene glycols) and derivatives thereof, perfluorinated copolymers of the hydrocarbon-fluorocarbon type, gelatin and derivatives thereof, preferably Pluronic F68 (Kolliphor P188) or Pluronic F108 7 or bovine serum albumin (BSA) or human serum albumin (HSA) or recombinant human serum albumin or derivatives thereof

a cosurfactant, preferably in an amount of 0.35-0.60 g/L, more preferably in an amount of 0.35-0.50 g/L, which is: -perfluorinated: (1H, 1H, 2H, 2H-Perfluorooctyl)phosphocholine or (1H, 1H, 2H, 2H-Perfluorooctyl)-β-D-maltopyranoside or modified lipids: 1,2-dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or the sodium salt of N-(carbonyl-methoxy polyethyleneglycol-2000)-1,2-distearoyl-sn-glycero-3-phosphoethanolamine, perfluorinated phospholipids (e.g. phosphatidylethanolamines, phosphatidylserine, phosphatidylinositol, PEGylated phospholipids, sphingomyelins, glycerophosphocholine), perfluorinated glycolipids, preferably (1H, 1H, 2H, 2H-Perfluorooctyl)phosphocholine or (1H, 1H, 2H, 2H-Perfluorooctyl)-β-D-maltopyranoside or modified lipids: 1,2-dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or N-(carbonyl-methoxy polyethylene glycol-2000)-1,2-distearoyl-sn-glycero-3-phosphoethanolamine sodium salt

- an oncotic agent, preferably in an amount of 20.00-40.00 g/L, more preferably in an amount of 40.00 g/L, which is one oncotic agent selected from the group comprising of bovine serum albumin (BSA) or human serum albumin (HSA) or PEG 35 kDa or PEG 20 kDa or dextran 40 kDa or succinylated gelatin or hydroxyethylated starch or recombinant human albumin or derivatives thereof, dextran 30 kDa, dextran 50 kDa or derivatives thereof, gelatin derivatives, cyclodextrins, preferably bovine serum albumin (BSA) or human serum albumin (HSA) or PEG 35 kDa or PEG 20 kDa or dextran 40 kDa or succinylated gelatin or hydroxyethylated starch

- a main osmotic agent, preferably in an amount of 10.00-100.00 mM, most preferably in an amount of 21.40-50.00 nM, which is mannitol or raffinose or sucrose or glucose or citrate chelates or salts of lactobionic acid or gluconic acid, preferably mannitol,

- the main components of the buffer are one or more compounds selected from the

  - D-glucose · $H_2$O or fructose or sucrose or dextran, preferably D-glucose · $H_2$O preferably in an amount of 5.00-20.00 mM, most preferably in an amount of 10.00 mM;

  - sodium salt of DL-β-hydroxybutyric acid, preferably in an amount of 0.10-0.50 mM, most preferably in an amount of 0.20 mM,

  - glutathione or vitamin C or vitamin E or deferoxamine or N-acetylcysteine or catalase or peroxidase, preferably glutathione, preferably in an amount of 1.50-6.00 mM, most preferably in an amount of 3.00 mM, and

  - allopurinol or adenine, adenosine or phosphates or hormones: insulin, steroids i.e. dexamethasone, preferably allopurinol, preferably in an amount of 0.50-3.00 mM most preferably in an amount of 1.00 mM,

- buffer components constitute $Na^+$, $K^+$, $Mg^{2+}$, $Ca^{2+}$, $Cl^-$ salts, preferably selected from the group comprising chloride, sulphate, phosphate, lactobionate, citrate, gluconate, preferably containing $Na^+$, $K^+$, $Mg^{2+}$ $Ca^{2+}$, $Cl^-$ ions, preferably one or more of NaCl, preferably in an amount of 20,00-100.00 mM, most preferably in an amount of 60.00 mM, KCl, preferably in an amount of 2.00-10.00 mM, most preferably in an amount of 4.60 mM, $CaCl_2$ preferably in an amount of 0.30-2.00 mM, most preferably in an amount of 0.60 mM, $MgCl_2$ · $6H_2$O, preferably in an amount of 0.20-2.00 mM, most preferably in an amount of 0.40 mM;

- buffer components maintaining the correct pH, preferably one or more compounds selected from the group $Na_2HPO_4$, $NaH_2PO_4$, bicarbonate buffers consisting of a mixture of $NaHCO_3$ and $H_2CO_3$, phosphate buffers consisting of a mixture of $KH_2PO_4$ and $K_2HPO_4$, citrate buffers consisting of a mixture of $Na_3C_6H_5O_7$·$2H_2O$ and $C_6H_8O_7$·$H_2O$, a histidine buffers consisting of a mixture of histidine-HCl and L-histidine, preferably $Na_2HPO_4$ and/or preferably $Na_2HPO_4$, preferably $Na_2HPO_4$ in an amount of 6.00-15.00 mM, most preferably in an amount of 8.00 mM and NaH$_2$PO$_4$ , preferably in an amount of 0.50-3.00 mM, most preferably in an amount of 1.5 mM;

wherein the perfusion fluid advantageously exhibits osmotic pressures in the range of: 280-400 mOsm/kgH$_2$O, pH in the range of 7.1-7.6, oncotic pressure in the range of 15-36 mmHg, concentration of the main ions: sodium in the range of 80-200 mM, potassium in the range of 1-15 mM, calcium in the range of 0.1-2.5 mM and chlorine in the range of 50-170 mM;

furthermore, the average diameter of the dispersed phase nanoparticles does not exceed 300 nm, preferably is below 220 nm, most preferably is below 130 nm.

Preferably the aqueous phase further comprises amino acids including preferably one or more amino acids selected from the group of: taurine preferably in an amount of 5.00-15.00 mM, most preferably in an amount of 10.00 mM, L-arginine preferably in an amount of 0.05-0.20 mM, most preferably in an amount of 0.10 mM, L-tryptophan preferably in an amount of 1,00-3.00 mM, most preferably 2.00 mM, L-glutamine preferably in an amount of 0.10-1.00, most preferably in an amount of 0.50 mM, L-isoleucine preferably in an amount of 0.50-10.00 mM, most preferably in an amount of 1.52-4.19 mM, L-leucine preferably in an amount of 1.00-10.00 mM, most preferably in an amount of 3.05-6.86 mM, L-lysine preferably in an amount of 1.00-10.00 mM, most preferably in an amount of 2,05-4.79 mM, L-methionine preferably in an amount of 0.50-10.00 mM, most preferably in an amount of 1.34-3.35 mM, L-threonine preferably in an amount of 0.50-10.00 mM, most preferably in an amount of 1,69-3.78 mM, L-valine preferably in an amount of 0.50-10.00 mM, most preferably in an amount of 2.56-5.55, L-histidine preferably in an amount of 0.10-10.00 mM, most preferably in an amount of 0.64-2,26 mM, L-alanine preferably in an amount of 1.00-20.00 mM, most preferably in an amount of 5.61-12.35 mM, L-glycine preferably in an amount of 1.00-20.00 mM, most preferably in an amount of 6.66-17,32 mM, L-aspartic acid preferably in an amount of 0.50-10.00 mM most preferably in an amount of 1.88-4.51 mM, L-proline preferably in an amount of 0.50-10.00 mM most preferably in an amount of 2.17-5,21 mM, L-serine preferably in an amount of 0.10-10.00 mM, most preferably in an amount of 0.95-2.38 mM, L-tyrosine preferably in an amount of 0.01-1.00 mM, most preferably in an amount of 0.06-0.28 mM.

Preferably the oncotic agent is bovine/human albumin or succinylated gelatin, preferably bovine/human albumin, in an amount of: 20-40 g/L, most preferably in an amount of 40 g/L.

Preferably the buffer is mannitol, D-glucose · H$_2$O, glutathione, allopurinol, DL-β-hydroxybutyric acid sodium salt, L-arginine, taurine, L-tryptophan, L-glutamine, L-isoleucine, L-leucine, L-lysine, L-methionine, L-threonine, L-valine, L-histidine, L-alanine, L-glycine, L-aspartic acid, L-proline, L-serine, L-tyrosine, and Na$_2$HPO$_4$, NaH$_2$PO$_4$, NaCl, KCl, CaCl$_2$, MgCl$_2$ ·6H$_2$O and more preferably mannitol, D-glucose · H$_2$ O, glutathione, allopurinol, DL-β-hydroxybutyric acid sodium salt, L-arginine, taurine, L- tryptophan, L-glutamine, and Na$_2$HPO$_4$, NaH$_2$PO$_4$, NaCl, KCl, CaCl$_2$, MgCl$_2$ · 6H$_2$ O.

Preferably, the perfusion fluid contains perfluorooctyl bromide (PFOB) in an amount of 187.21 g/L; perfluorodecyl bromide (PFDB) in an amount of 5.79 g/L; Pluronic F68 (Kolliphor P188) in an amount of 27.20 g/L; (1H, 1H, 2H, 2H-Perfluorooctyl)phosphocholine in an amount of 4.00 g/L; bovine albumin in an amount of 40.00 g/L; L-arginine in an amount of 0.10 mM; allopurinol in an amount of 1.00 mM; mannitol in an amount of 50.00 mM; taurine in an amount of 10.00 mM; L-tryptophan in an amount of 2.00 mM; D-glucose · H$_2$ O in an amount of 10.00 mM; NaCl in an amount of 60.00 mM; KCl in an amount of 4.60 mM; Na$_2$HPO$_4$ in an amount of 8.00 mM; NaH$_2$PO$_4$ in an amount of 1.50 mM; CaCl$_2$ in an amount of 0.60 mM; MgCl$_2$ · 6H$_2$ O in an amount of 0.40 mM; L-glutamine in an amount of 0.50 mM; glutathione in an amount of 3.00 mM; DL-β-hydroxybutyric acid sodium salt in an amount of 0.20 mM; NaOH to adjust a pH in the range of 6.8-7.4.

Preferably, the perfusion fluid contains perfluorooctyl bromide (PFOB) in an amount of 374.42 g/L; perfluorodecyl bromide (PFDB) in an amount of 11.58 g/L; Pluronic F68 (Kolliphor P188) in an amount of 34.00 g/L; (1H, 1H, 2H, 2H-Perfluorooctyl)phosphocholine in an amount of 5.00 g/L; bovine albumin in an amount of 40.00 g/L; L-arginine in an amount of 0.10 mM; allopurinol in an amount of 1.00 mM; mannitol in an amount of 21.40 mM; taurine in an amount of 10.00 mM; L-tryptophan in an amount of 2.00 mM; D-glucose · H$_2$ O in an amount of 10.00 mM; NaCl in an amount of 60.00 mM; KCl in an amount of 4.60 mM; Na$_2$HPO$_4$ in an amount of 8.00 mM; NaH$_2$PO$_4$ in an amount of 1.50 mM; CaCl$_2$ in an amount of 0.60 mM; MgCl$_2$ · 6H$_2$ O in an amount of 0.40 mM; L-glutamine in an amount of 0.50 mM; glutathione in an amount of 3.00 mM; DL-β-hydroxybutyric acid sodium salt in an amount of 0.20 mM; NaOH to adjust a pH in the range of 6.8-7.4.

The subject matter of the present invention is a method for producing a perfusion fluid in the form of a PFC oil-in-water nonoemulsion, comprising the steps of

**1.** preparation of the components of the primary perfusion fluid including

**1a.** preparation of the solution of surfactant or surfactants,

**1b.** preparation of perfluorinated phase,

**1c.** preparation of the buffer

**2.** mechanical homogenization of the components prepared in step **1** to obtain the primary perfusion fluid;

**3.** microfluidization of the primary perfusion fluid obtained in step 2 to obtain the perfusion fluid, wherein:

in step **1a.** the solution of surfactant or surfactants constitutes:

ultrapure water and

the main surfactant or at least one main surfactant and at least one cosurfactant wherein:
the main surfactant is Pluronic F68 (Kolliphor P188) or Pluronic F108 or bovine serum albumin (BSA) or human serum albumin (HSA) or recombinant human serum albumin or derivatives thereof, diblock/triblock/-multiblock pluronics, such as e.g. pluronics (condensation polymers of polypropylene glycols with polyoxyethylene glycols) and derivatives thereof, perfluorinated copolymers of the hydrocarbon-fluorocarbon type, gelatin and derivatives thereof, preferably Pluronic F68 (Kolliphor P188) or Pluronic F108 or bovine serum albumin (BSA) or human serum albumin (HSA) or recombinant human serum albumin or derivatives thereof

cosurfactant constitutes: (1H, 1H, 2H, 2H-Perfluorooctyl)phosphocholine or (1H, 1H, 2H, 2H-Perfluorooctyl)-β-D-maltopyranoside or modified lipids: 1,2-dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or the sodium salt of N-(carbonyl-methoxy polyethyleneglycol-2000)-1,2-distearoyl-sn-glycero-3-phosphoethanolamine, perfluorinated phospholipids (e.g. phosphatidylethanolamines, phosphatidylserine, phosphatidylinositol, PEGylated phospholipids, sphingomyelins, glycerophosphocholine), perfluorinated glycolipids, preferably (1H, 1H, 2H, 2H-Perfluorooctyl)phosphocholine or (1H, 1H, 2H, 2H-Perfluorooctyl)-β-D-maltopyranoside or modified lipids: 1,2-dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or N-(carbonyl-methoxy polyethylene glycol-2000)-1,2-distearoyl-sn-glycero-3-phosphoethanolamine sodium salt

in step **1b.** the perfluorinated phase is one or more compounds selected from perfluorooctyl bromide, perfluorodecyl bromide; perfluoropentane, perfluorohexane, perfluoroheptane, perfluorotributylamine, perfluorodecalin perfluorooctane, perfluoromethylcyclohexylpiperidine, perfluorodichlorooctane, perfluorobutylcyclohexane, perfluoro-15-crown ether (PFCE), perfluorocyclohexane, preferably perfluorooctyl bromide, perfluorodecyl bromide; perfluoropentane, perfluorohexane, perfluoroheptane, perfluorotributylamine, perfluorodecalin

in step **1c.** the solid component of the buffer is:

a. an oncotic agent which is one oncotic agent selected from the group comprising of bovine serum albumin (BSA) or human serum albumin (HSA) or PEG 35 kDa or PEG 20 kDa or dextran 40 kDa or succinylated gelatin or hydroxyethylated starch or recombinant human albumin or derivatives thereof, dextran 30 kDa, , dextran 50 kDa, or derivatives thereof, gelatin derivatives, cyclodextrins, preferably bovine serum albumin (BSA) or human serum albumin (HSA) or PEG 35 kDa or PEG 20 kDa or dextran 40 kDa or succinylated gelatin or hydroxyethylated starch;

b. the main osmotic agent, which is mannitol or raffinose or sucrose or glucose or citrate chelates or salts of lactobionic acid or gluconic acid, preferably mannitol;

c. the main component of a buffer which is one or more compounds such as

- D-glucose · $H_2O$ or fructose or sucrose or dextran, preferably D-glucose · $H_2O$,

- DL-β-hydroxybutyric acid sodium salt,

- glutathione or vitamin C or vitamin E or deferoxamine or N-acetylcysteine or catalase or peroxidase, preferably glutathione,

- allopurinol or adenine or adenosine or phosphates or hormones preferably insulin or steroids preferably dexamethasone, most preferably allopurinol;

d. buffer components constitute $Na^+$, $K^+$, $Mg^{2+}$, $Ca^{2+}$, $Cl^-$ salts, preferably selected from the group consisting of chloride, sulphate, phosphate, lactobionate, citrate, gluconate, preferably containing $Na^+$, $K^+$, $Mg^{2+}$, $Ca^{2+}$, $Cl^-$ ions, preferably one or more, preferably all, of NaCl, KCl, $CaCl_2$ , $MgCl_2 \cdot 6H_2O$;

e. buffering components maintaining the correct pH preferably one or more compounds selected from the group $Na_2HPO_4$, $NaH_2PO_4$, buffers such as bicarbonate consisting of a mixture of $NaHCO_3$ and $H_2CO_3$, phosphate consisting of a mixture of $KH_2PO_4$ and $K_2HPO_4$, citrate consisting of a mixture of $Na_3C_6H_5O_72H_2O$ and $C_6H_8O_7 \cdot H_2O$, histidine consisting of a mixture of histidine-HCl and L-histidine, preferably $Na_2HPO_4$ and/or $Na_2HPO_4$

f. possibly other constituents that are amino acids including preferably one or more amino acids selected from the group taurine, L-arginine, L-tryptophan, L-glutamine, L-isoleucine, L-leucine, L-lysine, L-methionine, L-threonine, L-valine, L-histidine, L-alanine, L-glycine, L-aspartic acid, L-proline, L-serine, L-tyrosine.

Preferably, in **step 3,** the primary perfusion fluid obtained in step **(2)** is pumped at a pressure of 2065 bar through a polycrystalline diamond Y-type microfluidizer chamber with an inner diameter of 75 µm microchannels and cooled using a water bath so that the temperature of the fluid leaving the microfluidizer chamber does not exceed 30 °C; and/or
in **step 3,** the microfluidization of the primary perfusion fluid obtained in step **2** is carried out in cycles of 1-15 passes of the pre-emulsion obtained in step **2** through the microfluidizer chamber, preferably 3-9 passes.

The subject matter of the present invention is a method for producing a perfusion fluid in the form of an oil-in-water PFC nonoemulsion, comprising the steps of

1. preparation of the initial perfusion fluid components including

1. preparation of the pre-emulsion components comprising

**Ia.** preparation of the solution of surfactant or surfactants

**Ib.** preparation of the perfluorinated phase

**II.** mechanical homogenization of the components prepared in step **I** to obtain a primary pre-emulsion

**III.** microfluidization of the primary pre-emulsion obtained in step **II** to obtain the pre-emulsion,

**IV.** the preparation of the buffer comprising

**IVa.** preparation of buffer components

**IVR.** pH adjustment

**IVQ.** quality control of the buffer obtained

2. mixing the pre-emulsion obtained in step **III** with the buffer obtained in step **IV** to obtain the primary perfusion fluid, wherein the components being preferably mixed in a ratio of pre-emulsion to buffer of 1: 1 to 1:2;

3. microfluidization of the primary perfusion fluid obtained in step **2** to obtain the perfusion fluid; whereby:

in step **1a.** the solution of surfactant or surfactants constitutes:

ultrapure water and

the main surfactant or at least one main surfactant and at least one cosurfactant with:
the main surfactant is Pluronic F68 (Kolliphor P188) or Pluronic F108 or bovine serum albumin (BSA) or human serum albumin (HSA) or recombinant human serum albumin or derivatives thereof, diblock/triblock/-multiblock pluronics, such as e.g. pluronics (condensation polymers of polypropylene glycols with polyoxyethylene glycols) and derivatives thereof, perfluorinated copolymers of the hydrocarbon-fluorocarbon type, gelatin and derivatives thereof, preferably Pluronic F68 (Kolliphor P188) or Pluronic F108 or bovine serum albumin (BSA) or human serum albumin (HSA) or recombinant human serum albumin or derivatives thereof

cosurfactant constitutes: (1H, 1H, 2H, 2H-Perfluorooctyl)phosphocholine or (1H, 1H, 2H, 2H-Perfluorooctyl)-β-D-maltopyranoside or modified lipids: 1,2-dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or the sodium salt of N-(carbonyl-methoxy polyethyleneglycol-2000)-1,2-distearoyl-sn-glycero-3-phosphoethanolamine, perfluorinated phospholipids (e.g. phosphatidylethanolamines, phosphatidylserine, phosphatidylinositol, PEGylated phospholipids, sphingomyelins, glycerophosphocholine), perfluorinated glycolipids, preferably (1H, 1H, 2H, 2H-Perfluorooctyl)phosphocholine or (1H, 1H, 2H, 2H-Perfluorooctyl)-β-D-maltopyranoside or modified lipids: 1,2-dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or N-(carbonyl-methoxy polyethylene glycol-2000)-1,2-distearoyl-sn-glycero-3-phosphoethanolamine sodium salt

in step **1b.** the perfluorinated phase constitutes of one or more compounds selected from perfluorooctyl bromide, perfluorodecyl bromide; perfluoropentane, perfluorohexane, perfluoroheptane, perfluorotributylamine, perfluorodecalin perfluorooctane, perfluoromethylcyclohexylpiperidine, perfluorodichlorooctane, perfluorobutylcyclohexane, perfluoro-15-crown ether (PFCE), perfluorocyclohexane, preferably perfluorooctyl bromide, perfluorodecyl bromide; perfluoropentane, perfluorohexane, perfluoroheptane, perfluorotributylamine, perfluorodecalin

in step **1c.** the solid component of the buffer is:

a. an oncotic agent, which is one oncotic agent selected from the group comprising of bovine serum albumin (BSA) or human serum albumin (HSA) or PEG 35 kDa or PEG 20 kDa or dextran 40 kDa or succinylated gelatin or hydroxyethylated starch or recombinant human albumin or derivatives thereof, dextran 30 kDa, dextran 50 kDa or derivatives thereof, gelatin derivatives, cyclodextrins, preferably bovine serum albumin (BSA) or human serum albumin (HSA) or PEG 35 kDa or PEG 20 kDa or dextran 40 kDa or succinylated gelatin or hydroxyethylated starch;

b. the main osmotic agent, which is mannitol or raffinose or sucrose or glucose or citrate chelates or salts of lactobionic acid or gluconic acid, preferably mannitol;

c. the main component of the buffer, which is one or more compounds selected from the

- D-glucose · $H_2O$ or fructose or sucrose or dextran, preferably D-glucose · $H_2O$,

- DL-β-hydroxybutyric acid sodium salt,

- glutathione or vitamin C or vitamin E or deferoxamine or N-acetylcysteine or catalase or peroxidase, preferably glutathione, and

- allopurinol or adenine or adenosine or phosphates or hormones preferably insulin or steroids preferably dexamethasone, most preferably allopurinol;

d. buffer components constitute $Na^+$, $K^+$, $Mg^{2+}$, $Ca^{2+}$, $Cl^-$ salts, preferably selected from the group comprising of chloride, sulphate, phosphate, lactobionate, citrate, gluconate, preferably containing $Na^+$, $K^+$, $Mg^{2+}$, $Ca^{2+}$, $Cl^-$ ions, preferably one or more, preferably all, of NaCl, KCl, $CaCl_2$, $MgCl_2$ · $6H_2O$,;

e. buffer components that maintain the correct pH: preferably one or more compounds selected from the group $Na_2HPO_4$, $NaH_2PO_4$, buffers such as bicarbonate consisting of a mixture of $NaHCO_3$ and $H_2CO_3$, phosphate consisting of a mixture of $KH_2PO_4$ and $K_2HPO_4$, citrate consisting of a mixture of $Na_3C_6H_5O_7·2H_2O$ and $C_6H_8O_7·H_2O$, histidine consisting of a mixture of histidine-HCl and L-histidine,

preferably $Na_2HPO_4$ and/or $Na_2HPO_4$

f. optionally other constituents that are amino acids including preferably one or more amino acids selected from the group of taurine, L-arginine, L-tryptophan, L-glutamine, L-isoleucine, L-leucine, L-lysine, L-methionine, L-threonine, L-valine, L-histidine, L-alanine, L-glycine, L-aspartic acid, L-proline, L-serine, L-tyrosine.

Preferably, in **stage III,** preferably the primary pre-emulsion obtained in stage **(II)** is pumped at a pressure of 2065 bar through a polycrystalline diamond Y-type microfluidizer chamber with an inner diameter of 75 $\mu$m microchannels and cooled by means of a water bath so that the temperature of the fluid leaving the microfluidizer chamber does not exceed 30 °C.

and in **step 3.** preferably the primary perfusion fluid obtained in step **(2)** is pumped at a pressure of 2065 bar through a polycrystalline diamond Y-type microfluidizer chamber with an inner diameter of 75 $\mu$m microchannels and cooled by means of a water bath so that the temperature of the fluid leaving the microfluidizer chamber does not exceed 30 °C; and/or

in **stage III.** microfluidization of the primary pre-emulsion obtained in stage **II** to obtain the pre-emulsion is carried out in cycles of 1-10 passings of the PFC primary pre-emulsion through the microfluidizer chamber, most preferably 8 passings

wherein

in **step 3,** microfluidization of the primary perfusion fluid obtained in step **2** is carried out in cycles of 1-10 passes of the pre-emulsion obtained in step **2** through the microfluidizer chamber, most preferably 1-5 passes.

Preferably, the oncotic agent is bovine/human albumin or succinylated gelatin, and preferably bovine/human albumin, in an amount of: 20-40 g/L, preferably in an amount of 40 g/L.

Preferably, the buffer is mannitol, D-glucose · $H_2O$, glutathione, allopurinol, DL-$\beta$-hydroxybutyric acid sodium salt, L-arginine, taurine, L-tryptophan, L-glutamine, L-isoleucine, L-leucine, L-lysine, L-methionine, L-threonine, L-valine, L-histidine, L-alanine, L-glycine, L-aspartic acid, L-proline, L-serine, L-tyrosine, and $Na_2HPO_4$, $NaH_2PO_4$, NaCl, KCl, $CaCl_2$, $MgCl_2$ · $6H_2O$ and more preferably mannitol, D-glucose · $H_2O$, glutathione, allopurinol, DL-$\beta$-hydroxybutyric acid sodium salt, L-arginine, taurine, L-tryptophan, L-glutamine, and $Na_2HPO_4$, $NaH_2PO_4$, NaCl, KCl, $CaCl_2$ $MgCl_2$ · $6H_2O$.

Preferably, the primary surfactant is: Pluronic F68 (Kolliphor P188) or Pluronic F108 or Pluronic F127 or recombinant bovine/human albumin, in an amount of: 23.80-40.08 g/L, preferably 23.80-34.00 g/L, and the cosurfactant is: -perfluorinated: (1H, 1H, 2H, 2H-Perfluorooctyl)phosphocholine or (1H, 1H, 2H, 2H-Perfluorooctyl)-$\beta$-D-Maltopyranoside or modified lipid: 1,2-Dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or N-(carbonyl-methoxy polyethylene glycol-2000)-1,2-distearoyl-sn-glycero-3-phosphoethanolamine sodium salt, in amounts: 0.35-0.60 g/L, preferably 0.35-0.50 g/L.

Preferably, steps **1** to **3 of** the method are carried out under sterile/sterile conditions and/or step **3** shall be followed by step **4** involving sterilization of the perfusion fluid obtained in step 3

to obtain sterile perfusion fluid,

wherein sterilization of the perfusion fluid obtained in step **3** is carried out by sterilization filtration, gamma radiation, sterilization by ethylene oxide, preferably by sterilizing filtration,

alternatively, after steps **1-3** or after step **4,** packaging of the resulting sterile perfusion fluid is carried out.

The subject matter of the present invention is the perfusion fluid obtained by the method according to invention, preferably having a qualitative and quantitative composition and physicochemical properties as disclosed above.

The subject matter of the present invention is also the perfusion fluid for use in ex vivo machine perfusion of organs for transplantation, preferably donations of organs after brain death - DBD and donations taken from donors after irreversible cardiac death - DCD or donors aged 60 years and over and donors aged 50-59 years with at least two of the following risk factors: hypertension, death due to stroke, blood creatinine level at the time of death above 1.5 mg/dL - ECD, for better storage and reconditioning of the organ before transplantation.

The subject matter of the present invention is the perfusion fluid for use in ex vivo machine perfusion of organs, preferably including kidney, liver, heart.

The subject matter of the present invention is the perfusion fluid for use in ex vivo machine perfusion of organs, preferably including subnormothermic and normothermic conditions, in particular hybrid conditions containing periods of hypothermic perfusion, periods of controlled linear temperature increase or decrease and periods of subnormothermic or normothermic perfusion over the whole application range of 4-37 °C and a MAP range of 30-120 mmHg.

**Detailed description of the solution**

**Method**

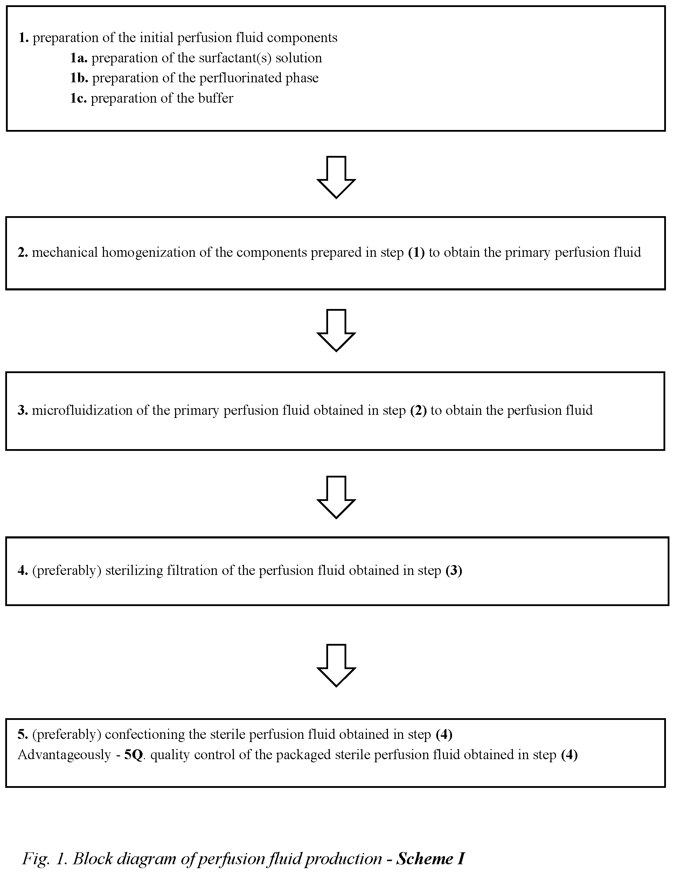

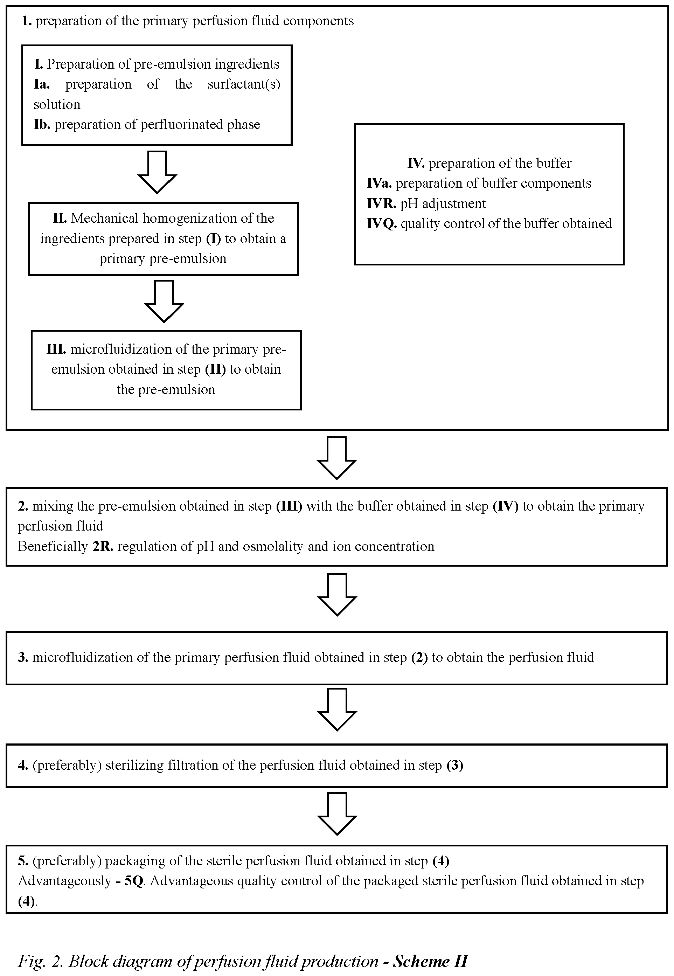

Perfusion fluid is prepared according to the methods shown in Fig. 1 and Fig. 2.

**Method I for** the manufacture of a perfusion fluid in the form of an oil-in-water PFC nanoemulsion as is shown in Fig. 1 includes the steps of

   **1.** preparation of the primary perfusion fluid components including:

   **1a.** preparation of surfactant solution(s),

Suitable surfactants are weighed on an analytical balance. and are dissolved in ultrapure water.

The appropriate compounds and their quantities are indicated in Table 1, with the compounds used being the main surfactant or at least one main surfactant and at least one cosurfactant.

The solution is placed on a magnetic stirrer at room temperature for about 24 h. The clear solution of the surfactant mixture is then transferred to a volumetric flask and made up to volume with ultrapure water.

   **1b.** preparation of the perfluorinated phase,

The perfluorinated phase is a mixture or a single compound. The mixture is prepared by dissolving the appropriate compound(s) and stirring mechanically, at room temperature for approximately 0.25 h.

The appropriate compounds and their quantities are indicated in Table 1.

   **1c.** buffer preparation,

The buffer components are weighed on an analytical balance and dissolved in ultrapure water. The appropriate compounds and their amounts are indicated in Table 1.

The buffer is mixed on a magnetic stirrer using 350 rpm at 37 °C for about 2 h. Once all components are dissolved, pH adjustment is performed (correction/elevation with 5 M NaOH to a value of 7.4). The solution is then transferred to a 2 L volumetric flask and topped up with an appropriate amount of ultrapure water. A preliminary quality check of the resulting buffer is also performed, including: measurement of pH, osmotic pressure and ion concentration - sodium, potassium, chlorine and calcium.

   **2.** mechanical homogenization of the components prepared in step **1** to obtain the initial perfusion fluid;

The primary perfusion fluid is obtained by measuring into a 250 mL beaker using graduated cylinders and automatic pipettes the respective volumes of the surfactant solution (**1a**), perfluorinated phase (**1b**) and buffer (**1c**) prepared in step **1.**

The mixture is emulsified by mechanical homogenzation to obtain the primary perfusion fluid **(2).**

In the examples below, this step uses:

-    PRO25D mechanical homogenizer with 20 x 115 mm homogenizing tip,

-    Process parameters: the speed of the homogenizer motor was approximately 12000 rpm, the duration of the process - 1 min homogenization process: 1 minute interval/100 mL pre-emulsion.

-    The homogenization process was carried out for about 4 minutes.

However, this stage is not limited to this device and the above parameters.

   **3.** microfluidization of the primary perfusion fluid obtained in step **2** to obtain the perfusion fluid;

Essentially, the microfluidic technique used in the present invention makes it possible to obtain more stable nanoparticle systems. The technique involves forcing particles of a given mixture/solution to collide under high pressure of about 2000 bar in a diamond interaction chamber of specific design, resulting in an effective reduction in the size of the nanoparticles obtained and a reduction in their polydispersity index. The technique is typical for a variety of nanoparticle solutions, including PFCs, but is not typical for other perfusion fluids.

In this step, the primary perfusion fluid obtained in step **(2)** is microfluidized **(3)** using a high-pressure homogenizer to obtain the perfusion fluid:

In the examples below, this step uses

- a high-pressure homogenizer (microfluidizer),

- the primary perfusion fluid obtained in step **(2)** is pumped, preferably at a pressure of 2065 bar, through a polycrystalline diamond Y-type microfluidizer chamber with an inner diameter of 75 $\mu$m microchannels and it is cooled by means of a water bath (so that the temperature of the fluid leaving the microfluidizer chamber does not exceed 30 °C).

- the number of repetitions of pumping the total volume of the primary perfusion fluid through the microfluidizer chamber is 1-15 passes/cycles, preferably 9 passes/cycles,

However, this stage is not limited to this device and the above parameters.

In this step, samples are taken to test the pH and osmolality and the ion concentration; if the measurements indicated an inadequate pH, it is adjusted by the addition of a solution of alkali (5M NaOH) or acid (1M HCl), to a value of 7.4 at T = 21-23° C. A test of the osmolality of the preparation is also performed (it must not exceed 400 mOsmol/kgH$_2$O), and the ion concentration is tested (concentration in the range: 80-200 mmol/L for Na$^+$, 1-15 mmol/L for K$^+$, 0.1-2.5 mmol/L for Ca$^{2+}$, 50-170 mmol/L for Cl$^-$ ).

**4.** sterilization of the perfusion fluid obtained in step **3;**

The perfusion fluid is subjected to sterilization, by means known to the expert in the field, for example by sterilizing filtration, gamma radiation or ethylene oxide sterilization. Advantageously, as illustrated in the following examples, the fluid is subjected to sterilization filtration, carried out under sterile conditions (under a chamber with laminar air flow, provided with HEPA filters).

The filtration process aims to remove possible contaminants from mixing and microfluidization (metal filings, fragments of equipment seals) and microbial infections. In the examples shown below, ready-to-use filter sets are used for sterilization by this method, consisting of a funnel with a 0.22 $\mu$m pore diameter membrane and a 250 mL receiver, MerckMillipore, USA. Perfusion fluid is placed on the filter and filtered using a vacuum pump into a sterile container.

Alternatively, as also mentioned above, the method can be carried out under sterile/sterile conditions as a possible alternative to the sterilization step.

**5.** Confection of the sterile perfusion fluid obtained in step **4;**

The ways of packaging are known to the expert in the field and the way should not be limited to them.

In the examples shown below, the resulting perfusion fluid is transferred from an intermediate pack, to a sterile target pack under a laminar chamber. The fluid is bottled into sterile 250 mL or 100 mL plastic bottles fitted with septa stoppers, Nalgene, Thermo Fischer. Each bottle is labelled with batch number, date of production and storage conditions. In addition, samples are also bottled for perfusion fluid quality control **(5Q)** tests: physicochemical tests (measurement of particle size distribution, zeta potential, osmolality, pH, concentration of selected ions, viscosity, oncotic pressure, biological tests (cytotoxicity).

Method **II for** the production of the perfusion fluid in the form of an oil-in-water PFC nonoemulsion as shown in Fig. 2 includes the steps:

**1.** preparation of the primary perfusion fluid components

**I.** Preparation of pre-emulsion ingredients

**Ia.** Preparation of surfactant solution(s)

Appropriate surfactants are weighed on an analytical balance.

The appropriate compounds and their quantities are indicated in Table 1, with the compounds used being the main surfactant or at least one main surfactant and at least one cosurfactant.

They are then placed in suitable beakers and dissolved in ultrapure water. The solutions are stirred on a magnetic stirrer at room temperature, approximately 24 hours. The clear solution is then transferred to suitable volumetric flasks and topped up with ultrapure water to the appropriate volume, and when combined, a surfactant or surfactants solution is obtained.

**Ib.** Preparation of the perfluorinated phase

The perfluorinated phase is a mixture or a single compound. The mixture is prepared by dissolving the appropriate compound(s) and stirring mechanically, at room temperature, for about 15 min.

The appropriate compounds and their quantities are indicated in Table 1.

**II.** Mechanical homogenization of the ingredients prepared in stage **I** to obtain a primary pre-emulsion

In this step, the resulting perfluorinated phase **Ib** is combined with the surfactant solution **Ia** by mechanical homogenization to obtain a primary pre-emulsion.

In the examples below, this step uses:

- PRO25D mechanical homogenizer with 20 × 115 mm homogenizing tip,

- and the process parameters: the homogenizer motor speed is approximately 12,000 rpm, the process duration is 1 min homogenization process: 1 minute interval/100 mLprimary pre-emulsion,

However, this stage is not limited to this device and the above parameters.

**III.** Microfluidization of the primary pre-emulsion obtained in step **II** to obtain the pre-emulsion

In this step, microfluidization of the primary pre-emulsion obtained in step **II** is carried out using a high-pressure homogenizer:

In the examples below, this step uses

- a high-pressure homogenizer (microfluidizer),

- the primary pre-emulsion obtained in step **II** is pumped, preferably at a pressure of 2065 bar, through a polycrystalline diamond Y-type microfluidizer chamber with an inner diameter of 75 $\mu$m microchannels and cooled using a water bath (so that the temperature of the fluid leaving the microfluidizer chamber does not exceed 30 °C).

- the number of times the total volume of emulsion is forced through the microfluidizer chamber was 1-10 passes/cycles, preferably 8 passes/cycles.

However, this stage is not limited to this device and the above parameters.

Basically, with a certain number of permeations/microfluidization cycles, a certain size of nanoparticles is obtained. The higher the number of repetitions, the better the particle size distribution of the pre-emulsion.

In this step, a pre-emulsion is obtained.

**IV.** Preparation of the buffer

**IVa.** preparation of buffer components

In this step, the buffer ingredients will be prepared. In the non-restrictive examples shown below, the buffer components are weighed on an analytical balance and placed in a beaker. The appropriate compounds and their amounts are indicated in Table 1. The buffer is mixed on a magnetic stirrer using 350 rpm at 37 °C for approximately 2 hours.

**IVR.** pH regulation

Once all components have dissolved, pH adjustment is performed (correction/elevation with 5 M NaOH to a value of 7.4).

The pH adjustment at this stage affects the stability of the final obtained emulsion, as a significant change in pH may cause or accelerate ageing of the nanoemulsion and separation of two nanoemulsion phases.. The final aim is a pH in the range of 7.1-7.6. Adjustment of the pH is necessary to obtain a fluid with the correct pH. At this stage, it is also possible to dissolve the buffer components more quickly.

**IVQ.** Quality control of the resulting buffer .

A preliminary quality control of the resulting buffer **(4Q)** is also performed at this stage, including: measurement of pH, osmotic pressure and ion concentration - sodium, potassium, chlorine and calcium.

This is a typical preparation for the final microfluidization and is an activity to check that the buffer obtained has the right parameters and can be used for the further process according to standard quality control at production. This activity is intended to exclude possible coarse weighing errors.

**2.** mixing of the pre-emulsion with the buffer

The primary perfusion fluid is obtained by mixing the pre-emulsion and the buffer in appropriate proportions. In the examples shown below, the volume of the pre-emulsion and the buffer was measured using a measuring cylinder in the ratio: 1:2 (for 1 L of the pre-emulsion, 2 L of the buffer was added) or the pre-emulsion was mixed with buffer in the ratio: 1:1 (for 1.5 L of the pre-emulsion, 1.5 L of the buffer was added).

The primary perfusion fluid is mixed on a magnetic stirrer at 250 rpm, at room temperature, approximately 1h.

**2R.** Regulation of pH and osmolality and ion concentration

Samples are then taken to test pH and osmolality and ion concentration **(2Q),** if measurements indicated an inadequate pH, in the range 7.1-7.6 it is adjusted by the addition of an alkali solution (5M NaOH) or acid (1M HCl), to a value of 7.4 at T = 21-23 °C **(2R).**

However, care is taken to ensure that the osmolality of the initial perfusion fluid does not exceed 400 $mOsmol/kgH_2O$ and that ion concentrations are in the range: 80-100 mmol/L for $Na^+$, 1-15 mmol/L for $K^+$, 0.1-2.5 mmol/L for $Ca^{2+}$, 50-170 mmol/L for $Cl^-$.

**3.** Microfluidization of the primary perfusion fluid to obtain the perfusion fluid

The primary perfusion fluid obtained in step **(2)** is microfluidized **(3)** using a high-pressure homogenizer to obtain the perfusion fluid:
In the examples below, this step uses:

- a high-pressure homogenizer (microfluidizer),

    - the initial perfusion fluid obtained in step **(2)** is pumped, preferably at a pressure of 2065 bar, through a polycrystalline diamond Y-type microfluidizer chamber with an inner diameter of 75 $\mu$m microchannels and cooled by means of a water bath (so that the temperature of the fluid leaving the microfluidizer chamber does not exceed 30 °C),

    - the number of times the total volume of emulsion is forced through the microfluidizer chamber was 1-5 passes/cycles.

However, this stage is not limited to this device and the above parameters.

At the higher PFC concentration characteristic of the perfluorinated phase, a so-called thick emulsion is formed, in which the PFC nanoparticles are larger due to its concentration. At this stage, at a given target concentration, the microfluidization step allows the smallest possible nanoparticles to be obtained at a given PFC and surfactant concentration. This stage may be called final microfluidization.

The parameters for both microfluidizations (in step **(3)** and in step **III.**) are the same, they differ in the composition of the mixture that is microfluidized.

**4.** sterilizing filtration

The perfusion fluid is subjected to sterilization, by methods known to the expert in the field, as already indicated above, for example by sterilizing filtration, gamma radiation or sterilization with ethylene oxide. Advantageously, as shown in the following examples, the perfusion fluid obtained in step **(3)** is subjected to a sterilization filtration step **(4).** The sterilization filtration step **(4)** is carried out under sterile conditions (under a chamber with laminar air flow, provided with HEPA filters). The sterilization filtration process **(4)** is carried out in order to get rid of possible contaminants generated after mixing and microfluidization (metal filings, fragments of equipment seals) and microbial infections.

In the examples shown below, ready-made filter sets were used for sterilization by this method, consisting of a funnel with a 0.22 $\mu$m pore diameter membrane and a 250-500 mL receiver.

The perfusion fluid obtained in step **(3)** is placed on filters and filtered using a vacuum pump into sterile containers.

Alternatively, as also mentioned above, the method can be carried out under sterile/sterile conditions as a possible alternative to the sterilization step.

**5.** Confection

The ways of packaging are known to the expert in the field and the way should not be limited to them.

In the examples shown below, after the perfusion fluid has been cleared of particulates and microorganisms using the sterile filtration process **(4),** the next step is to transfer the perfusion fluid from the intermediate package, to the sterile target package under the laminar chamber. The perfusion fluid is bottled into sterile 1000 mL, 500 mL, 250 mL or 100 mL plastic bottles fitted with septa stoppers, Nalgene, Thermo Fischer or into 10 mL, 20 mL, 50 mL or 100 mL glass vials capped with aluminium septa caps. Appropriate volumes of perfusion fluid are measured using graduated cylinders or an automatic pipettor. Each bottle is labelled with the batch number, date of manufacture and storage conditions.

**SQ.** Quality control

In addition, samples are also bottled for perfusion fluid quality control **(SQ)** tests: physicochemical tests (measurement of: particle size distribution, zeta potential, osmolality, pH, concentration of selected ions, oncotic pressure, viscosity and cytotoxicity tests).

**Perfusion fluid**

The perfusion fluid which is the subject of this invention is in the form of an emulsion (o/w type) in which the dispersed phase is perfluorinated compounds having the ability to dissolve and transport the respiratory gases oxygen and carbon dioxide. The perfluorinated phase is composed of mixtures or single PFCs from among perfluorooctyl bromide, perfluorodecyl bromide, perfluoropentane, perfluorohexane, perfluoroheptane, perfluorotributylamine, perfluorodecalin (based on examples of the implementation of the invention) and based on literature data (DOI: 10.2217/nnm-2019-0260) perfluorooctane, perfluoromethylcyclohexylpiperidine, perfluorodichlorooctane, perfluorobutylcyclohexane, perfluoro-15-crown ether (PFCE), perfluorocyclohexane.

The PFC nanoparticles are stabilised by the addition of biocompatible surfactants. F68 (Kolliphor P188) or Pluronic F-108 or Pluronic F127 or their diblock/triblock/multiblock copolymers or condensation polymers of polypropylene glycols with polyoxyethylene glycols (https://doi.org/10.1166/jnn.2006.449) or perfluorinated copolymers of the hydrocarbon-fluorocarbon type or perfluorinated copolymers of the hydrocarbon-fluorocarbon type (https://doi.org/10.1002/macp.201700558) or mammalian albumin: bovine serum albumin (BSA), human serum albumin (HSA) and recombinant human albumin or their derivatives, gelatine and its derivatives. Perfluorinated surfactants were used as cosurfactant: (1H, 1H, 2H, 2H-perfluorooctyl)phosphocholine, (1H, 1H, 2H, 2H-perfluorooctyl)-$\beta$-D-maltopyranoside, modified lipids: 1,2-dioleoyl-sn-glycero-3-phospho-L-serine sodium salt, 1,2-dipalmitoyl-sn-glycero-3-phosphate sodium salt or 1,2-dimyristoyl-sn-glycero-3-phospho-rac-glycerol sodium salt, sodium salt of N-(carbonyl-methoxypolyethyleneglycol-2000)-1,2-distearoyl-sn-glycero-3-phosphoethanolamine or perfluorinated phospholipids (e.g. Phosphatidylethanolamines, phosphatidylserine, phosphatidylinositol, PEGylated phospholipids, sphingomyelins, glycerophosphocholine), perfluorinated glycolipids.

The continuous phase of the emulsion is a buffer solution, ensuring physiological (for human blood) pH, osmotic pressure and oncotic pressure of the perfusion fluid. To ensure adequate perfusion fluid parameters, mammalian albumin is used as the oncotic agent: bovine serum albumin (BSA), human serum albumin (HSA), recombinant human albumin or their derivatives, gelatine and its derivatives or PEG 35 kDa or PEG 20 kDa or dextran 70 kDa, dextran 40 kDa, dextran 30 kDa, dextran 50 kDa or succinylated gelatine or hydroxyethylated starch or cyclodextrin derivatives.

Mannitol is used as the osmotic agent, but other osmotic agents described in the literature are possible, i.e. raffinose, sucrose, glucose, citrate chelates, lactobionic acid salts and gluconic acid.

In addition to oxygen-carrying substances, the perfusion fluid contains a source of energy for the cells (i.e. D-glucose · $H_2O$, fructose, sucrose, dextran), compounds that enable the regeneration of the high-energy phosphate compound - ATP (i.e. sodium salt of D-$\beta$-hydroxybutyric acid), substances that reduce/neutralise the formation of free radicals and peroxides and prevent intracellular acidosis (i.e. glutathione, vitamin C, vitamin E, deferoxamine, N-acetylcysteine, catalase, peroxidase or xanthine oxidase inhibitors, i.e. allopurinol, adenine, adenosine, phosphates;

hormones: insulin, steroids - dexamethasone). The perfusion fluid also contains amino acids with various functions. These include: stimulation of nitric oxide synthesis, prevention of epithelial cell damage, antioxidant, osmotic pressure regulation, anti-inflammatory and anti-apoptotic effects, protection of cells against lipid peroxidation and harmful changes to membrane structure, substrates for protein and coenzyme synthesis, participation in protein synthesis, role in nitrogen metabolism and in the synthesis of purines, pyrimidines and other amino acids. In the perfusion fluid are used: L-isoleucine, L-leucine, L-lysine, L-methionine, L-threonine, L-valine, L-histidine, L-alanine, L-glycine, L-aspartic acid, L-proline, L-serine, L-tyrosine. Buffering ingredients and electrolytes that affect the osmotic pressure of the perfusion fluid are also used, i.e.: $Na_2HPO_4$, $NaH_2PO_4$, NaCl, KCl, $CaCl_2$, $MgCl_2 \cdot 6H_2O$, NaOH. It is possible to use other buffer systems and salts described in the literature, i.e. buffer systems: bicarbonate (mixture of $NaHCO_3$ and $H_2CO_3$), phosphate (containing $KH_2PO_4$ and $K_2HPO_4$), citrate (containing $Na_3C_6H_5O_7 \cdot 2H_2O$ and $C_6H_8O_7 \cdot H_2O$), Histidine (containing histidine-HCl and L-histidine), or sodium, potassium, calcium, magnesium and chlorine salts: sulphate (VI), phosphate, lactobionate, citrate, gluconate.

**Table 1** shows the overall composition of the fluid according to the invention, including the role of the individual components and the range of concentrations.

**Table 1.** General composition of the perfusion fluid.

| Individual component groups | Examples of compounds to be claimed in the present invention | Concentration | Units | Benefits of the perfusion fluid |
|---|---|---|---|---|
| PFC - oxygen carrier, mixture or single compound | A mixture of perfluorooctyl bromide and perfluorodecyl bromide or perfluoropentane or perfluorohexane or perfluoroheptane or perfluorotributylamine or perfluorodecalin | 96.51-772.00 favourably 96.51-386.00 most favourably 193.00-386.00 | g/L | The main component of the nanoemulsion responsible for the transport of respiratory gases |
| Surfactant, mixture: selected main surfactant and cosurfactant | Main surfactant: Pluronic F68 (Kolliphor P188) or Pluronic F-108 or Pluronic F-127 or bovine/human serum albumin. | 0.0-7.6 favourably 23.8-34.0 most favourably 27.2-34.0 | g/L | The surfactant is responsible for lowering the surface tension of the liquid, enables the formation of a stable nanoemulsion, slows down the ageing processes e.i. Ostwald ripening |
| | Cosurfactant: perfluorinated: (1H, 1H, 2H, 2H-Perfluorooctyl)phosphocholine or (1H, 1H, 2H, 2H-Perfluorooctyl)-β-D-Maltopyranoside or modified lipids: 1,2-Dioleoyl-sn-glycero-3 -phospho-L-serine sodium salt or 1,2-dipalmitoyl-sn-glycero-3 -phosphate sodium salt or 1,2-dimyristoyl-sn-glycero-3 -phospho-rac-glycerol sodium salt or N-(carbonyl-methoxy polyethylene glycol-2000)-1,2-distearoyl-sn-glycero-3-phosphoethanolamine sodium salt | 3.5-7.0 preferably 3.5-5.0 preferably 4.0-5.0 | g/L | |
| Oncotic factor, single compound | Bovine/human serum albumin or PEG 35 kDa or PEG 20 kDa or dextran 70 kDa or dextran 40 kDa or succinylated gelatin or hydroxyethylated starch | 20.0-70.0 favourably 40.0 | g/L | Substances that regulate oncotic pressure, preventing organ swelling during perfusion |
| Main osmotic agent | mannitol | 10.0-100.0 favourably 21.4-50.0 | mM | The main osmotic agent, in addition to preventing cell swelling, protects against the formation of reactive oxygen species (antioxidant) |

(continued)

| Individual component groups | Examples of compounds to be claimed in the present invention | Concentration | Units | Benefits of the perfusion fluid |
|---|---|---|---|---|
| Basic buffer components | D-glucose · $H_2O$ | 5.00-20.00 favourably 10.00 | mM | Main source of energy for cells |
| | sodium salt of DL-β-hydroxybutyric acid | 0.10-0.50 favourably 0.20 | mM | Energy source for cells |
| | glutathione | 1.50-6.00 favourably 3.00 | mM | Main antioxidant - antioxidant (prevents the formation of free radicals), in addition to being an important substrate and cofactor in many metabolic reactions |
| | allopurinol | 0.50-3.00 favourably 1.00 | mM | Xanthine oxidase inhibitor, antioxidant |
| Buffer components - Amino acids | taurine | 5.00-15.00 favourably 10.00 | mM | Amino acids have various functions including.: stimulation of nitric oxide synthesis, prevention of epithelial cell damage, antioxidants, regulation of osmotic pressure, anti-inflammatory and anti-apoptotic |
| | L-arginine | 0.05-0.20 favourably 0.10 | mM | |
| | L-tryptophan | 1.00-3.00 favourably 2.00 | mM | |
| | L-glutamine | 0.10-1.00 favourably 0.50 | mM | |
| | L-isoleucine | 0.50-10.00 favourably 1.52-4.19 | mM | |

(continued)

| Individual component groups | Examples of compounds to be claimed in the present invention | Concentration | Units | Benefits of the perfusion fluid |
|---|---|---|---|---|
| | L-leucine | 1.00-10.00 favourably 3.05-6.86 | mM | effects, protection of cells against lipid peroxidation and harmful changes in membrane structure, substrates for protein synthesis and coenzymes, participation in protein synthesis, role in nitrogen metabolism and in the synthesis of purines, pyrimidines and other amino acids, a mixture of compounds |
| | L-lysine | 1.00-10.00 favourably 2.05-4.79 | mM | |
| | L-methionine | 0.50-10.00 favourably 1.34-3.35 | mM | |
| | L-threonine | 0.50-10.00 favourably 1.69-3.78 | mM | |
| | L-valine | 0.50-10.00 favourably 2.56-5.55 | mM | |
| | L-histidine | 0.10-10.00 favourably 0.64-2.26 | mM | |
| | L-alanine | 1.00-20.00 favourably 5.61-12.35 | mM | |
| | L-glycine | 1.00-20.00 favourably 6.66-17.32 | mM | |
| | L-aspartic acid | 0.50-10.00 favourably 1.88-4.51 | mM | |
| | L-proline | 0.50-10.00 | mM | |
| | | favourably 2.17-5.21 | | |
| | L-serine | 0.10-10.00 favourably 0.95-2.38 | mM | |
| | L-tyrosine | 0.01-1.00 favourably 0.06-0.28 | mM | |

(continued)

| Individual component groups | Examples of compounds to be claimed in the present invention | Concentration | Units | Benefits of the perfusion fluid |
|---|---|---|---|---|
| Buffer components and electrolytes | $Na_2HPO_4$ | 6.00-15.00 favourably 8.00 | mM | Buffering components and electrolytes, affect the osmotic pressure of the perfusion fluid, a mixture of compounds |
| | $NaH_2PO_4$ | 0.50-3.00 favourably 1.50 | mM | |
| | NaCl | 20.00-100.00 favourably 60.00 | mM | Inorganic salts, containing the most important ions needed by the organism (sodium, potassium, calcium, magnesium and chlorine), also affect the osmotic pressure of the perfusion fluid, a mixture of compounds |
| | KCl | 2.00-10.00 favourably 4.60 | mM | |
| | $CaCl_2$ | 0.30-2.00 favourably 0.60 | mM | |
| | $MgCl_2 \cdot 6H_2O$ | 0.20-2.00 favourably 0.40 | mM | |
| | NaOH | 0.50-20.00 favourably 1.4-14.00 | mM | |

Table 2 shows the parameters characterising the perfusion fluid according to the invention. **Table 2.** Parameters characterising perfusion fluid

| Test parameter | Acceptance criteria | |
|---|---|---|
| Size distribution | Average diameter | <200-300 nm |
| | PdI | <0.2 |
| Zeta potential | $\neq 0$ mV | |
| Osmolality | 280 - 400 mOsm/kgH$_2$O | |
| pH | 7.1-7.6 | |
| Concentration of selected ions | Na$^+$ [mM]: 80-200 | |
| | K$^+$ [mM]: 1-15 | |
| | Ca$^{2+}$ [mM]: 0.1-2.5 | |
| | Cl$^-$ [mM]: 50-170 | |
| Oncotic pressure | For blood: 15-36 mmHg | |
| Cytotoxicity | Cell viability [%] | Liquid concentration in culture medium [%]: 50 |
| | $\geq 70\%$ | |

The perfusion fluid produced according to the manufacturing methods in question makes it possible to obtain nanoemulsions with the parameters shown in Table 2.

The method of producing the perfusion fluid according to this invention, consisting of a combination of mechanical homogenization techniques and microfluidization of a PFC mixture of suitable composition, makes it possible to obtain stable nanoparticles with a size below 300 nm, preferably below 220 nm, most preferably below 130 nm.

The key steps in the production of perfusion fluid are, according to mode I of production: mechanical homogenization and microfluidization, and according to mode II of production: mechanical homogenization and microfluidization of the pre-emulsion and microfluidization of the primary perfusion fluid.

The size of the PFC nanoparticles obtained in the perfusion fluid and the stability of the perfusion fluid determine the microfluidization step, which uses a pump to generate high pressure to break up the PFC mixture by shear forces. The PFC suspension is fed into the inlet and then pumped at high pressure through the microchannels of a Y-type diamond chamber, resulting in a high shear rate and ultimately breakdown into nanoparticles. One of the main advantages of microfluidization is the high reproducibility of the process due to the fixed geometry and easy scalability.

There is a sterilizing filtration step in the perfusion fluid manufacturing process (Methods I and II), which can be replaced by realising the process under sterile conditions or realising the sterilization of the perfusion fluid by other commercial sterilization methods.

In the examples of the implementation of the invention, it has been confirmed that the perfusion fluid_02 and the perfusion fluid _04 obtained according to production methods **I** and **II** have the same parameters. Therefore, both methods are equally effective and lead to a perfusion fluid.

All perfusion fluid formulations, prepared according to the invention, are characterised by physicochemical properties similar to human blood, in terms of: osmotic pressure (in the range: 280-400 mOsm/kgH$_2$O), pH (7.1-7.6), oncotic pressure (15-36 mmHg), concentration of the main ions: sodium (80-200 mM), potassium (1-15 mM), calcium (0.1-2.5 mM) and chlorine (50-170 mM). Furthermore, the average diameter of the nanoparticles of the dispersed (perfluorinated) phase of the present invention does not exceed 300 nm, and the homogeneity of the described emulsions, expressed by the polydispersity index (PdI), does not exceed 0.1 (over a period of 3 months after manufacture). In addition, the zeta potential value of the nanoparticles is different from zero, and is at least: -5 mV or +5mV, which prevents coalescence and aggregation of the particles of the dispersed phase of the emulsion. The perfusion fluid formulations presented in the present invention, are nontoxic towards L929 mouse fibroblast cells, as indicated by a cell viability above 70%, for a fluid concentration in the culture medium equal to 50%.

The above-mentioned composition of the perfusion fluid according to the invention, enables the use of warm machine perfusion technology to improve the storage conditions of organs for transplantation. Existing procedures for storing an organ in a static cold room on ice after rinsing with crystalline solutions, such as the University of Wisconsin solution or Custodiol, do not completely stop cellular metabolism in organs; they only lead to a slowing down of enzymatic reactions and a delay in cell death. In contrast, the use of perfusion fluid in warm ex vivo machine perfusion of an organ allows the organ to be maintained under full metabolic conditions, with the direct result that the organ is in better condition and the organ can be stored for a longer period of time (up to about 30h). In addition, the perfusion fluid according to the invention used for ex vivo machine perfusion of organs intended for transplantation, i.e. donations of organs after brain death - DBD and organs taken from donors after irreversible cardiac arrest - DCD or donors aged 60 and over and donors aged 50-59 with at least two of the following risk factors: arterial hypertension, death caused by stroke, blood creatinine level at the time of death above 1.5 mg/dL - ECD, enables better storage and reconditioning of the organ before transplantation.

The above is confirmed by the perfusion results of isolated organs damaged during controlled DCD, autotransplantation results and proteomic-metabolomic results.

The above-mentioned composition of the perfusion fluid according to the invention enables warm ex vivo machine perfusion of organs, i.e. kidney, liver, heart, as confirmed in the examples.

The above composition of the perfusion fluid allows it to be used in the resuscitation process after haemorrhagic shock as a blood substitute, as confirmed in the examples.

**Examples of the implementation of the invention**

The following examples are included only to illustrate the invention and to clarify particular aspects of the invention, not to limit the invention, and should not be equated with the entire scope of the invention as defined in the appended claims. In the following examples, unless otherwise indicated, standard materials and methods used in the field have been used or manufacturers' recommendations for specific reagents and methods have been followed.

**Example I: Preparation of PFC nanoemulsions (organ perfusion fluids):**

**Example I.1. Obtaining perfusion fluid_02_II production method**

**Table 3.** Quantitative composition of the perfusion fluid_02_II production method.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |

(continued)

| Component | CAS | Concentration | Units |
|---|---|---|---|
| Bovine serum albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 · 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

The method for obtaining the perfusion fluid involves the formation of a pre-emulsion from a surfactant solution and a perfluorinated phase.

For this purpose, the components (surfactant solution and perfluorinated phase, in the block diagram for obtaining perfusion fluid - **Fig. 2.,** marked with No. **(I))** were prepared by weighing them on an analytical balance.

The surfactant solution was obtained by dissolving the surfactants in ultrapure water in two beakers: a volume of 500 mL (amounts: 58.2857 g Pluronic F68 and 8.5714 g (1H, 1H, 2H, 2H-Perfluorooctyl)phosphocholine) and a volume of 200 mL (amounts: 23.3143 g Pluronic F68 and 3.4286 g (1H, 1H, 2H, 2H-Perfluorooctyl)phosphocholine. The solutions were stirred on a magnetic stirrer at room temperature for about 24 hours. Then, the clear solutions were transferred to two volumetric flasks (500 mL and 200 mL) and made up to volume with ultrapure water. The two solutions were combined to give a 700 mL solution of the surfactants mixture.

The perfluorinated phase, i.e. a mixture of perfluorocarbons: perfluorooctyl bromide with perfluorodecyl bromide, was prepared by dissolving 17.37 g of perfluorodecyl bromide in 561.63 g of perfluorooctyl bromide, (mechanical stirring, at room temperature, approximately 0.25 hours).

This yielded approximately 300 mL of perfluorinated phase, which was combined with 700 mL of aqueous phase (surfactanst solution) by mechanical homogenization (primary pre-emulsification) **(II).** A PRO25D mechanical homogenizer with a 20 × 115 mm homogenizing tip, PRO Scientific Inc., USA, was used. Process parameters: the speed of the homogenizer motor was approximately 12 000 rpm, process duration - 1 min homogenization process: 1 minute interval/100 mL primary pre-emulsion. After approximately 20 minutes, the primary pre-emulsification process was completed.

The next step was a primary pre-emulsion **(III)** microfluidization process, carried out using a high-pressure homogenizer: Microfluidizer® LM20 processor, Microfluidics (IDEX Health & Science, LLC), Canada. The primary pre-emulsion was pressed at 2065 bar through an F12Y type diamond chamber cooled by a water bath. The number of times the total volume of emulsion was forced through the microfluidizer chamber was 8.

The final sub-step was the preparation of a 2 L buffer by weighing and dissolving the ingredients in ultrapure water **(IVa),** according to the following order (the order of addition of the ingredients being irrelevant): 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1.0288 g KCl, 3.4070 g $Na_2HPO_4$, 0.5399 g $NaH_2PO_4$, 0.1998 g $CaCl_2$, 0.2440 g $MgCl_2$ · $6H_2O$, 0.2192 g L-glutamine, 2.7660 g glutathione, 0.0757 g DL-β-hydroxybutyric acid sodium. The buffer was stirred on a magnetic stirrer using 350 rpm at 37° C for approximately 2 hours. Once all components were dissolved, pH adjustment (correction/elevation with 5 M NaOH to 7.4) was performed **(IVR).** Then, the solution was placed in a 2 L volumetric flask and topped up with an appropriate amount of ultrapure water. At this stage, a preliminary quality control of the resulting buffer **(IVQ)** was also performed, including: measurement of pH, osmotic pressure and ion concentration - sodium, potassium, chlorine and calcium.

The primary perfusion fluid is formed when the pre-emulsion and the buffer are mixed in the correct proportions **(2).** A measuring cylinder was used to measure the volume of the pre-emulsion and the buffer in the ratio: 1:2 (for 1 L of the pre-emulsion, 2 L of the buffer was added). The preparation was stirred at 250 rpm, at room temperature, for about one hour, on a magnetic stirrer. Samples were then taken for pH and osmolality and ion concentration testing **(2R),** if measurements indicated inadequate pH, it was adjusted by the addition of an alkali solution (5M NaOH) or acid (1M HCl), to a value of 7.4 at T = 21-23° **C (2R)** Special attention was paid to ensure that the osmolality of the preparation did not exceed 400 mOsmol/kg$H_2$O, and that the ion concentration was in the range: 80-200 mmol/L for Na$^+$, 1-15 mmol/L for K$^+$, 0.1-2.5 mmol/L for Ca$^{2+}$, 50-170 mmol/L for Cl.$^-$

The obtained primary perfusion fluid was microfluidized (final microfluidization) **(3)** using a high-pressure homogenizer: Microfluidizer® LM20 processor, Microfluidics (IDEX Health & Science, LLC), Canada. The primary perfusion fluid was pressed once at a pressure of 2065 bar through a type F 12Y diamond chamber cooled by a water bath.

The sterilizing filtration stage **(4)** took place under sterile conditions (under a chamber with laminar air flow, equipped with HEPA filters). The filtration process was carried out to get rid of possible contaminants from mixing and microfluidization (metal filings, fragments of equipment seals) and microbial infections. For sterilization by this method, off-the-shelf filter sets were used, consisting of a funnel with a 0.22 μm pore diameter membrane and a 250-500 mL receiver, MerckMillipore, USA. Perfusion fluid was placed on the filters (6 sets of 500 mL each were used) and passed through the membrane pore using a vacuum pump into sterile containers.

After the perfusion fluid was cleared of particulates and microorganisms by means of a filtration process, the next step was to transfer the obtained perfusion fluid from the intermediate package, into a sterile target package under a laminar chamber **(5).** The resulting perfusion fluid_02_II production method was bottled into sterile 1000 mL, 500 mL, 250 mL or 100 mL plastic bottles fitted with septa stoppers, Nalgene, Thermo Fischer, or into 10 mL, 20 mL, 50 mL or 100 mL glass vials capped with aluminium septa caps. Corresponding volumes of perfusion fluids were measured using graduated cylinders or an automatic pipettor. Each bottle was labelled with the batch number, date of manufacture and storage conditions. In addition, samples were also spilled for perfusion fluid quality control tests **(SQ):** physicochemical tests (measurement of particle size distribution, zeta potential, osmolality, pH, concentration of selected ions, viscosity, oncotic pressure, biological tests (cytotoxicity).

**Example I.2. Obtaining perfusion fluid_04_II production method**

**Table 4.** Quantitative composition of perfusion fluid_04_II production method.

| Component | CAS | Concentration | Units |
|---|---|---|---|
| Perfluorooctyl bromide (PFOB) | 423-55-2 | 374.42 | g/L |
| Perfluorodecyl bromide (PFDB) | 307-43-7 | 11.58 | g/L |
| Pluronic F68 (Kolliphor P188) | 9003-11-6 | 34.00 | g/L |
| (1H, 1H, 2H, 2H-Perfluorooctyl)phosphocholine | 313997-22-7 | 5.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 21.40 | mM |
| Taurine | 107-35-7 | 10.00 | mM |
| L-tryptophan | 73-22-3 | 2.00 | mM |
| D-glucose · $H_2$O | 14431-43-7 | 10.00 | mM |
| NaCl | 7647-14-5 | 60.00 | mM |
| KCl | 7447-40-7 | 4.60 | mM |

(continued)

| Component | CAS | Concentration | Units |
|---|---|---|---|
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 \cdot 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

The perfusion fluid_04_II production method was obtained according to the method of obtaining the perfusion fluid_02_ II production method described in example I.1.

The surfactants solution was obtained by dissolving the surfactants in ultrapure water in three beakers: one with a volume of 500 mL (amounts: 56.6667 g Pluronik F68 and 8.3333 g (1H, 1H, 2H, 2H-Perfluorooctyl)phosphocholine and two with a volume of 200 mL each (amounts: 22.6667 g Pluronik F68 and 3.3333 g (1H, 1H, 2H, 2H-Perfluorooctyl) phosphocholine.

A mixture of perfluorocarbons: perfluorooctyl bromide with perfluorodecyl bromide, was prepared by dissolving 34.74 g of perfluorodecyl bromide in 11,232.60 g of perfluorooctyl bromide, (mechanical stirring, at room temperature, approximately 0.25 hours).

This yielded approximately 600 mL of perfluorinated phase, which was combined with 900 mL of aqueous phase (surfactants solution) by mechanical homogenization (primary pre-emulsification) **(II).** A PRO25D mechanical homogenizer with a $20 \times 115$ mm homogenizing tip, PRO Scientific Inc., USA, was used. Process parameters: the speed of the homogenizer motor was approximately 12 000 rpm, process duration - 1 min homogenization process: 1 minute interval/100 mL primary pre-emulsion. After approximately 30 minutes, the primary pre-emulsification process was completed.

The primary pre-emulsion microfluidization process **(III)** was carried out according to the perfusion fluid_02_II production method, as described in example I.**1**

The buffer was obtained by weighing and dissolving the ingredients in ultrapure water, using mechanical stirring **(IV)**, according to the following order (whereby the order of addition of the ingredients does not matter): 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose $\cdot$ $H_2O$, 10.5192 g NaCl, 1.0288 g KCl, 3.4070 g $Na_2HPO_4$, 0.5399 g $NaH_2PO_4$, 0.1998 g $CaCl_2$, 0.2440 g $MgCl_2 \cdot 6H_2O$, 0.2192 g L-glutamine, 2.7660 g glutathione, 0.0757 g DL-β-hydroxybutyric acid sodium.

According to the perfusion fluid_02_II production method, a primary perfusion fluid was obtained, except that the ratio of the pre-emulsion to the buffer was 1:1 (for 1.5 L of the pre-emulsion, 1.5 L of the buffer was added).

The obtained primary perfusion fluid was microfluidized (final microfluidization) **(3)** using a high-pressure homogenizer: Microfluidizer® LM20 processor, Microfluidics (IDEX Health & Science, LLC), Canada. The primary perfusion fluid was pressed five times at a pressure of 2065 bar through an F12Y type diamond chamber, cooled by a water bath.

The steps of sterilizing filtration **(4)** and confectioning **(5)** were carried out according to the method for obtaining perfusion fluid_02_ II production method, described in example I.1. Perfusion fluid_04_II production method was obtained.

**Example 1.3 Obtaining perfusion fluid_01 _I method of production** (hereafter referred to as **perfusion fluid_01**)

**Table 5.** Quantitative composition of perfusion fluid_01.

| Component | CAS | Concentration | Units |
|---|---|---|---|
| Perfluorooctyl bromide (PFOB) | 423-55-2 | 93.61 | g/L |
| Perfluorodecyl bromide (PFDB) | 307-43-7 | 2.90 | g/L |
| Pluronic F68 (Kolliphor P188) | 9003-11-6 | 23.80 | g/L |

(continued)

| Component | CAS | Concentration | Units |
|---|---|---|---|
| (1H, 1H, 2H, 2H-Perfluorooctyl)phosphocholine | 313997-22-7 | 3.50 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 · 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

The following method of obtaining perfusion fluid is shown in Fig. 1.

The surfactants solution was prepared by weighing 116.6000 g of Pluronic F68 and 17.1400 g of (1H, 1H, 2H, 2H-Perfluorooctyl)phosphocholine and dissolving in ultrapure water. The solution was stirred on a magnetic stirrer at room temperature for about 24 h. The clear solution of the surfactants mixture was then transferred to a 1000 mL volumetric flask and made up to volume with ultrapure water.

The perfluorinated phase was prepared by weighing 30.9278 g of perfluorodecyl bromide in 1000 g of perfluorooctyl bromide and stirring mechanically at room temperature for approximately 0.25 h.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2 · 6H_2O$, 0.2192 g L-glutamine, 2.7660 g glutathione, 0.0757 g DL-β-hydroxybutyric acid sodium salt and dissolving the components in ultrapure water. The buffer was stirred on a magnetic stirrer using 350 rpm at 37 °C for approximately 2 h. Once all the ingredients were dissolved, pH adjustment was performed (correction/elevation with 5 M NaOH to a value of 7.4). The solution was then transferred to a 2 L volumetric flask and topped up with an appropriate amount of ultrapure water. A preliminary quality control of the resulting buffer was also performed, including: measurement of pH, osmotic pressure and ion concentration - sodium, potassium, chlorine and calcium.

The primary perfusion fluid was prepared by dispensing appropriate volumes of 40.84 mL of the surfactants solution, 133.33 mL of the buffer, 15.83 mL of the ultrapure water and 10 mL of the perfluorocarbons mixture into a 250 mL beaker using graduated cylinders and automatic pipettes. The mixture was emulsified by mechanical homogenization, and 200 mL of the primary perfusion fluid was obtained **(2).** A PRO25D mechanical homogenizer with a 20 × 115 mm homogenizing tip, PRO Scientific Inc., USA, was used. Process parameters: the motor speed of the homogenizer was approximately 12000 rpm, the process duration was 1 min of homogenization: 1 minute interval/100 mL emulsion. The homogenization process was carried out for approximately 4 minutes.

In the next step, microfluidization of the primary perfusion fluid **(3)** was carried out using a high-pressure homogenizer: Microfluidizer® LM20 processor, Microfluidics (IDEX Health & Science, LLC), Canada. The primary perfusion fluid was pressurized at 2065 bar through an F12Y type diamond chamber cooled by a water bath. The number of times the total volume of emulsion was forced through the microfluidizer chamber was 9, thus obtaining the perfusion fluid. Samples were taken for pH and osmolality testing, as well as ion concentration; if the measurements indicated inadequate pH, it was adjusted by the addition of a solution of alkali (5M NaOH) or acid (1M HCl), to a value of 7.4 at T = 21-23° C. The osmolality of the preparation was tested (not to exceed 400 mOsmol/kgH$_2$O), and the ion concentration was tested (concentration in the range: 80 - 200 mmol/L for Na$^+$, 1-15 mmol/L for K$^+$, 0.1-2.5 mmol/L for Ca$^{2+}$, 50-170 mmol/L for Cl$^-$).

The perfusion fluid was subjected to sterilizing filtration **(4),** carried out under sterile conditions (under a laminar airflow chamber equipped with HEPA filters). The filtration process is designed to remove possible contaminants generated after mixing and microfluidization (metal filings, fragments of device seals) and microbial infections. For sterilization by this method, off-the-shelf filter kits were used, consisting of a funnel with a 0.22 $\mu$m pore diameter membrane and a 250 mL receiver, MerckMillipore, USA. Perfusion fluid was placed on the filter and filtered using a vacuum pump into a sterile container.

The obtained perfusion fluid was then transferred from the intermediate pack to the sterile target pack under the laminar chamber **(5).** The fluid was bottled into sterile 250 mL or 100 mL plastic bottles fitted with septa stoppers, Nalgene, Thermo Fischer. Each bottle was labelled with batch number, date of manufacture and storage conditions. In addition, samples were also spilled for perfusion fluid quality control tests **(5Q):** physicochemical tests (measurement of particle size distribution, zeta potential, osmolality, pH, concentration of selected ions, viscosity, oncotic pressure, biological tests (cytotoxicity).

### Example I.4 Obtaining perfusion fluid_02

**Table 6.** Quantitative composition of perfusion fluid_02.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F68 (Kolliphor P188) | 9003-11-6 | 27 20 | g/L |
| (1H, 1H, 2H, 2H-Perfluorooctyl)phosphocholine | 313997-22-7 | 4.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · H$_2$O | 14431-43-7 | 10.00 | mM |
| NaCl | 7647-14-5 | 60.00 | mM |
| KCl | 7447-40-7 | 4.60 | mM |
| Na$_2$HPO$_4$ | 7558-79-4 | 8.00 | mM |
| NaH$_2$PO$_4$ | 7558-80-7 | 1.50 | mM |
| CaCl$_2$ | 10043-52-4 | 0.60 | mM |
| MgCl$_2$ · 6H$_2$O | 7791-18-6 | 0.40 | mM |
| L-glutamine | 56-85-9 | 0.50 | mM |
| Glutathione | 70-18-8 | 3.00 | mM |
| DL-$\beta$-hydroxybutyric acid sodium salt | 150-83-4 | 0.20 | mM |
| NaOH | 1310-73-2 | to determine the pH | |

Perfusion fluid_02 was obtained according to the method for obtaining perfusion fluid_01, described in Example I.3.

The surfactants solution was prepared by dissolving 116.6000 g of Pluronic F68 and 17.1400 g of (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1.0288 g KCl, 3.4070 g $Na_2HPO_4$, 0.5399 g $NaH_2PO_4$, 0.1998 g $CaCl_2$, 0.2440 g $MgCl_2$ · $6H_2O$, 0.2192 g L-glutamine, 2.7660 g glutathione, 0.0757 g DL-β-hydroxybutyric acid sodium salt and dissolving the ingredients in ultrapure water.

A total of 46.67 mL of the surfactants solution, 133.33 mL of the buffer and 20 mL of the perfluorocarbons mixture were combined to obtain 200 mL of the primary perfusion fluid, which was homogenized and microfluidized by running 9 repetitions of pumping the total emulsion volume through the microfluidizer chamber. After the sterilizing filtration, perfusion fluid_02 was obtained.

## Example I.5 Obtaining perfusion fluid_03

Table 7. Quantitative composition of perfusion fluid_03.

| Component | CAS | Concentration | Units |
|---|---|---|---|
| Perfluorooctyl bromide (PFOB) | 423-55-2 | 280.82 | g/L |
| Perfluorodecyl bromide (PFDB) | 307-43-7 | 8.69 | g/L |
| Pluronic F68 (Kolliphor P188) | 9003-11-6 | 30.60 | g/L |
| (1H, 1H, 2H, 2H-Perfluorooctyl)phosphocholine | 313997-22-7 | 4.50 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 21.40 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2$ · $6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_03 was obtained according to the method for obtaining perfusion fluid_01, described in Example I.3.

The surfactants solution was prepared by dissolving 116.6000 g of Pluronik F68 and 17.1400 g of (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 160.0000 g bovine serum albumin, 0.0697 g L-arginine, 0.5445 g allopurinol, 15.6229 g mannitol, 5.0080 g taurine, 1.6338 g L-tryptophan, 7.9268 g D-glucose · $H_2O$, 14.0256 g NaCl, 1,3717 g KCl, 4.5427 g $Na_2HPO_4$, 0.7199 g $NaH_2PO_4$, 0.2664 g $CaCl_2$, 0.3253 g $MgCl_2 \cdot 6H_2O$, 0.2923 g L-glutamine, 3.6880 g glutathione, 0.1009 g DL-β-hydroxybutyric acid sodium salt and dissolving the ingredients in ultrapure water.

A total of 52.51 mL of the surfactants solution, 100 mL of the buffer, 17.49 mL of the ultrapure water and 30 mL of the perfluorocarbons mixture were combined to obtain 200 mL of the primary perfusion fluid, which was homogenized and microfluidized by conducting 9 repetitions of pumping the total emulsion volume through the microfluidizer chamber. After the sterilizing filtration, perfusion fluid_03 was obtained.

## Example I.6 Obtaining perfusion fluid_04

**Table 8.** Quantitative composition of perfusion fluid_04.

| Component | CAS | Concentration | Units |
|---|---|---|---|
| Perfluorooctyl bromide (PFOB) | 423-55-2 | 374.42 | g/L |
| Perfluorodecyl bromide (PFDB) | 307-43-7 | 11.58 | g/L |
| Pluronic F68 (Kolliphor P188) | 9003-11-6 | 34.00 | g/L |
| (1H, 1H, 2H, 2H-Perfluorooctyl)phosphocholine | 313997-22-7 | 5.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 21.40 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60,00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 \cdot 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_04 was obtained according to the method for obtaining perfusion fluid_01, described in Example I.3.

The surfactants solution was prepared by dissolving 116.6000 g of Pluronic F68 and 17.1400 g of (1H, 1H, 2H, 2H-Perfluorooctyl)phosphocholine in ultrapure water.

The perfluorinated phase was prepared by dissolving 30.9278 g of perfluorodecyl bromide and 1000 g of perfluorooctyl bromide.

The buffer was prepared by weighing 160.0000 g bovine serum albumin, 0.0697 g L-arginine, 0.5445 g allopurinol, 15.6229 g mannitol, 5.0080 g taurine, 1.6338 g L-tryptophan, 7.9268 g D-glucose · $H_2O$, 14.0256 g NaCl, 1,3717 g KCl , 4.5427 g $Na_2HPO_4$, 0.7199 g $NaH_2PO_4$, 0.2664 g $CaCl_2$, 0.3253 g $MgCl_2 \cdot 6H_2O$, 0.2923 g L-glutamine, 3.6880 g glutathione, 0.1009 g DL-β-hydroxybutyric acid sodium salt and dissolving the ingredients in ultrapure water.

A total of 58.34 mL of the surfactants solution, 100 mL of the buffer, 1.66 mL of the ultrapure water and 40 mL of the perfluorocarbons mixture were combined to obtain 200 mL of the primary perfusion fluid, which was homogenized and microfluidized by conducting 9 repetitions of pumping the total emulsion volume through the microfluidizer chamber. After the sterilizing filtration, perfusion fluid_04 was obtained.

## Example 1.7 Obtaining perfusion fluid_05

**Table** 9. Quantitative composition of perfusion fluid_05.

| Component | CAS | Concentration | Units |
|---|---|---|---|
| Perfluorooctyl bromide (PFOB) | 423-55-2 | 468.03 | g/L |
| Perfluorodecyl bromide (PFDB) | 307-43-7 | 14.48 | g/L |
| Pluronic F68 (Kolliphor P188) | 9003-11-6 | 37.40 | g/L |
| (1H, 1H, 2H, 2H-Perfluorooctyl)phosphocholine | 313997-22-7 | 5.50 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 21.40 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 · 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_05 was obtained according to the method for obtaining perfusion fluid_01, described in Example I.3.

The surfactants solution was prepared by dissolving 47.6000 g of Pluronik F68 and 7.0000 g of (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 160.0000 g bovine serum albumin, 0.0697 g L-arginine, 0.5445 g allopurinol, 15.6229 g mannitol, 5.0080 g taurine, 1.6338 g L-tryptophan, 7.9268 g D-glucose · $H_2O$, 14.0256 g NaCl, 1,3717 g KCl, 4.5427 g $Na_2HPO_4$, 0.7199 g $NaH_2PO_4$, 0.2664 g $CaCl_2$, 0.3253 g $MgCl_2 · 6H_2O$, 0.2923 g L-glutamine, 3.6880 g glutathione, 0.1009 g DL-β-hydroxybutyric acid sodium salt and dissolving the ingredients in ultrapure water.

15.71 mL of the surfactants solution, 100 mL of the buffer, 34.29 mL of the ultrapure water and 50 mL of the perfluorocarbons mixture were combined to obtain 200 mL of the primary perfusion fluid, which was homogenized and microfluidized by running 14 repetitions of pumping the total volume of emulsion through the microfluidizer chamber. After the sterilizng filtration, perfusion fluid_05 was obtained.

**Example I.8 Obtaining perfusion fluid_06**

Table 10. Quantitative composition of perfusion fluid_06.

| Component | CAS | Concentration | Units |
|---|---|---|---|
| Perfluorooctyl bromide (PFOB) | 423-55-2 | 561.63 | g/L |
| Perfluorodecyl bromide (PFDB) | 307-43-7 | 17.37 | g/L |
| Pluronic F68 (Kolliphor P188) | 9003-11-6 | 40.80 | g/L |
| (1H, 1H, 2H, 2H-Perfluorooctyl)phosphocholine | 313997-22-7 | 6.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 21.40 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2$ · $6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid 06 was obtained according to the method for obtaining perfusion fluid_01, described in Example I.3.

The surfactants solution was prepared by dissolving 47.6000 g of Pluronic F68 and 7.0000 g of (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 160.0000 g bovine serum albumin, 0.0697 g L-arginine, 0.5445 g allopurinol, 15.6229 g mannitol, 5.0080 g taurine, 1.6338 g L-tryptophan, 7.9268 g D-glucose · $H_2O$, 14.0256 g NaCl, 1,3717 g KCl , 4.5427 g $Na_2HPO_4$, 0.7199 g $NaH_2PO_4$, 0.2664 g $CaCl_2$, 0.3253 g $MgCl_2$ · $6H_2O$, 0.2923 g L-glutamine, 3.6880 g glutathione, 0.1009 g DL-β-hydroxybutyric acid sodium salt and dissolving the ingredients in ultrapure water.

A total of 17.14 mL of the surfactants solution, 100 mL of the buffer, 22.86 mL of the ultrapure water and 60 mL of the perfluorocarbons mixture were combined to obtain 200 mL of the primary perfusion fluid, which was homogenized and microfluidized by conducting 14 repetitions of pumping the total emulsion volume through the microfluidizer chamber. After the sterilizng filtration, perfusion fluid_06 was obtained.

**Example I.9 Obtaining perfusion fluid_07**

Table 11. Quantitative composition of perfusion fluid_07.

| Component | CAS | Concentration | Units |
|---|---|---|---|
| Perfluorooctyl bromide (PFOB) | 423-55-2 | 655.24 | g/L |
| Perfluorodecyl bromide (PFDB) | 307-43-7 | 20.27 | g/L |
| Pluronic F68 (Kolliphor P188) | 9003-11-6 | 44.20 | g/L |
| (1H, 1H, 2H, 2H-Perfluorooctyl)phosphocholine | 313997-22-7 | 6.50 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 21.40 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 · 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid 07 was obtained according to the method for obtaining perfusion fluid_01, described in Example I.3.

The surfactants solution was prepared by dissolving 47.6000 g Pluronic F68 and 7.0000 g (1H, 2H, 2H- (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 160.0000 g bovine serum albumin, 0.0697 g L-arginine, 0.5445 g allopurinol, 15.6229 g mannitol, 5.0080 g taurine, 1.6338 g L-tryptophan, 7.9268 g D-glucose · $H_2O$, 14.0256 g NaCl, 1,3717 g KCl, 4.5427 g $Na_2HPO_4$, 0.7199 g $NaH_2PO_4$, 0.2664 g $CaCl_2$, 0.3253 g $MgCl_2 · 6H_2O$, 0.2923 g L-glutamine, 3.6880 g glutathione, 0.1009 g DL-β-hydroxybutyric acid sodium salt and dissolving the ingredients in ultrapure water.

18.57 mL of the surfactants solution, 100 mL of the buffer, 11.43 mL of the ultrapure water and 70 mL of the perfluorocarbons mixture were combined to obtain 200 mL of the primary perfusion fluid, which was homogenized and microfluidized by conducting 14 repetitions of pumping the total emulsion volume through the microfluidizer chamber. After the sterilizng filtration, perfusion fluid_07 was obtained.

**Example I.10 Obtaining perfusion fluid_08**

Table 12. Quantitative composition of perfusion fluid_08.

| Component | CAS | Concentration | Units |
|---|---|---|---|
| Perfluorooctyl bromide (PFOB) | 423-55-2 | 748.84 | g/L |
| Perfluorodecyl bromide (PFDB) | 307-43-7 | 23.16 | g/L |
| Pluronic F68 (Kolliphor P188) | 9003-11-6 | 47.60 | g/L |
| (1H, 1H, 2H, 2H-Perfluorooctyl)phosphocholine | 313997-22-7 | 7.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 21.40 | mM |
| Taurine | 107-35-7 | 10.00 | mM |
| L-tryptophan | 73-22-3 | 2.00 | mM |
| D-glucose $\cdot$ $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 \cdot 6H_2O$ | 7791-18-6 | 0.40 | mM |
| L-glutamine | 56-85-9 | 0.50 | mM |
| Glutathione | 70-18-8 | 3.00 | mM |
| DL-$\beta$-hydroxybutyric acid sodium salt | 150-83-4 | 0.20 | mM |
| NaOH | 1310-73-2 | to determine the pH | |

Perfusion fluid_08 was obtained according to the method for obtaining perfusion fluid_01, described in Example I.3.

The surfactants solution was prepared by dissolving 47.6000 g Pluronic F68 and 7.0000 g (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 160.0000 g bovine serum albumin, 0.0697 g L-arginine, 0.5445 g allopurinol, 15.6229 g mannitol, 5.0080 g taurine, 1.6338 g L-tryptophan, 7.9268 g D-glucose $\cdot$ $H_2O$, 14.0256 g NaCl, 1,3717 g KCl, 4.5427 g $Na_2HPO_4$, 0.7199 g $NaH_2PO_4$, 0.2664 g $CaCl_2$, 0.3253 g $MgCl_2 \cdot 6H_2O$, 0.2923 g L-glutamine, 3.6880 g glutathione, 0.1009 g DL-$\beta$-hydroxybutyric acid sodium salt and dissolving the ingredients in ultrapure water.

20 mL of the surfactants solution, 100 mL of the buffer and 80 mL of the perfluorocarbons mixture were combined to obtain 200 mL of the primary perfusion fluid, which was homogenized and microfluidized by running 14 repetitions of pumping the total emulsion volume through the microfluidizer chamber. After the sterilizing filtration, perfusion fluid_08 was obtained.

**Example I.11 Obtaining perfusion fluid_09**

Table 13. Quantitative composition of perfusion fluid_09.

| Component | CAS | Concentration | Units |
|---|---|---|---|
| Perfluorodecalin (PFD) | 306-94-5 | 192.00 | g/L |

(continued)

| Component | CAS | Concentration | Units |
|---|---|---|---|
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2$ · $6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | | to determine the pH |

Perfusion fluid_09 was obtained according to the method for obtaining perfusion fluid_01, described in Example I.3.

The surfactants solution was prepared by dissolving 116.6000 g of Pluronic F68 and 17.1400 g of (1H, 1H, 2H, 2H-Perfluorooctyl)phosphocholine in ultrapure water.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2$ · $6H_2O$, 0.2192 g L-glutamine, 2.7660 g glutathione, 0.0757 g DL-β-hydroxybutyric acid sodium and dissolving the components in ultrapure water.

A total of 46.67 mL of the surfactants solution, 133.33 mL of the buffer and 20 mL of perfluorodecalin were combined to obtain 200 mL of the primary perfusion fluid, which was homogenized and microfluidized by running nine repetitions of pumping the total emulsion volume through the microfluidizer chamber. After the sterilizing filtration, perfusion fluid_09 was obtained.

**Example I.12 Obtaining perfusion fluid_10**

**Table 14.** Quantitative composition of perfusion fluid _10.

| Component | CAS | Concentration | Units |
|---|---|---|---|
| Perfluorotributylamine (PFTBA) | 311-89-7 | 188.40 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |
| Bovine albumin | 9048-46-8 90604-29-8 | 40.00 | g/L |

(continued)

| Component | CAS | Concentration | Units |
|---|---|---|---|
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 · 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_10 was obtained according to the method for obtaining perfusion fluid_01, described in Example I.3.

The surfactants solution was prepared by dissolving 116.6000 g of Pluronic F68 and 17.1400 g of (1H, 1H, 2H, 2H-Perfluorooctyl)phosphocholine in ultrapure water.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2 · 6H_2$ O, 0.2192 g L-glutamine, 2.7660 g glutathione, 0.0757 g DL-β-hydroxybutyric acid sodium and dissolving the components in ultrapure water.

A total of 46.67 mL of the surfactants solution, 133.33 mL of the buffer and 20 mL of perfluorotributylamine were combined to obtain 200 mL of the primary perfusion fluid, which was homogenized and microfluidized by conducting 9 repetitions of pumping the total volume of emulsion through the microfluidizer chamber. After the sterilizing filtration, perfusion fluid_10 was obtained.

**Example I.13 Obtaining perfusion fluid_11**

**Table 15.** Quantitative composition of perfusion fluid_11.

| Component | CAS | Concentration | Units |
|---|---|---|---|
| Perfluoropentane (DDFP) | 678-26-2 | 166.40 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |

(continued)

| Component | CAS | Concentration | Units |
|---|---|---|---|
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 · 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid _11 was obtained according to the method for obtaining perfusion fluid_01, described in Example I.3.

The surfactants solution was prepared by dissolving 116.6000 g of Pluronic F68 and 17.1400 g of (1H, 1H, 2H, 2H-Perfluorooctyl)phosphocholine in ultrapure water.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2 · 6H_2 O$, 0.2192 g L-glutamine, 2.7660 g glutathione, 0.0757 g DL-β-hydroxybutyric acid sodium and dissolving the components in ultrapure water.

A total of 46.67 mL of the surfactant solution, 133.33 mL of the buffer and 20 mL of perfluoropentane were combined to obtain 200 mL of the primary perfusion fluid, which was homogenized and microfluidized by running nine repetitions of pumping the total emulsion volume through the microfluidizer chamber. After the sterilizing filtration, perfusion fluid_11 was obtained.

**Example 1.14 Obtaining perfusion fluid_12**

Table 16. quantitative composition of perfusion fluid_12.

| Component | CAS | Concentration | Units |
|---|---|---|---|
| Perfluorohexane | 355-42-0 | 169.10 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |

(continued)

| Component | CAS | Concentration | Units |
|---|---|---|---|
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 \cdot 6H_2O$ | 7791-18-6 | 0.40 | mM |
| L-glutamine | 56-85-9 | 0.50 | mM |
| Glutathione | 70-18-8 | 3.00 | mM |
| DL-$\beta$-hydroxybutyric acid sodium salt | 150-83-4 | 0.20 | mM |
| NaOH | 1310-73-2 | to determine the pH | |

Perfusion fluid_12 was obtained according to the method for obtaining perfusion fluid_01, described in Example I.3.

The surfactants solution was prepared by dissolving 116.6000 g of Pluronic F68 and 17.1400 g of (1H, 1H, 2H, 2H-perfluorooctyl)phosphocholine in ultrapure water.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose $\cdot$ H$_2$O, 10.5192 g NaCl, 1,0288 g KCl, 3.4070 g Na$_2$HPO$_4$, 0.5399 g NaH$_2$PO$_4$, 0.1998 g CaCl$_2$, 0.2440 g MgCl$_2$ $\cdot$ 6H$_2$O, 0.2192 g L-glutamine, 2.7660 g glutathione, 0.0757 g DL-$\beta$-hydroxybutyric acid sodium and dissolving the components in ultrapure water.

A total of 46.67 mL of the surfactant solution, 133.33 mL of the buffer and 20 mL of perfluorohexane were combined to obtain 200 mL of the primary perfusion fluid, which was homogenized and microfluidized by conducting 9 repetitions of pumping the total volume of emulsion through the microfluidizer chamber. After the sterilizing filtration, perfusion fluid_12 was obtained.

## Example 1.15 Obtaining perfusion fluid_13

**Table 17.** Quantitative composition of perfusion fluid_13.

| Component | CAS | Concentration | Units |
|---|---|---|---|
| Perfluoroheptane | 335-57-9 | 174.50 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose $\cdot$ H$_2$O | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 \cdot 6H_2O$ | 7791-18-6 | 0.40 | mM |

(continued)

| Component | CAS | Concentration | Units |
|---|---|---|---|
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_13 was obtained according to the method for obtaining perfusion fluid_01, described in Example I.3.

The surfactants solution was prepared by dissolving 116.6000 g Pluronic F68 and 17.1400 g (1H, 1H, 2H, 2H-perfluorooctyl)phosphocholine in ultrapure water.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2$ · $6H_2O$, 0.2192 g L-glutamine, 2.7660 g glutathione, 0.0757 g DL-β-hydroxybutyric acid sodium and dissolving the components in ultrapure water.

A total of 46.67 mL of the surfactant solution, 133.33 mL of the buffer and 20 mL of perfluoroheptane were combined to obtain 200 mL of the primary perfusion fluid, which was homogenized and microfluidized by running nine repetitions of pumping the total emulsion volume through the microfluidizer chamber. After the sterilizing filtration, perfusion fluid_13 was obtained.

### Example 1.16 Obtaining perfusion fluid_14

Table 18. Quantitative composition of perfusion fluid_14.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |
| Hydroxyethylated starch 130/0.4 | 9005-27-0 | 60.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2$ · $6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |

(continued)

| Component | CAS | Concentration | Units |
|---|---|---|---|
| NaOH | 1310-73-2 | to determine the pH | |

Perfusion fluid_14 was obtained according to the method for obtaining perfusion fluid_01, described in Example I.3.

The surfactants solution was prepared by dissolving 116.6000 g Pluronic F68 and 17.1400 g (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 22.5000 g hydroxyethylated starch, 0.0065 g L-arginine, 0.0510 g allopurinol, 3.4157 g mannitol, 0.4695 g taurine, 0.1532 g L-tryptophan, 0.7431 g D-glucose · $H_2O$, 1.3149 g NaCl, 0,1286 g KCl, 0.4259 g $Na_2HPO_4$, 0.0675 g $NaH_2PO_4$, 0.0250 g $CaCl_2$, 0.0305 g $MgCl_2$ · $6H_2O$, 0.0274 g L-glutamine, 0.3457 g glutathione, 0.0095 g DL-β-hydroxybutyric acid sodium and dissolving the ingredients in ultrapure water.

A total of 46.67 mL of the surfactants solution, 133.33 mL of buffer and 20 mL of the perfluorocarbons mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_14 was obtained.

**Example I.17 Obtaining perfusion fluid_15**

Table 19. Quantitative composition of perfusion fluid_15.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |
| Dextran 70 kDa | 9004-54-0 | 50.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2$ · $6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_15 was obtained according to the method for obtaining perfusion fluid_01, described in Example 1.3.

The surfactants solution was prepared by dissolving 116.6000 g of Pluronic F68 and 17.1400 g of (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 18.7500 g Dextran 70 kDa, 0.0065 g L-arginine, 0.0510 g allopurinol, 3.4157 g mannitol, 0.4695 g taurine, 0.1532 g L-tryptophan, 0.7431 g D-glucose · $H_2O$, 1.3149 g NaCl, 0.1286 g KCl, 0.4259 g $Na_2HPO_4$, 0.0675 g $NaH_2PO_4$, 0.0250 g $CaCl_2$, 0.0305 g $MgCl_2$ · $6H_2O$, 0.0274 g L-glutamine, 0.3457 g glutathione, 0.0095 g DL-β-hydroxybutyric acid sodium and dissolving the ingredients in ultrapure water.

A total of 46.67 mL of the surfactants solution, 133.33 mL of the buffer and 20 mL of the perfluorocarbons mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_15 was obtained.

### Example 1.18 Obtaining perfusion fluid_16

Table 20. Quantitative composition of perfusion fluid_16.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |
| Dextran 40 kDa | 9004-54-0 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2$ · $6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_16 was obtained according to the method for obtaining perfusion fluid_01, described in Example I.3.

The surfactants solution was prepared by dissolving 116.6000 g of Pluronic F68 and 17.1400 g of (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 15.0000 g Dextran 40 kDa, 0.0065 g L-arginine, 0.0510 g allopurinol, 3.4157 g mannitol, 0.4695 g taurine, 0.1532 g L-tryptophan, 0.7431 g D-glucose · $H_2O$, 1.3149 g NaCl, 0.1286 g KCl, 0.4259 g $Na_2HPO_4$, 0.0675 g $NaH_2PO_4$, 0.0250 g $CaCl_2$, 0.0305 g $MgCl_2$ · $6H_2O$, 0.0274 g L-glutamine, 0.3457 g glutathione, 0.0095 g DL-β-hydroxybutyric acid sodium and dissolving the ingredients in ultrapure water.

A total of 46.67 mL of the surfactants solution, 133.33 mL of the buffer and 20 mL of the perfluorocarbon mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_16 was obtained.

**Example 1.19 Obtaining perfusion fluid_17**

Table 21. Quantitative composition of perfusion fluid_17.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |
| Poly(ethylene glycol) 35 kDa | 25322-68-3 | 20.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2$ · $6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_17 was obtained according to the method for obtaining perfusion fluid_01, described in Example I.3.

The surfactants solution was prepared by dissolving 116.6000 g of Pluronic F68 and 17.1400 g of (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 7.5000 g Poly(ethylene glycol) 35 kDa, 0.0065 g L-arginine, 0.0510 g allopurinol, 3.4157 g mannitol, 0.4695 g taurine, 0.1532 g L-tryptophan, 0.7431 g D-glucose · $H_2O$, 1.3149 g NaCl, 0,1286 g KCl, 0.4259 g $Na_2HPO_4$ , 0.0675 g $NaH_2PO_4$, 0.0250 g $CaCl_2$, 0.0305 g $MgCl_2$ · $6H_2O$, 0.0274 g L-glutamine, 0.3457 g glutathione, 0.0095 g DL-β-hydroxybutyric acid sodium and dissolving the ingredients in ultrapure water.

A total of 46.67 mL of the surfactants solution, 133.33 mL of the buffer and 20 mL of the perfluorocarbons mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_17 was obtained.

**Example 1.20 Obtaining perfusion fluid_18**

**Table 22.** Quantitative composition of perfusion fluid_18.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |
| Poly(ethylene glycol) 20 kDa | 25322-68-3 | 20.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2$ · $6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_18 was obtained according to the method for obtaining perfusion fluid_01, described in Example I.3.

The surfactants solution was prepared by dissolving 116.6000 g of Pluronic F68 and 17.1400 g of (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 7.5000 g Poly(ethylene glycol) 20 kDa, 0.0065 g L-arginine, 0.0510 g allopurinol, 3.4157 g mannitol, 0.4695 g taurine, 0.1532 g L-tryptophan, 0.7431 g D-glucose · $H_2O$, 1.3149 g NaCl, 0,1286 g KCl, 0.4259 g $Na_2HPO_4$, 0.0675 g $NaH_2PO_4$, 0.0250 g $CaCl_2$, 0.0305 g $MgCl_2$ · $6H_2O$, 0.0274 g L-glutamine, 0.3457 g glutathione, 0.0095 g DL-β-hydroxybutyric acid sodium and dissolving the ingredients in ultrapure water.

A total of 46.67 mL of the surfactants solution, 133.33 mL of the buffer and 20 mL of the perfluorocarbons mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_18 was obtained.

**Example 1.21 Obtaining perfusion fluid_19**

Table 23. Quantitative composition of perfusion fluid_19.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |
| Succinylated gelatine | 68915-24-2 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 \cdot 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_19 was obtained according to the method for obtaining perfusion fluid_01, described in Example I.3.

The surfactants solution was prepared by dissolving 116.6000 g of Pluronic F68 and 17.1400 g of (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 15.0000 g succinylated gelatin, 0.0065 g L-arginine, 0.0510 g allopurinol, 3.4157 g mannitol, 0.4695 g taurine, 0.1532 g L-tryptophan, 0.7431 g D-glucose · $H_2O$, 1.3149 g NaCl, 0,1286 gKCl, 0.4259 g $Na_2HPO_4$, 0.0675 g $NaH_2PO_4$, 0.0250 g $CaCl_2$, 0.0305 g $MgCl_2 \cdot 6H_2$ O, 0.0274 g L-glutamine, 0.3457 g glutathione, 0.0095 g DL-β-hydroxybutyric acid sodium and dissolving the ingredients in ultrapure water.

A total of 46.67 mL of the surfactants solution, 133.33 mL of the buffer and 20 mL of the perfluorocarbons mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_19 was obtained.

**Example 1.22 Obtaining perfusion fluid_20**

Table 24. quantitative composition of perfusion fluid_20.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F68 (Kolliphor P188) | 9003-11-6 | 27.20 | g/L |
| (1H, 1H, 2H, 2H-Perfluorooctyl)-β-D-Maltopyranoside | 118680-70-9 | 4.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 \cdot 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_20 was obtained according to the method for obtaining perfusion fluid_01, described in Example I.3.

The surfactants solution was prepared by dissolving 11.6600 g of Pluronic F68 and 1.7140 g of (1H, 1H, 2H, 2H-Perfluorooctyl)-β-D-maltopyranoside in ultrapure water.

The perfluorinated phase was prepared by dissolving 30.9278 g of perfluorodecyl bromide in 1000 g of perfluorooctyl bromide.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2 \cdot 6H_2$ O, 0.2192 g L-glutamine, 2.7660 g glutathione, 0.0757 g DL-β-hydroxybutyric acid sodium and dissolving the components in ultrapure water.

A total of 46.67 mL of the surfactants solution, 133.33 mL of the buffer and 20 mL of the perfluorocarbons mixture were combined to obtain 200 mL of the primary perfusion fluid, which was homogenized and microfluidized, conducting 9 repetitions of pumping the total emulsion volume through the microfluidizer chamber. After the sterilizing filtration, perfusion fluid_20 was obtained.

## Example 1.23 Obtaining perfusion fluid_21

Table 25. Quantitative composition of perfusion fluid_21.

| Component | CAS | Concentration | Units |
|---|---|---|---|
| Perfluorooctyl bromide (PFOB) | 423-55-2 | 187.21 | g/L |

(continued)

| Component | CAS | Concentration | Units |
|---|---|---|---|
| Perfluorodecyl bromide (PFDB) | 307-43-7 | 5.79 | g/L |
| Pluronic F68 (Kolliphor P188) | 9003-11-6 | 27.20 | g/L |
| 1,2-Dioleoyl-sn-glycero-3-phospho-L-serine sodium salt (PS 18:1/18:1 DOPS-Na) | 70614-14-1 | 4.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 \cdot 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_21 was obtained according to the method for obtaining perfusion fluid_01, described in Example I.3.

The surfactants solution was prepared by dissolving 11.6600 g of Pluronic F68 and 1.7140 g of 1,2-Dioleoyl-sn-glycero-3-phospho-L-serine sodium salt (PS 18:1/18:1 DOPS-Na) in ultrapure water.

The perfluorinated phase was prepared by dissolving 30.9278 g of perfluorodecyl bromide in 1000 g of perfluorooctyl bromide.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2 \cdot 6H_2O$, 0.2192 g L-glutamine, 2.7660 g glutathione, 0.0757 g DL-β-hydroxybutyric acid sodium and dissolving the components in ultrapure water.

A total of 46.67 mL of the surfactants solution, 133.33 mL of the buffer and 20 mL of the perfluorocarbons mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_21 was obtained.

**Example 1.24 Obtaining perfusion fluid_22**

**Table 26.** Quantitative composition of perfusion fluid_22.

| Component | CAS | Concentration | Units 1 |
|---|---|---|---|
| Perfluorooctyl bromide (PFOB) | 423-55-2 | 187.21 | g/L |

(continued)

| Component | CAS | Concentration | Units 1 |
|---|---|---|---|
| Perfluorodecyl bromide (PFDB) | 307-43-7 | 5.79 | g/L |
| Pluronic F68 (Kolliphor P188) | 9003-11-6 | 27.20 | g/L |
| sodium salt of 1,2-dipalmitoyl-sn-glycero-3-phosphate (PA 16:0/16:0 DPPA-Na) | 169051-60-9 | 4.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 · 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_22 was obtained according to the method for obtaining perfusion fluid_01, described in Example I.3.

The surfactants solution was prepared by dissolving 11.6600 g of Pluronik F68 and 1.7140 g of 1,2-dipalmitoyl-sn-glycero-3-phosphate sodium (PA 16:0/16:0 DPPA-Na) in ultrapure water.

The perfluorinated phase was prepared by dissolving 30.9278 g of perfluorodecyl bromide in 1000 g of perfluorooctyl bromide.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2 · 6H_2O$, 0.2192 g L-glutamine, 2.7660 g glutathione, 0.0757 g DL-β-hydroxybutyric acid sodium and dissolving the components in ultrapure water.

A total of 46.67 mL of the surfactants solution, 133.33 mL of the buffer and 20 mL of the perfluorocarbons mixture were combined to obtain 200 mL of the primary perfusion fluid, which was homogenized and microfluidized by conducting 13 repetitions of pumping the total emulsion volume through the microfluidizer chamber. After the sterilizing filtration, perfusion fluid_22 was obtained.

**Example 1.25 Obtaining perfusion fluid_23**

**Table 27.** Quantitative composition of perfusion fluid_23.

| Component | CAS | Concentration | Units |
|---|---|---|---|
| Perfluorooctyl bromide (PFOB) | 423-55-2 | 187.21 | g/L |

(continued)

| Component | CAS | Concentration | Units |
|---|---|---|---|
| Perfluorodecyl bromide (PFDB) | 307-43-7 | 5.79 | g/L |
| Pluronic F68 (Kolliphor P188) | 9003-11-6 | 27.20 | g/L |
| 1,2-dimyristoyl-sn-glycero-3-phospho-rac-glycerol sodium salt (PG 14:0/14:0 DMPG-Na) | 67232-80-8 | 4.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10,00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 · 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_23 was obtained according to the method for obtaining perfusion fluid_01, described in Example I.3.

The surfactants solution was prepared by dissolving 11.6600 g of Pluronic F68 and 1.7140 g of 1,2-dimyristoyl-sn-glycero-3-phospho-rac-glycerol sodium salt (PG 14:0/14:0 DMPG-Na) in ultrapure water.

The perfluorinated phase was prepared by dissolving 30.9278 g of perfluorodecyl bromide in 1000 g of perfluorooctyl bromide.

The Buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2 · 6H_2O$, 0.2192 g L-glutamine, 2.7660 g glutathione, 0.0757 g DL-β-hydroxybutyric acid sodium and dissolving the components in ultrapure water.

A total of 46.67 mL of the surfactants solution, 133.33 mL of the buffer and 20 mL of the perfluorocarbons mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_23 was obtained.

**Example 1.26 Obtaining perfusion fluid_24**

**Table 28.** quantitative composition of perfusion fluid_24.

| Component | CAS | Concentration | Units |
|---|---|---|---|
| Perfluorooctyl bromide (PFOB) | 423-55-2 | 187.21 | g/L |

(continued)

| Component | CAS | Concentration | Units |
|-----------|-----|---------------|-------|
| Perfluorodecyl bromide (PFDB) | 307-43-7 | 5.79 | g/L |
| Pluronic F68 (Kolliphor P188) | 9003-11-6 | 27.20 | g/L |
| Sodium salt of N-(carbonyl-methoxypolyethyleneglycol-2000)-1,2-distearoyl-sn-glycero-3-phosphoethanolamine (PE 18:0/18:0-PEG 2000) | 147867-65-0 | 4.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 \cdot 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_24 was obtained according to the method for obtaining perfusion fluid_01, described in Example I.3.

The surfactants solution was prepared by dissolving 11.6600 g of Pluronic F68 and 1.7140 g of N-(carbonyl-methoxypolyethyleneglycol-2000)-1,2-distearoyl-sn-glycero-3-phospho-ethanolamine sodium salt (PE 18:0/18:0-PEG 2000) in ultrapure water.

The perfluorinated phase was prepared by dissolving 30.9278 g of perfluorodecyl bromide in 1000 g of perfluorooctyl bromide.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2 HPO_4$, 0.5399 g $NaH_2 PO_4$, 0.1998 g $CaCl_2$ - $H_2 O$, 0.2440 g $MgCl_2$ - $6H_2 O$, 0.2192 g L-glutamine, 2.7660 g glutathione, 0.0757 g DL-β-hydroxybutyric acid sodium and dissolving the components in ultrapure water.

A total of 46.67 mL of the surfactanst solution, 133.33 mL of the buffer and 20 mL of the perfluorocarbons mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_24 was obtained.

**Example 1.27 Obtaining perfusion fluid_25**

Table 29. quantitative composition of perfusion fluid_25.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| (1H, 1H, 2H, 2H-Perfluorooctyl)phosphocholine | 313997-22-7 | 4.00 | g/L |
| Bovine albumin | 9048-46-8 90604-29-8 | 67.20 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2$ · $6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_25 was obtained according to the method for obtaining perfusion fluid_01, described in Example I.3.

The surfactants solution was prepared by dissolving 11.6600 g of bovine serum albumin and 1.7140 g of (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2$ · $6H_2O$, 0.2192 g L-glutamine, 2.7660 g glutathione, 0.0757 g DL-β-hydroxybutyric acid sodium and dissolving the components in ultrapure water.

A total of 46.67 mL of the surfactants solution, 133.33 mL of the buffer and 20 mL of the perfluorocarbons mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_25 was obtained.

**Example 1.28 Obtaining perfusion fluid_26**

Table 30. Quantitative composition of perfusion fluid_26.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |

(continued)

| Component | CAS | Concentration | Units |
|---|---|---|---|
| (1H, 1H, 2H, 2H-Perfluorooctyl)phosphocholine | 313997-22-7 | 4.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2$ · $6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_26 was obtained according to the method for obtaining perfusion fluid_01, described in Example I.3.

A surfactant solution was prepared by dissolving 1.7140 g of (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2$ · $6H_2O$, 0.2192 g L-glutamine, 2.7660 g glutathione, 0.0757 g DL-β-hydroxybutyric acid sodium and dissolving the components in ultrapure water.

A total of 46.67 mL of the surfactant solution, 133.33 mL of the buffer and 20 mL of the perfluorocarbons mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_26 was obtained.

**Example 1.29 Obtaining perfusion fluid_27**

**Table 31.** Quantitative composition of perfusion fluid_27.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F-108 | 9003-11-6 | 27.20 | g/L |
| (1H, 1H, 2H, 2H-Perfluorooctyl)phosphocholine | 313997-22-7 | 4.00 | g/L |

(continued)

| Component | CAS | Concentration | Units |
|---|---|---|---|
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 · 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_27 was obtained according to the method for obtaining perfusion fluid_01, described in Example I.3.

The surfactants solution was prepared by dissolving 11.6600 g Pluronic F-108 and 17.1400 g (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2 · 6H_2O$, 0.2192 g L-glutamine, 2.7660 g glutathione, 0.0757 g DL-β-hydroxybutyric acid sodium and dissolving the components in ultrapure water.

A total of 46.67 mL of the surfactants solution, 133.33 mL of the buffer and 20 mL of the perfluorocarbon mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_27 was obtained.

**Example 1.30 Obtaining perfusion fluid_28**

**Table 32.** quantitative composition of perfusion fluid_28.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F-127 | 9003-11-6 | 27.20 | g/L |
| (1H, 1H, 2H, 2H-Perfluorooctyl)phosphocholine | 313997-22-7 | 4.00 | g/L |

(continued)

| Component | CAS | Concentration | Units |
|---|---|---|---|
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 · 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_28 was obtained according to the method for obtaining perfusion fluid_01, described in Example I.3.

The surfactants solution was prepared by dissolving 11.6600 g of Pluronic F68 and 17.1400 g of (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 120.0000 g bovine serum albumin, 0.0523 g L-arginine, 0.4083 g allopurinol, 27.3255 g mannitol, 3.7560 g taurine, 1.2254 g L-tryptophan, 5.9451 g D-glucose · $H_2O$, 10.5192 g NaCl, 1,0288 g KCl, 3.4070 g $Na_2HPO_4$, 0.5399 g $NaH_2PO_4$, 0.1998 g $CaCl_2$, 0.2440 g $MgCl_2 · 6H_2O$, 0.2192 g L-glutamine, 2.7660 g glutathione, 0.0757 g DL-β-hydroxybutyric acid sodium and dissolving the components in ultrapure water.

A total of 46.67 mL of the surfactants solution, 133.33 mL of the buffer and 20 mL of the perfluorocarbons mixture were combined to obtain 200 mL of the primary perfusion fluid, which was homogenized and microfluidized by conducting 14 repetitions of pumping the total emulsion volume through the microfluidizer chamber. After the sterilizing filtration, perfusion fluid_28 was obtained.

## Example 1.31 Obtaining perfusion fluid_29

**Table 33.** Quantitative composition of perfusion fluid_29.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |

(continued)

| Component | CAS | Concentration | Units |
|---|---|---|---|
| Bovine albumin | 9048-46-8<br>90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| L-isoleucine | 73-32-5 | 1.52 | mM |
| L-leucine | 61-90-5 | 3.05 | mM |
| L-lysine | 56-87-1 | 2.05 | mM |
| L-methionine | 63-68-3 | 1.34 | mM |
| L-threonine | 72-19-5 | 1.68 | mM |
| L-valine | 72-18-4 | 2.56 | mM |
| L-histidine | 71-00-1 | 0.64 | mM |
| L-alanine | 56-41-7 | 5.61 | mM |
| L-glycine | 56-40-6 | 6.66 | mM |
| L-aspartic acid | 56-84-8 | 1.88 | mM |
| L-proline | 147-85-3 | 2.17 | mM |
| L-serine | 56-45-1 | 0.95 | mM |
| L-tyrosine | 60-18-4 | 0.06 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 · 6H_2O$ | 7791-18-6 | 0.40 | mM |
| L-glutamine | 56-85-9 | 0.50 | mM |
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_29 was obtained according to the method for obtaining perfusion fluid_01, described in Example I.3.

The surfactants solution was prepared by dissolving 116.6000 g Pluronic F68 and 17.1400 g (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 15.0000 g bovine serum albumin, 0.0065 g L-arginine, 0.0510 g allopurinol, 3.4157 g mannitol, 0.4695 g taurine, 0.1532 g L-tryptophan, 0,0500 g L-isoleucine, 0.1000 g L-leucine, 0.0750 g L-lysine, 0.0500 g L-methionine, 0.0500 g L-threonine, 0.0750 g L-valine, 0.0250 g L-histidine, 0.1250 g L-alanine, 0.1250 g L-glycine, 0.0625 g L-aspartic acid, 0.0625 g L-proline, 0.0250 g L-serine, 0.0025 g L-tyrosine, 0.7431 g D-glucose · $H_2O$, 1.3149 g NaCl, 0.1286 g KCl, 0.4259 g $Na_2HPO_4$, 0.0675 g $NaH_2PO_4$, 0,0250 g $CaCl_2$, 0.0305 g $MgCl_2 · 6H_2O$, 0.0274 g L-glutamine, 0.3457 g glutathione, 0.0095 g DL-β-hydroxybutyric acid sodium salt and dissolving the components in ultrapure water.

A total of 46.67 mL of the surfactants solution, 133.33 mL of the buffer and 20 mL of the perfluorocarbons mixture were combined to obtain 200 mL of the primary perfusion fluid, which was homogenized and microfluidized by running nine repetitions of pumping the total emulsion volume through the microfluidizer chamber. After the sterilizing filtration, perfusion fluid_29 was obtained.

**Example 1.32 Obtaining perfusion fluid_30**

Table 34. quantitative composition of perfusion fluid_30.

| Component | CAS | Concentration | Units |
|---|---|---|---|
| Perfluorooctyl bromide (PFOB) | 423-55-2 | 187.21 | g/L |
| Perfluorodecyl bromide (PFDB) | 307-43-7 | 5.79 | g/L |
| Pluronic F68 (Kolliphor P188) | 9003-11-6 | 27.20 | g/L |
| (1H, 1H, 2H, 2H-Perfluorooctyl)phosphocholine | 313997-22-7 | 4.00 | g/L |
| Bovine albumin | 9048-46-8 90604-29-8 | 40.00 | g/L |
| L-arginine | 74-79-3 | 0.10 | mM |
| Allopurinol | 315-30-0 | 1.00 | mM |
| Mannitol | 69-65-8 | 50.00 | mM |
| Taurine | 107-35-7 | 10.00 | mM |
| L-tryptophan | 73-22-3 | 2.00 | mM |
| L-isoleucine | 73-32-5 | 4.19 | mM |
| L-leucine | 61-90-5 | 6.86 | mM |
| L-lysine | 56-87-1 | 4.79 | mM |
| L-methionine | 63-68-3 | 3.35 | mM |
| L-threonine | 72-19-5 | 3.78 | mM |
| L-valine | 72-18-4 | 5.55 | mM |
| L-histidine | 71-00-1 | 2.26 | mM |
| L-alanine | 56-41-7 | 12.35 | mM |
| L-glycine | 56-40-6 | 17.32 | mM |
| L-aspartic acid | 56-84-8 | 4.51 | mM |
| L-proline | 147-85-3 | 5.21 | mM |
| L-serine | 56-45-1 | 2.38 | mM |
| L-tyrosine | 60-18-4 | 0.28 | mM |
| D-glucose · $H_2O$ | 14431-43-7 | 10.00 | mM |
| NaCl | 7647-14-5 | 60.00 | mM |
| KCl | 7447-40-7 | 4.60 | mM |
| $Na_2HPO_4$ | 7558-79-4 | 8.00 | mM |
| $NaH_2PO_4$ | 7558-80-7 | 1.50 | mM |
| $CaCl_2$ | 10043-52-4 | 0.60 | mM |
| $MgCl_2 \cdot 6H_2O$ | 7791-18-6 | 0.40 | mM |
| L-glutamine | 56-85-9 | 0.50 | mM |

(continued)

| Component | CAS | Concentration | Units |
|---|---|---|---|
| Glutathione | 70-18-8 | 3.00 | mM |
| DL-β-hydroxybutyric acid sodium salt | 150-83-4 | 0.20 | mM |
| NaOH | 1310-73-2 | to determine the pH | |

Perfusion fluid_30 was obtained according to the method for obtaining perfusion fluid_01, described in Example I.3.

The surfactants solution was prepared by dissolving 116.6000 g Pluronic F68 and 17.1400 g (1H, 1H, 2H, 2H-Perfluorooctyl)phosphocholine in ultrapure water.

The perfluorinated phase was prepared by dissolving 30.9278 g of perfluorodecyl bromide in 1000 g of perfluorooctyl bromide.

The buffer was prepared by weighing 15.0000 g bovine serum albumin, 0.0065 g L-arginine, 0.0510 g allopurinol, 3.4157 g mannitol, 0.4695 g taurine, 0.1532 g L-tryptophan, 0,1375 g L-isoleucine, 0.2250 g L-leucine, 0.1750 g L-lysine, 0.1250 g L-methionine, 0.1125 g L-threonine, 0.1625 g L-valine, 0.0875 g L-histidine, 0.2750 g L-alanine, 0.3250 g L-glycine, 0,1500 g L-aspartic acid, 0.1500 g L-proline, 0.0625 g L-serine, 0.0125 g L-tyrosine, 0.7431 g D-glucose · $H_2O$, 1.3149 g NaCl, 0.1286 gKCl, 0.4259 g $Na_2HPO_4$, 0.0675 g $NaH_2PO_4$, 0,0250 g $CaCl_2$, 0.0305 g $MgCl_2$ · $6H_2O$, 0.0274 g L-glutamine, 0.3457 g glutathione, 0.0095 g DL-β-hydroxybutyric acid sodium salt and dissolving the components in ultrapure water.

A total of 46.67 mL of the surfactants solution, 133.33 mL of the buffer and 20 mL of the perfluorocarbon mixture were combined to obtain 200 mL of the primary perfusion fluid, which was homogenized and microfluidized by running 9 repetitions of pumping the total emulsion volume through the microfluidizer chamber. After the sterilizing filtration, perfusion fluid_30 was obtained.

**Example II:**

**Methods for quality control of perfusion fluid**

**Example II.1. Determination of particle size distribution of perfusion fluid**

Emulsion particle size distributions were measured by dynamic light scattering (DLS) using a Malvern Zetasizer Nano ZS (Malvern Instruments. Ltd . Worcestershire. UK). A series of dilutions were performed for each sample: 5-fold and 50-fold with ultrapure water (MilliQ). Measurements were made at 25°C and at a scattering angle of 173°. Results were presented by two parameters: mean particle size and polydispersity index (PdI). The mean particle size (reported as mean diameter in nanometres) is a value calculated by the instrument from the particle intensity signal, according to the ISO standard provided by the manufacturer (ISO 13321:1996E and 22412). The polydispersity index is a dimensionless value expressing the width of the particle size distribution of the emulsion, i.e. the homogeneity of the analysed sample, calculated according to ISO 13321:1996E and 22412. All measurements were carried out in triplicate. Values were given as the mean value of the three measurements and the standard deviation was calculated.

**Example II.2. Determination of the zeta potential of particles in a perfusion fluid**

Zeta potential measurements were performed using Malvern's Zetasizer Nano ZS and DTS1070 zeta potential cuvettes. The resulting o/w emulsion was diluted 10-fold, with MilliQ ultrapure water. A minimum of 3 measurements were made, taking approximately 2 ml of the diluted emulsion each time. The result was given by averaging all the zeta potential values obtained, which were averaged, and the standard deviation was calculated, in units: mV

**Example II.3. Determination of osmotic pressure of perfusion fluid**

Osmolality measurements were performed using Osmometer 800CLG, Trident Med and OSMO-KRIO measuring vessels with crystallisation germs. Measurements were performed in a minimum of 3 replicates, taking 100 µl of the obtained emulsion each time. The result was given by averaging all the osmolality values obtained and calculating the standard deviation, in units: mOsm/kg$H_2O$.

**Example II.4. Determination of oncotic pressure of perfusion fluid**

Oncotic pressure measurements were performed using a BMT 923 Oncometer with a cellulose triacetate membrane with a cut-off mass of 20,000 daltons, BMT MESSTECHNIK GMBH, Germany. Measurements were performed in a minimum of 3 replicates, taking 100 $\mu$l of the fluid obtained each time. The result was given by averaging all osmolality values obtained and calculating the standard deviation, in units: mmHg.

**Example II.5. Determination of the concentration of selected ions in perfusion fluid**

Concentrations of sodium, potassium, calcium and chlorine ions were measured using an EC90 ion analyser with interchangeable iCa measuring cartridges[2+], Erba Polska Sp. z o.o.. A one- and two-point calibration of the instrument was performed prior to the measurements. Measurements were performed in a minimum of 3 repetitions, with the device taking 35 $\mu$l of the fluid obtained each time. The result was given by averaging all the ion concentration values obtained and calculating the standard deviation, in units: mmol/L.

**Example II.6. Determination of pH of perfusion fluid**

pH measurements were made using a pHmeter, Mettler Toledo, Italy, and calibration solutions of pH: 2.00, 4.00, 7.00, 9.21, 11.00. The instrument was calibrated before measurement. Measurements were performed in a minimum of 3 repetitions, taking 3 mL of the fluid obtained each time. The result was given by averaging all the pH values obtained and calculating the standard deviation, in dimensionless units.

**Example II.7. Assessment of cytotoxicity of perfusion fluid**

Tests on extracts from the obtained perfusion fluid, were performed *in vitro* on mouse fibroblast cell cultures (L929), using the Cell Proliferation Kit II (XTT) cytotoxicity assay. The test was performed according to ISO 10993-5 (standard describing test methods for the cytotoxicity evaluation of medical devices).
The first step of the work was to prepare mouse fibroblast cultures. In a 96-well plate, $10^4$ cells per well were seeded and 100 $\mu$L of culture medium (DMEM with 10 % (v/v) admixture of fetal bovine serum (FBS) and 1 % (v/v) addition of a mixture of penicillin and streptomycin) was added. The cells prepared in this way were kept in an incubator for 24 h. After 24 h under sterile conditions, 50 % (v/v) solutions of the resulting fluids in culture medium were prepared and then transferred to the cell plates, previously removing the old culture medium from the wells. In addition to the test materials, a negative control for cytotoxicity (NC) was prepared in the form of culture medium incubated under the same conditions as the perfusion fluid samples tested, and a positive control for cytotoxicity (PC) was prepared in the form of culture medium with 0.2 % (v/v) Triton X-100. After 24 h incubation with the cells, the solutions were removed from the cells and each well was washed three times with phosphate-buffered saline (PBS). The XTT assay was then performed according to the manufacturer's protocol. The results obtained were expressed as a percentage relative to the value of the corresponding negative control.

**Example II.8. Determination of viscosity of perfusion fluid**

Viscosity measurements were performed using a Brookfield DV-III Ultra rheometer with a CPE-40 conical spindle (shear rate range: 0-1500 1/s) and equipped with a thermostated sheath, BROOKFIELD ENGINEERING LABORATORIES, INC, USA. Measurements were performed in a minimum of 3 replicates, taking 500 $\mu$l of the fluid obtained each time. Samples were thermostated at 20-23 °C. The result was given by averaging all the viscosity values obtained and calculating the standard deviation, in units of: mPa · s

**Example II.9. Assessment of stability/sustainability of perfusion fluid**

Fluid stability/persistence was assessed by measuring emulsion particle size distribution by dynamic light scattering (DLS) using a Malvern Zetasizer Nano ZS (Malvern Instruments. Ltd.. Worcestershire. UK), according to Example 1. Fluid samples were stored under reduced temperature conditions of 4-6° C.

**Table 35.** Quality control results of organ perfusion fluids, particle size distribution, zeta potential, viscosity and pH.

| Name of the perfusion fluid | Average diameter [nm] | Pdl [-] | Average diameter after 7 days [nm] | Pdl after 7 days [-] | Zeta potential [mV] | Viscosity [mPa-s] | pH[-] |
|---|---|---|---|---|---|---|---|
| Perfusion fluid_01 | 105.31 ± 1.31 | 0.202 ± 0.005 | 178.70 ± 1.61 | 0.083 ± 0.016 | -2.57 ± 0.86 | 1.50-2.00 | 7.24 ± 0.01 |
| Perfusion fluid_02 | 121.29 ± 1.53 | 0.121 ± 0.008 | 200.60 ± 1.69 | 0.049 ± 0.009 | -1.96 ± 0.43 | 2.88 ± 0.04 | 7.23 ± 0.00 |
| Perfusion fluid_03 | 116.73 ± 1.42 | 0.111 ± 0.013 | 210.28 ± 2.54 | 0.027 ± 0.014 | -2.33 ± 0.86 | 3.50-4.00 | 7.26 ± 0.01 |
| Perfusion fluid_04 | 137.42 ± 1.39 | 0.069 ± 0.016 | 218.74 ± 2.05 | 0.038 ± 0.019 | -2.08 ± 0.34 | 5.33 ± 0.05 | 7.26 ± 0.00 |
| Perfusion fluid_05 | 124.79 ± 1.57 | 0.078 ± 0.017 | 215.94 ± 2.47 | 0.042 ± 0.015 | -3.28 ± 0.34 | 8.47 ± 0.04 | 7.24 ± 0.01 |
| Perfusion fluid_06 | 116.16 ± 1.12 | 0.062 ± 0.011 | 214.28 ±2.15 | 0.037 ± 0.019 | -2.67 ± 0.45 | 19.00 ±0.00 | 7.24 ± 0.01 |
| Perfusion fluid_07 | 135.77 ± 0.96 | 0.030 ± 0.008 | 220.16 ± 2.03 | 0.042 ± 0.019 | -1.70 ± 0.43 | 19.00-30.00 | 7.27 ± 0.00 |
| Perfusion fluid_08 | 131.71 ± 1.67 | 0.032 ± 0.011 | 222.36 ± 2.59 | 0.032 ± 0.016 | -2.70 ±0.51 | 30.00-40.00 | 7.29 ± 0.01 |
| Perfusion fluid_09 | 136.39 ± 1.13 | 0.166 ± 0.014 | 239.14 ± 3.53 | 0.081 ± 0.017 | 3.03 ± 0.91 | 2.95 ± 0.01 | 7.24 ± 0.02 |
| Perfusion fluid_10 | 95.72 ±0.71 | 0.199 ± 0.009 | 96.23 ± 1.33 | 0.197 ± 0.004 | 3.69 ± 0.39 | 2.85 ± 0.00 | 7.25 ± 0.00 |
| Perfusion fluid_11 | 388.14 ±7.08 | 0.138 ± 0.020 | 526.54 ± 12.73 | 0.081 ± 0.028 | -4.31 ± 1.36 | 2.70-3.00 | 7.27 ± 0.01 |
| Perfusion fluid_12 | 261.42 ±6.69 | 0.053 ± 0.039 | 457.32 ± 11.50 | 0.117 ± 0.032 | -2.06 ± 0.27 | 2.70-3.00 | 7.20 ± 0.01 |
| Perfusion fluid_13 | 184.79 ± 1.50 | 0.035 ± 0.020 | 277.92 ± 4.03 | 0.073 ± 0.035 | -2.31 ± 0.75 | 2.70-3.00 | 7.22 ± 0.01 |
| Perfusion fluid_14 | 94.84 ± 1.01 | 0.175 ± 0.010 | 219.87 ± 3.33 | 0.062 ± 0.017 | -1.65 ± 0.23 | 12.45 ± 1.39 | 7.20 ± 0.00 |
| Perfusion fluid_15 | 112.29 ± 1.02 | 0.098 ± 0.009 | 216.37 ± 2.82 | 0.032 ± 0.019 | -2.76 ± 0.14 | 11.33 ± 0.54 | 7.29 ± 0.01 |

(continued)

| Name of the perfusion fluid | Average diameter [nm] | PdI [-] | Average diameter after 7 days [nm] | PdI after 7 days [-] | Zeta potential [mV] | Viscosity [mPa-s] | pH[-] |
|---|---|---|---|---|---|---|---|
| Perfusion fluid_16 | 124.50 ± 1.03 | 0.066 ± 0.011 | 220.90 ± 1.76 | 0.064 ± 0.020 | -2.31 ± 0.83 | 7.85 ± 0.29 | 7.21 ± 0.01 |
| Perfusion fluid_17 | 114.52 ± 1.08 | 0.035 ± 0.009 | 222.17 ± 4.22 | 0.019 ± 0.012 | -1.64 ± 0.16 | 13.57 ± 0.21 | 7.31 ± 0.00 |
| Perfusion fluid_18 | 109.62 ± 1.10 | 0.050 ± 0.016 | 223.41 ± 2.63 | 0.033 ± 0.021 | -2.33 ± 0.70 | 8.34 ± 0.08 | 7.31 ± 0.00 |
| Perfusion fluid_19 | 122.63 ± 1.72 | 0.066 ± 0.013 | 215.71 ± 2.64 | 0.034 ± 0.021 | -1.66 ± 0.39 | 5.98 ± 0.32 | 6.08 ± 0.01 |
| Perfusion fluid_20 | 116.39 ± 1.02 | 0.139 ± 0.011 | 204.04 ± 2.18 | 0.055 ± 0.015 | -1.55 ± 0.57 | 2.70-3.00 | 7.20 ± 0.01 |
| Perfusion fluid_21 | 109.16 ± 0.82 | 0.189 ± 0.010 | 333.98 ± 5.63 | 0.164 ± 0.016 | -22.41 ± 1.53 | 2.70-3.00 | 7.23 ± 0.01 |
| Perfusion fluid_22 | 262.02 ± 3.62 | 0.391 ± 0.025 | 348.86 ± 3.39 | 0.232 ± 0.019 | -16.62 ± 1.08 | 2.70-3.00 | 7.16 ± 0.00 |
| Perfusion fluid_23 | 126.54 ± 1.29 | 0.117 ± 0.012 | 301.59 ± 4.93 | 0.144 ± 0.020 | -23.03 ± 1.07 | 2.70-3.00 | 7.22 ± 0.00 |
| Perfusion fluid_24 | 110.82 ± 0.96 | 0.165 ± 0.011 | 242.79 ± 2.48 | 0.061 ± 0.023 | -14.78 ± 1.10 | 2.70-3.00 | 7.24 ± 0.00 |
| Perfusion fluid_25 | 74.27 ± 1.89 | 0.279 ± 0.006 | 227.77 ± 2.18 | 0.242 ± 0.014 | -22.70 ± 1.99 | 2.00-3.00 | 7.15 ± 0.00 |
| Perfusion fluid_26 | 95.05 ± 0.690 | 0.203 ± 0.011 | 217.26 ± 1.80 | 0.172 ± 0.013 | -25.46 ± 1.48 | 1.50-2.00 | 7.21 ± 0.00 |
| Perfusion fluid_27 | 126.50 ± 1.33 | 0.172 ± 0.009 | 202.72 ± 2.31 | 0.043 ± 0.019 | -1.38 ± 0.34 | 2.70-3.00 | 7.21 ± 0.00 |
| Perfusion fluid_28 | 128.87 ± 0.75 | 0.183 ± 0.007 | 212.90 ± 2.26 | 0.047 ± 0.016 | -1.46 ± 0.48 | 2.70-3.00 | 7.22 ± 0.00 |
| Perfusion fluid_29 | 121.42 ± 1.33 | 0.118 ± 0.011 | 206.84 ± 1.94 | 0.049 ± 0.011 | -2.47 ± 0.87 | 2.79 ± 0.05 | 7.26 ± 0.01 |
| Perfusion fluid_30 | 119.06 ± 0.96 | 0.118 ± 0.007 | 206.78 ±1.59 | 0.046 ± 0.013 | -1.49 ± 0.44 | 3.01 ± 0.04 | 7.24 ± 0.01 |

(continued)

| Name of the perfusion fluid | Average diameter [nm] | PdI [-] | Average diameter after 7 days [nm] | PdI after 7 days [-] | Zeta potential [mV] | Viscosity [mPa-s] | pH[-] |
|---|---|---|---|---|---|---|---|
| Perfusion fluid_02_II production method | 163.81 ± 1.43 | 0.054 ± 0.012 | 220.52 ± 3.75 | 0.056 ± 0.019 | -2.61 ± 0.52 | 2.88 ± 0.04 | 7.43 ± 0.01 |
| Perfusion fluid_04_II production method | 190.70 ± 1.50 | 0.068 ± 0.020 | 213.46 ± 2.43 | 0.067 ± 0.019 | -4.86 ± 0.63 | 5.33 ± 0.05 | 7.32 ± 0.00 |

**Table 36.** Results of quality control of organ perfusion fluids, osmotic pressure, oncotic pressure, sodium, potassium, calcium and chlorine ion concentrations and cytotoxicity.

| Name of the perfusion fluid | Osmotic pressure [mOsm/kgH$_2$O]. | Oncotic pressure [mmHg]. | Na$^+$ [mM] | K$^+$ [mM] | Ca$^{2+}$ [mM] | Cl$^-$ [mM] | Cell viability [%] |
|---|---|---|---|---|---|---|---|
| Perfusion fluid_01 | 281 ± 1 | 34.9 ± 3.8 | 94.62 ± 0.78 | 5.16 ± 0.01 | 0.19 ± 0.00 | 67.21 ± 0.59 | 83 ± 4 |
| Perfusion fluid_02 | 317 ± 4 | 28.8 ± 2.1 | 101.08 ± 0.67 | 5.77 ± 0.04 | 0.22 ± 0.01 | 72.53 ± 0.42 | 89 ± 12 |
| Perfusion fluid_03 | 300 ± 4 | 26.9 ± 1.8 | 107.97 ± 0.85 | 6.17 ± 0.04 | 0.42 ± 0.01 | 81.31 ± 0.66 | 83 ± 20 |
| Perfusion fluid_04 | 328 ± 2 | 26.0 ± 1.0 | 114.34 ± 0.30 | 6.56 ± 0.04 | 0.44 ± 0.00 | 86.89 ± 0.44 | 83 ± 15 |
| Perfusion fluid_05 | 355 ± 2 | 18.3 ± 0.6 | 123.47 ± 0.32 | 6.97 ± 0.04 | 0.46 ± 0.00 | 96.16 ± 0.11 | 83 ± 2 |
| Perfusion fluid_06 | 395 ± 13 | 33.7 ± 5.3 | 136.32 ± 0.50 | 7.57 ± 0.16 | 0.50 ± 0.00 | 106.72 ± 0.52 | 90 ± 3 |
| Perfusion fluid_07 | 417 ± 10 | 35.4 ± 1.9 | 106. 85 ± 1.70 | 5.86 ± 0.11 | 0.37 ± 0.01 | 81.97 ± 1.02 | 88 ± 7 |
| Perfusion fluid_08 | 478 ± 17 | 22.8 ± 0.4 | 113.31 ± 1.13 | 6.20 ± 0.08 | 0.32 ± 0.01 | 86.80 ± 1.39 | 92 ± 4 |
| Perfusion fluid_09 | 303 ± 4 | 33.8 ± 5.1 | 98.83 ± 2.75 | 5.62 ± 0.19 | 0.20 ± 0.02 | 74.29 ± 3.41 | 117 ± 3 |
| Perfusion fluid_10 | 277 ± 4 | 23.2 ± 0.6 | 93.56 ± 0.95 | 5.24 ± 0.02 | 0.19 ± 0.01 | 67.99 ± 0.72 | 83 ± 7 |
| Perfusion fluid_11 | 334 ± 11 | 43.2 ± 4.6 | 105.68 ± 2.27 | 5.93 ± 0.03 | 0.24 ± 0.02 | 80.06 ± 0.53 | 123 ± 19 |
| Perfusion fluid_12 | 301 ± 1 | 25.3 ± 14.2 | 99.11 ± 1.28 | 5.52 ± 0.06 | 0.19 ± 0.01 | 73.09 ± 0.75 | 86 ± 13 |

| | | | | | | | |
|---|---|---|---|---|---|---|---|
| Perfusion fluid_13 | 301 ± 1 | 24.7 ± 1.3 | 99.64 ± 0.36 | 5.53 ± 0.01 | 0.20 ± 0.01 | 72.29 ± 0.21 | 92 ± 10 |
| Perfusion fluid_14 | 322 ± 4 | 33.1 ± 1.0 | 94.49 ± 1.58 | 5.85 ± 0.08 | 0.42 ± 0.01 | 74.67 ± 1.42 | 113 ± 14 |
| Perfusion fluid_15 | 310 ± 3 | 35.4 ± 1.3 | 90.62 ± 4.22 | 5.48 ± 0.22 | 0.43 ± 0.04 | 76.95 ± 3.15 | 98 ± 9 |
| Perfusion fluid_16 | 297 ± 2 | 33.9 ± 1.3 | 90.95 ± 1.22 | 5.57 ± 0.03 | 0.45 ± 0.01 | 71.94 ± 0.62 | 91 ± 13 |
| Perfusion fluid_17 | 302 ± 1 | 21.6 ± 0.9 | 93.42 ± 3.77 | 5.81 ± 0.22 | 0.44 ± 0.04 | 70.69 ± 2.95 | 104 ± 5 |
| Perfusion fluid_18 | 308 ± 3 | 19.3 ± 0.6 | 89.12 ± 3.45 | 5.55 ± 0.16 | 0.41 ± 0.03 | 73.87 ± 2.95 | 105 ± 7 |

(continued)

| Perfusion fluid_19 | 339 ± 7 | 19.8 ± 1.0 | 145.78 ± 1.19 | 4.35 ± 0.04 | 0.57 ± 0.01 | 103.68 ± 0.61 | 115 ± 11 |
|---|---|---|---|---|---|---|---|
| Perfusion fluid_20 | 295 ± 2 | 20.3 ± 0.7 | 99.62 ± 0.54 | 5.50 ± 0.05 | 0.21 ± 0.01 | 70.82 ± 0.85 | 97 ± 6 |
| Perfusion fluid_21 | 301 ± 2 | 31.4 ± 2.8 | 101.76 ± 0.21 | 5.42 ± 0.02 | 0.10 ± 0.00 | 71.92 ± 0.64 | 74 ± 6 |
| Perfusion fluid_22 | 305 ±3 | 24.2 ± 1.3 | 100.25 ± 0.95 | 5.53 ± 0.02 | 0.10 ± 0.00 | 73.96 ± 0.83 | 113 ± 12 |
| Perfusion fluid_23 | 305 ± 1 | 24.5 ± 0.9 | 103.77 ± 0.33 | 5.37 ± 0.01 | 0.10 ± 0.00 | 72.97 ± 0.63 | 71 ± 6 |
| Perfusion fluid_24 | 312 ± 3 | 25.1 ± 1.5 | 103.28 ± 0.35 | 5.65 ± 0.02 | 0.11 ± 0.01 | 74.64 ± 0.19 | 81 ± 13 |
| Perfusion fluid_25 | 282 ± 2 | 25.8 ± 1.4 | 103.22 ± 0.25 | 5.03 ± 0.02 | 0.11 ± 0.00 | 70.50 ± 0.29 | 77 ± 10 |
| Perfusion fluid_26 | 274 ± 2 | 15.9 ± 0.2 | 96.39 ± 0.27 | 4.97 ± 0.01 | 0.18 ± 0.01 | 69.26 ± 0.26 | 83 ± 12 |
| Perfusion fluid_27 | 305 ±0 | 25.3 ± 0.6 | 101.05 ± 0.44 | 5.91 ± 0.03 | 0.20 ± 0.00 | 73.19 ± 0.35 | 75 ± 12 |
| Perfusion fluid_28 | 309 ± 1 | 33.0 ± 1.7 | 102.07 ± 0.24 | 5.40 ± 0.02 | 0.20 ± 0.01 | 74.05 ± 0.07 | 93 ± 12 |
| Perfusion fluid_29 | 342 ± 2 | 20.8 ± 2.3 | 102.84 ± 0.33 | 5.68 ± 0.03 | 0.22 ± 0.00 | 74.61 ± 0.44 | 107 ± 14 |

| Perfusion fluid_30 | 406 ± 7 | 18.5 ± 0.8 | 104.26 ± 0.48 | 5.83 ± 0.03 | 0.21 ± 0.01 | 75.75 ± 0.73 | 100 ± 15 |
|---|---|---|---|---|---|---|---|
| Perfusion fluid_02_II production method | 320 ± 1 | 17.8 ± 0.9 | 105.20 ± 0.20 | 5.63 ± 0.03 | 0.22 ± 0.01 | 75.77 ± 0.58 | 91 ± 9 |
| Perfusion fluid_04_II production method | 343 ± 4 | 21.27 ± 3.45 | 118.90 ± 4.27 | 6.73 ± 0.06 | 0.26 ± 0.00 | 91.00 ± 0.69 | 97 ± 13 |

**Table 37.** Stability results of perfusion fluid_02_II production method, emulsion particle size distribution.

| Storage time at 4 C° | Average diameter [nm] | PdI [-] |
|---|---|---|
| 2 days | 163.81 ± 1.43 | 0.054 ± 0.012 |
| 7 days | 220.52 ± 3.75 | 0.056 ± 0.019 |
| month | 239.29 ± 0.79 | 0.041 ± 0.012 |
| 2 months | 244.50 ± 2.25 | 0.106 ± 0.020 |
| 3 months | 252.23 ± 1.44 | 0.080 ± 0.018 |
| 4 months | 281.61 ± 2.67 | 0.097 ± 0.026 |
| 8 months | 317.09 ± 3.04 | 0.135 ± 0.016 |

**Table 38.** Stability results of perfusion fluid_04_II production method, emulsion particle size distribution.

| Storage time at 4 C° | Average diameter [nm] | PdI [-] |
|---|---|---|
| 3 days | 190.70 ± 1.50 | 0.068 ± 0.020 |
| 7 days | 213.46 ± 2.43 | 0.067 ± 0.019 |
| month | 238.63 ± 1.36 | 0.091 ± 0.045 |
| 8 months | 360.78 ± 22.79 | 0.260 ± 0.045 |

**Example III.**

**Perfusion of isolated organs ex vivo.**

Organ transplantation is a modality used in the end-stage failure of an organ. The gold standard for ex vivo organ preservation is pumping (perfusion) through the vascular bed suitable fluid at a low temperature, which reduces the metabolic rate and alleviates the effects of ischaemia.

In order to demonstrate the efficacy of the perfusion fluid according to the invention, a number of the perfusion experiments were carried out on isolated organs (kidneys, liver and heart).

**Example III. Perfusion of porcine kidney ex vivo**

Efficacy studies of the perfusion fluids were conducted on isolated porcine kidneys (domestic Polish White Hog, females weighing 40-50kg) in the donation after cardiac death (DCD) model subjected to a 7-30 minutes warm ischaemic time (DCD model). This model involves organ retrieval after cardiac arrest in the organ. Maximum ischaemic time of the organ at physiological temperature (37 degrees C.) is 30 minutes. After retrieval, the organ undergoes preservation. The perfusion process was carried out using a prototype organ perfusion device that is the subject of an independent patent, application number P.449924. The main parameter used for assessing the efficiency of the kidney preservation and activity of the organ after reperfusion is the change in creatinine concentration in the reperfusion fluid as a function of the reperfusion duration. Creatinine clearance measured for the entire reperfusion process is included as an additional parameter. (von Horn C, Minor T. Improved approach for normothermic machine perfusion of cold stored kidney grafts. Am J Transl Res. 2018 Jun 15;10(6):1921-1929. PMID: 30018731; PMCID: PMC6038067).

**Example III.1.1. Pre-operative procedure**

The pigs were being acclimatised at the Jan Kielanowski Institute of Animal Physiology and Nutrition in Jablonna for at least 2 weeks. Eight hours before the operative procedure, food and drink were withdrawn.
Anaesthesia was started by intramuscular premedication: medetomidine 1mg/m2, midazolam 5mg/m2, ketamine 8mg/kg body weight, methadone 0.2mg/kg body weight. The animal was then transported to the operating theatre. 1.1 x 32 mm or 0.9 x 25 mm intravenous cannula was inserted into the marginal ear vein or the saphenous vein or the caudal vein. Drugs for induction of anaesthesia - propofol 1-2 mg/kg and lidocaine 1mg/kg - were administered through the cannula.
The animal was placed in the sternal position on the operating table. Preoxygenation of 3l/min with a mask was started, then monitoring of vital functions (spO2, PR, NIBP: SYS/DIA, MAP, temperature) was connected.
The isoflurane concentration was set at 2-2.5% and the oxygen flow rate at 2l/min and the pig was carefully being observed. Normal anaesthesia was induced by reducing the heart rate below 150 beats/minute and blood pressure (systolic value below 100 mmHg) no movement of the pig was observed. The incision site was anaesthetised intrathecally with 2% lidocaine; the total dose of lidocaine used mustn't have been more than 8 mg/kg. The concentration of isoflurane was gradually reduced to 1-1.5%.
Fluid therapy with lactate or Ringer's acetate solution was then initiated. A suitable fluid dose was calculated from the following formulas:

$$\text{Fluid deficiency (l): body weight (kg) x [\% dehydration/100)}$$

$$\text{daily fluid requirements: body weight (kg)0.75 x 70, average 40-60ml/kg/day}$$

$$\text{running losses: average 1ml/kg/h x body weight (kg)}$$

Under sterile conditions, the skin was incised caudally from the angle of the mandible, then the salivary gland was dissected bluntly. The external jugular vein was exposed under the salivary gland. The vessel was dissected and ligated intracranially from the planned site of the access using Silk 3-0 suture. For the catheter insertion, the vessel was pinched, e.g. with a thin peanut. A small incision was made in the vessel with vascular scissors and the catheter was inserted in the distocardial side. Using Silk 3-0 thread, a loop was placed over the vessel to fixate the catheter to the vein.
The free side of the catheter was threaded onto a suitably large cutting needle and brought to the skin surface dorsal to the incision line. The incision line was closed with a mattress suture. 20mg/kg of cefuroxime was administered 30 minutes before the start of surgery and every 90 minutes during surgery intravenously. Shotapen intramuscularly at a dose of 1 ml per 25kg and maropitant subcutaneously at a dose of 1mg/kg were also administered. Intramuscular metamizole at a dose of 50 mg/kg and intravenous bolus fentanyl at a dose of 1.5-3 ug/kg were administered as analgesic protection, followed by a CRI of fentanyl in 0.9% NaCl throughout the operation at a dose of 1.5 ug/kg/h. Veterinary ophthalmic ointment was applied to the eyes to prevent dryness during anaesthesia.

**Example III.1.2. Operating procedure**

After disinfecting the surgical field and spreading the drape, a 25 cm long median incision was made and a retractor was inserted. The large and small bowel were covered with a drape and placed on the side opposite the organ to be retrieved for optimal access.

The ureter and the kidney were freed from any adjacent tissue using coagulation.

The renal vein and renal artery were dissected using coagulation until their exit from the inferior vena cava and aorta, respectively, was freed.

After complete dissection, the kidney was tied (silk thread, 3-0) and the ureter was cut distally. An ice bowl and sterile organ bag were prepared.

100 IU/kg body weight of intravenous heparin was administered, the condition was then observed for 5 minutes. The renal artery close to the aorta and the renal vein close to the vena cava were then occluded with vascular clips for up to 30 minutes. Subsequently, the organ was harvested and a cannula was immediately inserted into the renal artery to start flushing the vascular bed. 500 ml cold Belzer UW solution (or equivalent e.g. Store Protect) with 5000 U/l heparin was used to flush out the blood. The renal vein and the ureter were also cannulated. The kidney was sterile packaged and stored in the refrigerator until the perfusion was initiated. After retrieval, the animal was euthanized by intravenous administration of pentobarbital at a dose of 140 mg/kg body weight.

**Example III.1.3.1. Normothermic perfusion of isolated kidney 260122N2** - **DCD model WIT: 10min, CIT: 60h**

Kidney from a female weighing approximately 40 kg. Organ harvesting followed a 10-minute warm ischaemia, the kidney was stored for 60h in hypothermia: on ice or in a refrigerator). The weight of the kidney before the perfusion was 100g and after the perfusion 130g. Perfusion was carried out with perfusion fluid_04_II production method. The normothermic perfusion plan included:

(a) initial hypothermic perfusion for 2.5 to 4 hours

(b) controlled, linear rise in temperature from 5°C to 37 °C for 90 minutes

(c) normothermic perfusion at 37°C for 24 hours

(d) controlled in-line cooling of the organ from 37°C to 5 °C for 90 minutes

(e) Langendorff model reperfusion at 37°C and MAP (pl. mean arterial pressure) 90 - 100 mmHg using Krebs-Henseleit solution enriched with albumin to simulate reperfusion in the recipient's body.

Urine production started at 6th hour of the trial and was low, however it allowed biochemical determinations to be made.

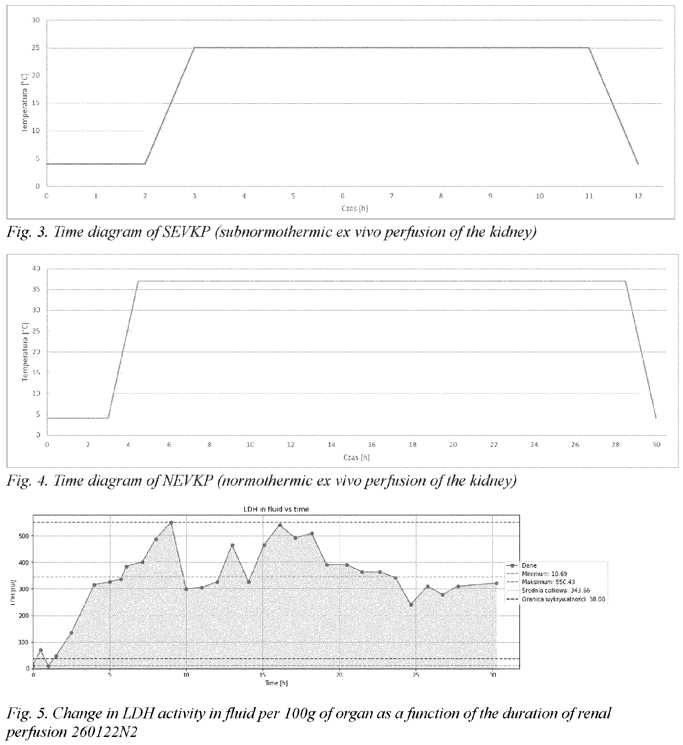

LDH activity in the fluid (see Fig. 5.) fluctuates strongly during the perfusion. The level of the parameter increases strongly during the first 9 hours of the process. The increase is caused by raising of the perfusion fluid's temperature to 37°C. It leads to precipitation of metabolic activity of the organ. Then it drops to a lower level, around 300 U/l. Increase is observed again around the 15th hour. From the 17th hour, a progressive decrease in enzyme activity is observed, with stabilisation occurring in the last three hours of the process.

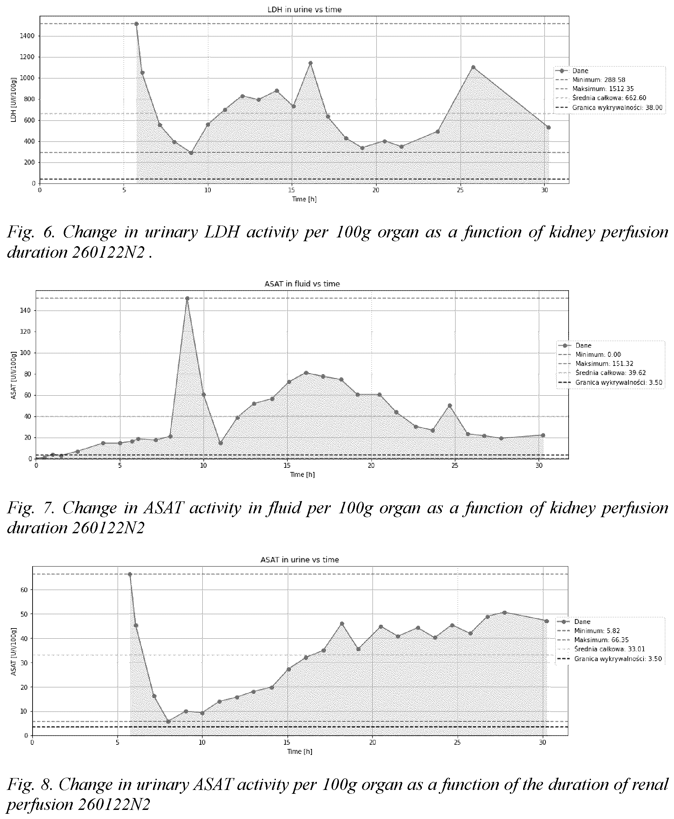

The urinary LDH activity curve (See Fig. 6.) changes intensely during the perfusion. At the 6th hour of the process, the level of the marker is at a very high level. However, it steadily decreases, reaching a minimum at the 9th hour of the process. From then on, the level starts to rise again, reaching value about 1,100 U/l. In the following hours, a decrease in LDH activity and a temporary stabilisation at a low level is observed. During the last hours of perfusion, the activity increases and exceeds the level of 1000 U/l. However, at the end of the process, the level of the parameter decreases to a below-average value.

The ASAT activity curve (See Fig. 7.) has an increasing trend. During the first eight hours of the perfusion, ASAT remains at a low activity level, slightly increasing. At the 9th hour of the process, there is an intense increase in the level of the parameter. From the 11th to the 16th hour of the process, a progressive increase is observed. Then the upward trend turns into a downward one. The decrease continues to the 26th hour, with a deviation peak at the 24th hour of the process. By the end of the perfusion, a stabilisation of the parameter level is observed.

The urinary ASAT activity (see Fig. 8.) is very high at the beginning of the process and reaches a value of approximately 66 U/l, the highest value during the entire perfusion. At the 8th hour of the process, a decrease in activity is observed. From the 9th hour, an increase in the level of the parameter is observed, this condition maintained until the very end of the process. There are local decreases in the level, however, when looking at the entire experiment the trend is upwards.

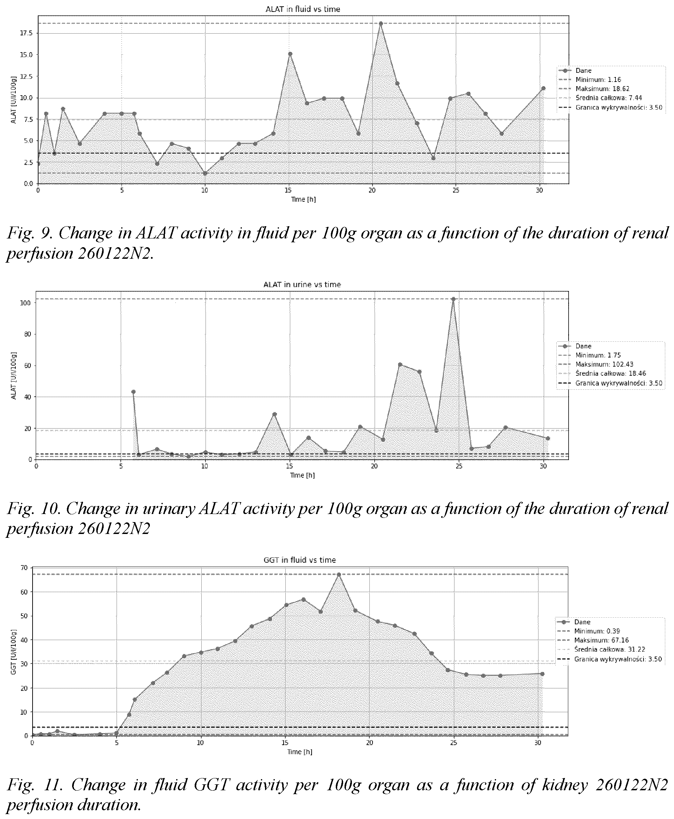

Curve of ALAT activity in the perfusion fluid (See Fig. 9.) is irregular. It can be seen that the level of the parameter alternately increases and decreases during the first part of the process, until around the 14th hour it remains just above the limit of quantification. In the second half of the experiment, greater fluctuations of the parameter are noticeable, but the trend of alternating increases and decreases is maintained.

Urinary ALAT activity (see Fig. 10.) remains at a level just above the parameter's limit of quantification for most of the process. An upward and downward trend is noticeable, the parameter has a very irregular course. The highest activity value is observed at 24th hour of the process (102 U/l).

The value of GGT activity in the perfusion fluid (see Fig. 11.) remains below the limit of quantification for the first 5 hours of the process. Thereafter, an increase in the level of the parameter is observed, this phenomenon is probably related to raising the temperature of the fluid to 37°C, with a maximum of 67 U/l at around 18hr of the perfusion. From then, the curve tends to decrease, until the end of the last four hours of the process, GGT activity stabilises and remains at around 25 U/l level.

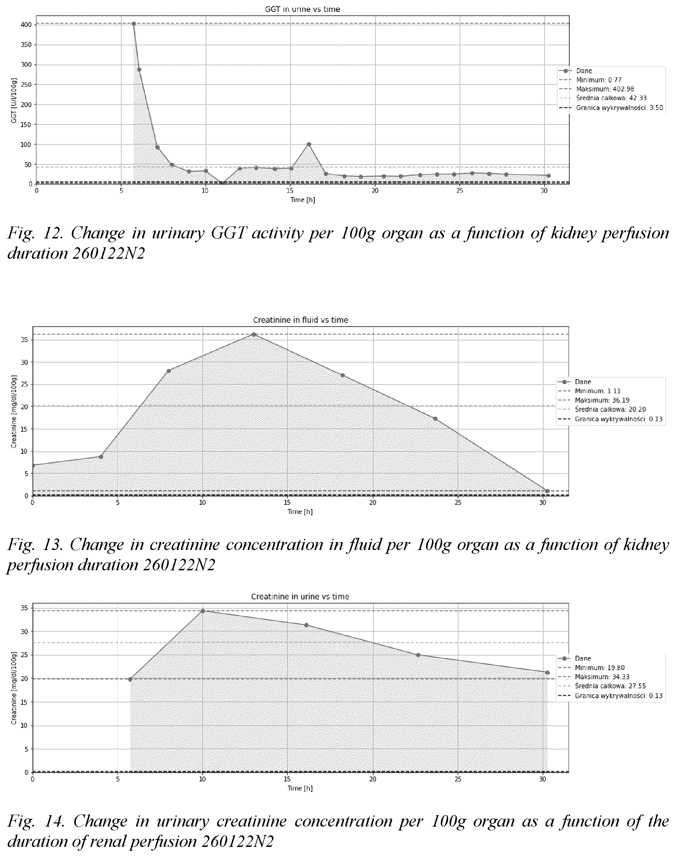

Urinary GGT activity (see Fig. 12.) is high at the 6th hour of the process, at 402 U/l level, however, it starts to decrease intensely in the following hours of the perfusion. From the 9th hour onwards, the activity remains at a low level, nevertheless, it is still higher than the limit of quantification. This trend is observed until the end of the experiment.

The creatinine concentration in the perfusion fluid (See Fig. 13.) increases from the beginning of the process until around the 13th hour of the perfusion, when the value reaches a maximum of 36 U/l. This is due to the rise in the temperature of the perfusion fluid to 37°C and consequently, acceleration of the metabolic processes in the organ. From the half the time of the perfusion, the level of the parameter begins to decrease, which indicates that the creatinine in the urine is being excreted. This is a confirmation that the kidney works and filters the perfusion fluid correctly.

Creatinine concentration in the urine (see Fig. 14.) at the beginning of its production is about 20 mg/dl, when at the 10th hour of the process it is already 35 mg/dl. In the following hours, a decrease in the concentration of the parameter is observed, but the decrease is smaller than that observed for creatinine concentration in the perfusion fluid. This is a confirmation of the correct functioning of the kidney, which correctly filters the perfusion fluid and excretes excess metabolites.

In summary, porcine kidney subjected to the ischaemic injury was successfully perfused. The kidney was initially subjected to ischaemia at physiological temperature (37°C), followed by further long-term simple hypothermia for a few dozen of hours. Based on the aforementioned conditions, kidney was damaged severely before the perfusion. The data obtained from the perfusion process allowed to consider the overall condition of the organ after perfusion as fit for transplantation. The kidney started working during the reperfusion process by producing urine and filtering creatinine.

**Example III.1.3.2. Normothermic perfusion of isolated kidney 070223N2** - **DCD model WIT: 30min, CIT: 32h**

Kidney from a female weighing approximately 40 kg. Organ retrieval followed a 30-minute warm ischaemia, the kidney was stored for 32 hours under cold conditions: on ice or in a refrigerator. Kidney weight before perfusion was 105g and 182g after the perfusion. Perfusion was carried out using perfusion fluid_04_II production method. The normothermic perfusion scheme included:

(a) initial hypothermic perfusion for 2.5 -4 hours

(b) controlled, linear rise in temperature from 5°C to 37 °C Cover 90 minutes

(c) normothermic perfusion at 37°C for 24 hours

(d) controlled in-line cooling of the organ from 37°C to 5 °C for 90 minutes

(e) Langendorff model reperfusion at 37°C and MAP (mean arterial pressure) 90 - 100 mmHg using Krebs-Henseleit solution enriched with albumin to simulate reperfusion in the recipient's body.

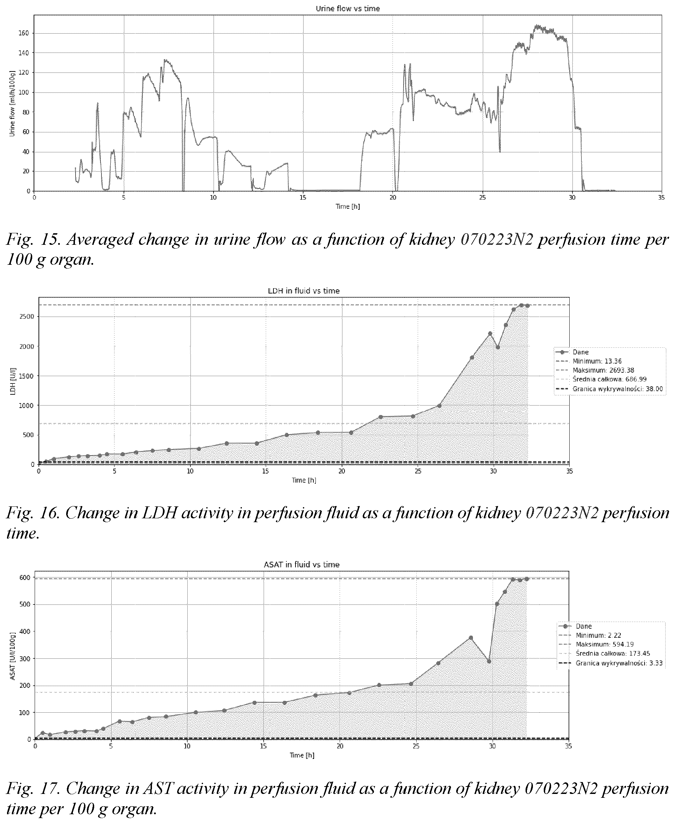

The kidney started to produce urine at the 3rd hour of the process. (See Fig. 15.). Fig. 15. urine flow was irregulardue to the sensitivity of the flowmeter to any disturbance.

The activity of LDH in the fluid (see Fig. 16.) gradually increases during perfusion, starting at values below the limit of quantification at the start of the process and reaching very high values of approximately 2600 U/l at the end of the experiment. The increase is related to the heating of the perfusion fluid up to 37°C.

ASAT activity in the fluid (see Fig. 17.) gradually increases during perfusion, starting at values below the limit of quantification at the start of the process and reaching values in the order of 600 U/l at the end of the experiment. The increase in the level of the parameter is related to the heating of the perfusion fluid to 37°C.

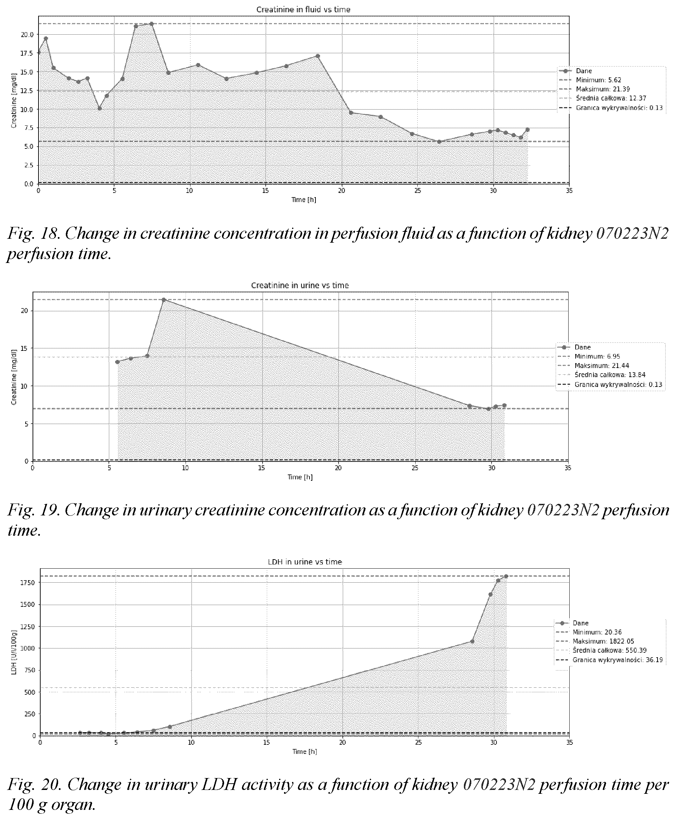

The creatinine concentration in the perfusion fluid (See Fig. 18.) decreases for the first five hours. Thereafter, the level of the parameter begins to rise slightly, reaching a value of over 20 mg/dl around the 7th hour of the process. Then, from 9 o'clock, it starts to oscillate around a value of 15 mg/dl, and from the 19th hour of the process it decreases steadily.

Creatinine concentration in the urine (See Fig. 19.) increases with the beginning of the urine production. Creatinine concentration in the urine decreases significantly and reaches its lowest value around the 30th hour of perfusion. The values measured at the very end of perfusion are significantly lower than those at the beginning of urine production. This condition confirms correct both functioning of the organ and creatinine filtration.

The activity of LDH in the urine (see Fig. 20.) is approximately constant during almost the entire experiment. Only at the end of the perfusion, around 28 hour, the activity increases strongly, and reaches values above 1,000 U/l.

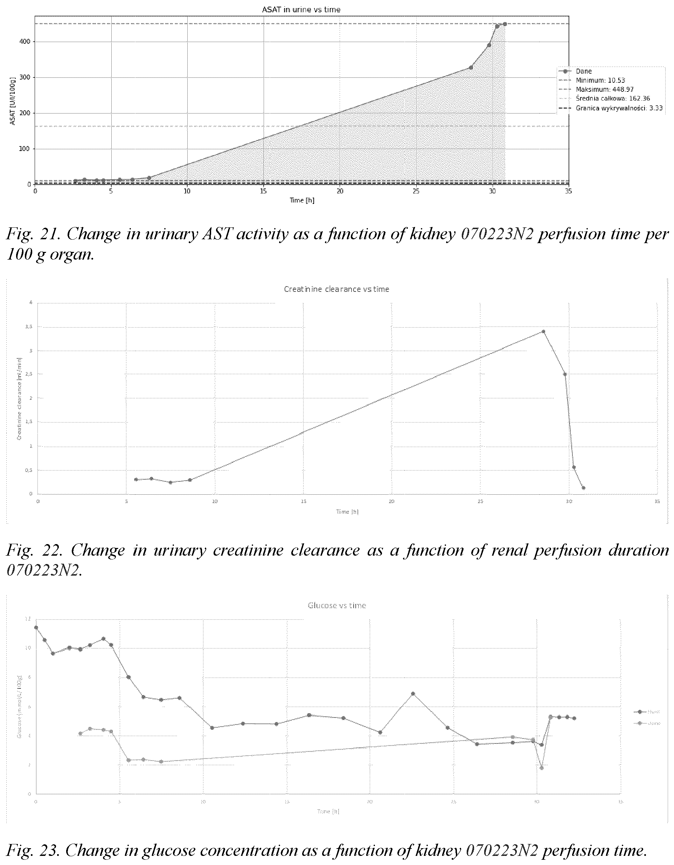

Urinary AST activity (see Fig. 21.) increases strongly during the perfusion. Initial values are located quite close to the limit of quantification. However, it changes with the perfusion duration and at the end of perfusion values reach levels of approximately 450 U/l.

Creatinine clearance (See Fig. 22.) is a parameter that necessary to assess the filtration capacity of the glomeruli. The amount of creatinine present in the perfusion fluid and urine is compared. The parameter increases during the process. However, the clearance value decreases at the end of the experiment. This may indicate a lower ability of the kidney to filter metabolites at the end of perfusion, this phenomenon may occure due to the low temperature.

Concentration of glucose in the perfusion fluid (see Fig. 23, blue line) successively decreases during the course of perfusion. This phenomenon occurs due to the increasing organ's demand for this component which is related to the accelerating metabolic processes. This demand increases specifically between the 4th and 30th hour of the process, where the temperature of the fluid is being rised from 4°C to 37°C. The urinary glucose concentration (orange line) at the start of the urine production is about 4 mmol/L, it falls to about 2 mmol/L by the 5th hour of the process. In the last hours of the perfusion t, glucose concentration rises slightly, which is due to the fact that glucose is externally dosed once the 4 mmol/L limit is reached.

In summary, porcine kidney subjected to significant ischaemic injury was successfully perfused. The kidney was initially subjected to ischaemia at physiological temperature (37°C), followed by further long-term simple hypothermia for a few dozen of hours. Based on the aforementioned conditions, the damage to the kidney before perfusion can be considered severe. The data obtained from the perfusion process allow the overall condition of the organ after perfusion to be determined as fit for transplantation. The kidney started working during the reperfusion process by producing urine and filtering creatinine.

**Example ID. 1.3.3. Normothermic perfusion of isolated kidney 270223N1** - **DCD model WIT: 30min, CIT: 16h**

Kidney from a female weighing approximately 40 kg. Organ harvesting followed a 30-minute warm ischaemia, the kidney was stored 16h under cold conditions: on ice or in a refrigerator. The weight of the kidney before perfusion was assessed at 128g and after perfusion at 230g. Perfusion was performed with perfusion fluid_04_II production method. The normothermic perfusion plan included:

(a) initial hypothermic perfusion for 2.5 to 4 hours

(b) controlled, linear rise in temperature from 5°C to 37°C over 90 minutes

(c) normothermic perfusion at 37°C for 24 hours

(d) controlled in-line cooling of the organ from 37°C to 5°C for 90 minutes

(e) Langendorff model reperfusion at 37°C and MAP (mean arterial pressure) 90 - 100 mmHg using Krebs-Henseleit solution enriched with albumin to simulate reperfusion in the recipient's body.

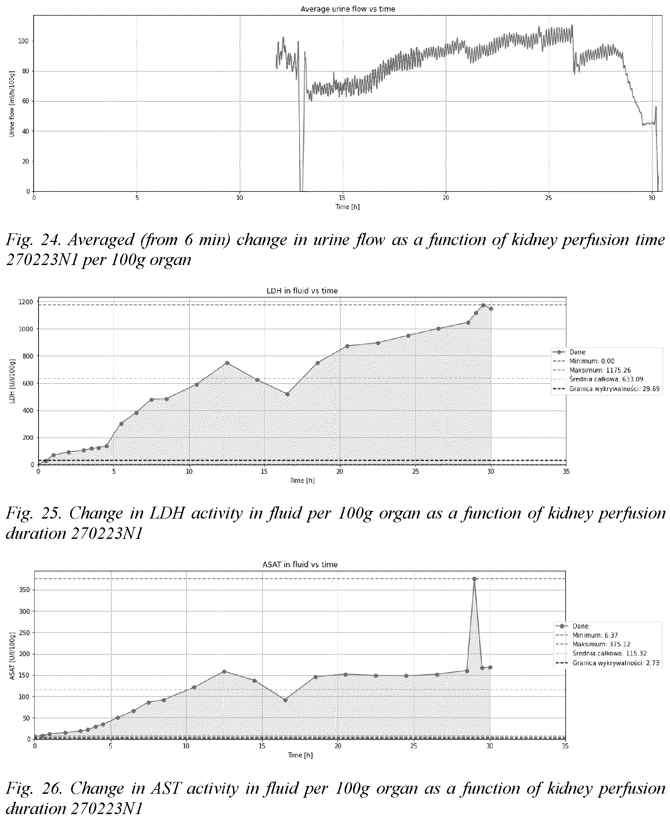

The kidney started to produce urine around the 6th hour of the perfusion process (See Fig. 24.).

LDH activity in the fluid (see Fig. 25.) gradually increases during perfusion, starting at a value around the limit of detection at the start of the process and reaching high values of approximately 1,200 U/l at the end of the experiment. The increase in the level of the parameter is related to the heating of the perfusion fluid to 37°C and the increase in metabolic activity of the organ

The activity of ASAT in the fluid (see Fig. 26.) gradually increases during perfusion, starting below the limit of detection at the start of the process and reaching values of 150 U/l around the 15th hour of the process. After the 15th hour of the process, the level of the parameter stabilises at the same level. Peak of activity value is observed at the 28th hour of the process, but the parameter level returns to the previously established value.

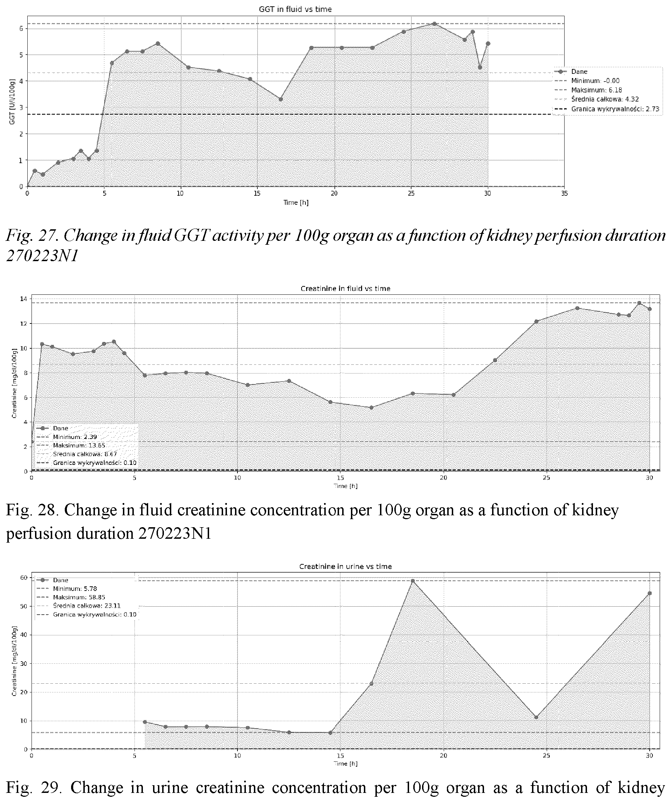

GGT activity (see Fig. 27.) maintain limit of detection up to 5th hour of perfusion. From the 6th hour the values start to increase reaching levels of approximately 5 U/l. Around the 9th hour of the trial, a downward trend is observed. At the 20th hour, the values stabilise at around 5 U/l until the end of the experiment. The level of the damage marker is not high for the entire process.

The concentration of creatinine in the fluid (See Fig. 28.) for the first 5 hours of the process is at a similar level. After 5 hours, a systematic decrease is observed, indicating the beginning of urine production and creatinine excretion. After the 20th hour of the process, an increase in the concentration of the parameter is observed.

The concentration of creatinine in the urine (See Fig. 29.) at the beginning of its production maintains level of about 10 mg/dl, with a slight decrease around the 8th hour of perfusion. Around the 13th hour of the process, a slight decrease in the parameter is noticeable; however, at the 19th hour of the process, an increasing creatinine concentration can be perceived.

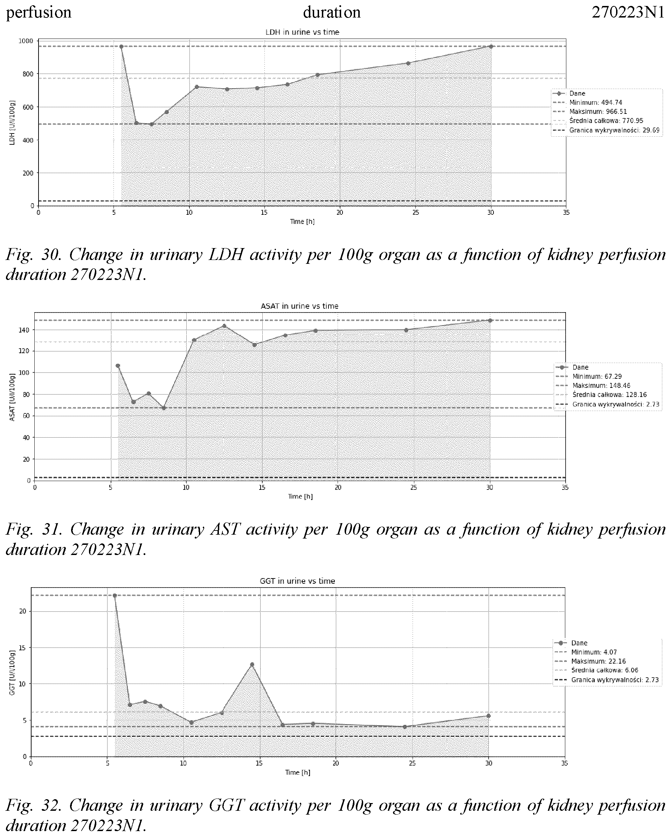

The urinary LDH activity (see Fig. 30.) increases during the process. From the beginning of urine production, the parameter reaches high values. At the 6th hour of the process, a decrease in the level of the parameter is visible, although as the process continues, the level of the parameter is increasing, reaching a value of approximately 1,000 U/l in the last hour .

The level of urinary AST activity (See Fig. 31.) increases during the perfusion. At the 6th hour of the process, a decrease in activity is observed, such level persists for another 2 hours.

Thereafter, the level of the parameter increases to approximately 140 U/l and maintain at this level until the end of the perfusion.

The value of GGT activity in the urine (see Fig. 32.) at the beginning of its production reaches a very high level, over 20 U/l. In the next hour, the parameter strongly decreases its level and stays until the end of the process, with a single peak value around the 17th hour of the experiment.

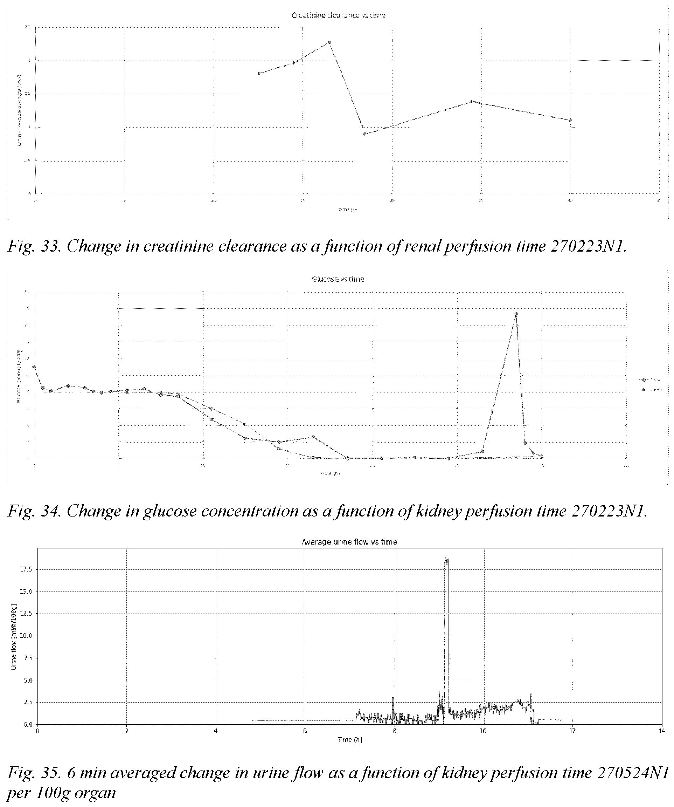

Creatinine clearance (See Fig. 33.) is a parameter that allows to assess the filtration capacity of the glomeruli. The amount of creatinine present in the perfusion fluid and urine is compared. The parameter increases in value from the 13th hour of the trial, however, the clearance value decreases at the end of the experiment.

The concentration of glucose in the perfusion fluid (See Fig. 34., blue line) successively decreases during perfusion. A concentration peak is observed at the 28th hour of the process, which is most likely due to an error in the measurement. The decrease in concentration is caused by the organ's demand for glucose in carrying out metabolic processes. This demand increases between the 4th and 30th hour of the process, where the fluid temperature changes from 4°C to 37°C. This results in an acceleration of metabolic processes. The urinary glucose concentration (orange line) at the start of production is around 4 mmol/L, lower to virtually zero by the 8th hour of the process.

In summary, a porcine kidney subjected to significant ischaemic injury was successfully perfused. The kidney was initially subjected to ischaemia at physiological temperature (37°C), followed by further long-term simple hypothermia for several hours. Based on the aforementioned conditions, the damage to the kidney before perfusion can be considered high. The data obtained from the perfusion process allow the overall condition of the organ after perfusion to be determined as suited for transplantation. The kidney started working during the reperfusion process by producing urine and filtering creatinine.

**Example III.1.3.4. Subnormothermic perfusion of isolated kidney 270524N1** - **DCD model WIT: 20 min, CIT: 1h**

Kidney from a female weighing approximately 47 kg. The organ was harvested after 20 min of warm ischaemia, the kidney was stored 1h in cold conditions: on ice or in a refrigerator. Kidney weight was assessed before perfusion at 156g and after perfusion at 184g. Perfusion was performed with perfusion fluid_02_II production method. The perfusion plan under subnormothermic conditions included:

(a) initial hypothermic perfusion for 2h
(b) controlled, linear increase in temperature from 4°C to 25°C over 60 minutes
(c) subnormothermic perfusion at 25°C for 8 h
(d) controlled, linear cooling of the body from 25°C to 4°C for 60 minutes
(e) reperfusion in the Langendorff model at 37°C and MAP 90 - 100 mmHg using Krebs-Henseleit solution enriched with albumin to simulate reperfusion in the recipient's body.

The production of urine (See Fig. 35.) started around the 5th hour of perfusion. (see Fig. 35.) Urine flow is irregular, this is due to the sensitivity of the flow meter to any disturbance.

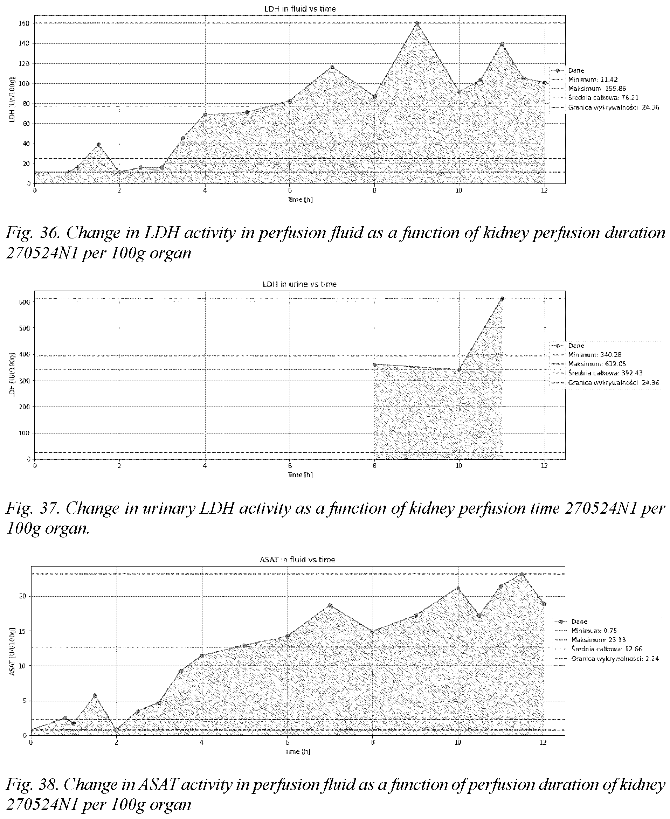

The activity of LDH in the perfusion fluid (see Fig. 36.) increases during the process. In the first three hours, it remains almost constantly below the limit of detection. At 3.5 hours, after finishing the warming of the perfusion fluid, the enzyme activity increases significantly. It's due to the higher rate of metabolic processes compared to their rate during hypothermia. A continuation of the increase is observed until the 7th hour of perfusion. In the last five hours of the process, the level of the parameter alternately increases and decreases.

LDH activity in the urine (see Fig. 37.) is high. Its level increases visibly towards the end of the process time.

The ASAT activity (see Fig. 38.) increases during process. In the first three hours, the level of the parameter keeps almost constantly below the limit of detection. In the 3rd hour, during warming of the perfusion fluid, the activity of the enzyme increases significantly. It's due to the higher rate of metabolic processes compared to their rate during hypothermia. A continuation of the increase is observed until the 7th hour of perfusion. In the last five hours of the process, the level of the parameter alternately increases and decreases.

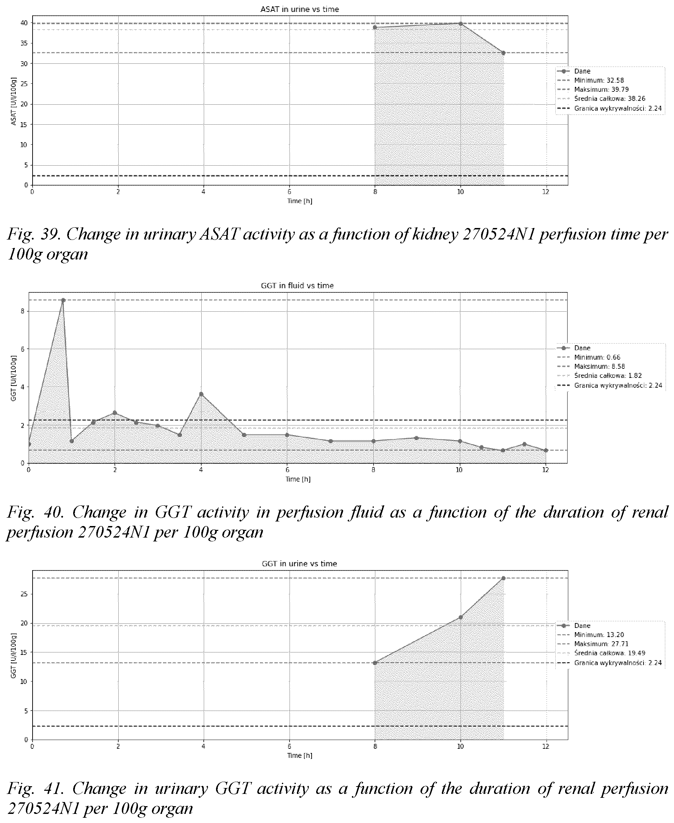

ASAT activity in urine (See Fig. 39.) is high. Its level decreases towards the end of the process time.

The level of GGT activity (see Fig. 40.) has an irregular pattern. In the first 30 minutes of the process, a peak in the activity of the enzyme is observed. Its level being the highest seen throughout the process. In the following hours, a strong decrease is observed, resulting in a stabilisation of the parameter level below the limit of detection or slightly above it.

Urinary GGT activity (see Fig. 41.) is high. Its level increases by the end of the perfusion.

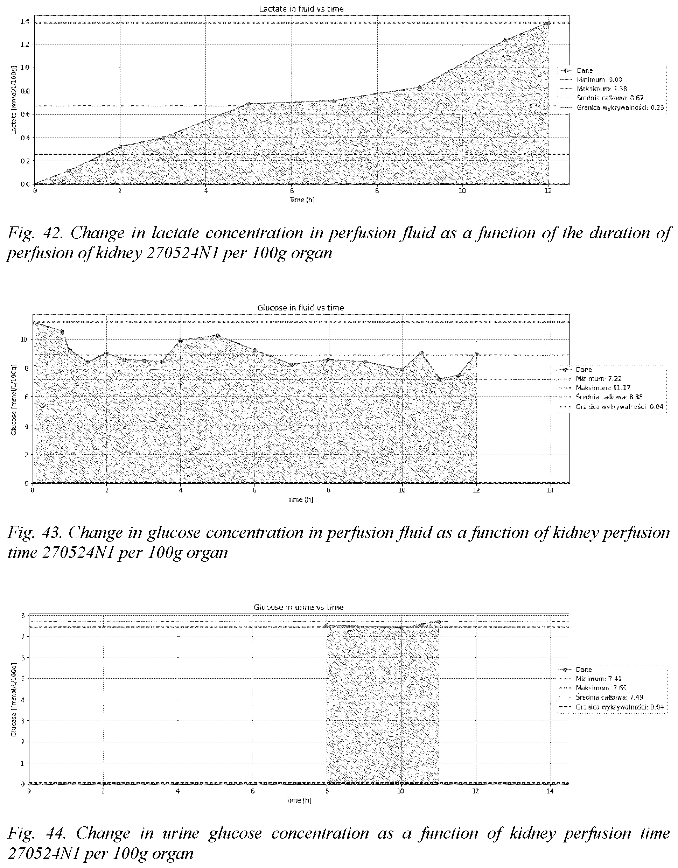

The lactate concentration (See Fig. 42.) increases during the process. Between 5th and 9th hour of the process, a slight decrease in acceleration of the parameter's concentration is observed.

The glucose concentration (see Fig. 43.), looking at the overall process, decreases during perfusion. In the first hour of the process, a decrease in the concentration value is observed. It keeps a similar level until the 4th hour. Over the next two hours, a slight increase is perceptible, but at the 7th hour of perfusion, the glucose concentration returns to the level it reached at the initial hours of the process. From the 10th to 12th hour of perfusion, glucose levels rise and fall alternately. By analysing the process, the normal functioning of the organ can be confirmed. Glucose was consumed by the kidney, which is the basis of the organ's function.

The urine glucose concentration (See Fig. 44.) remained constant during the last hours of the perfusion. Unfortunately, the amounts of urine in the earlier hours of perfusion were so small that it was not possible to make measurements for all time points.

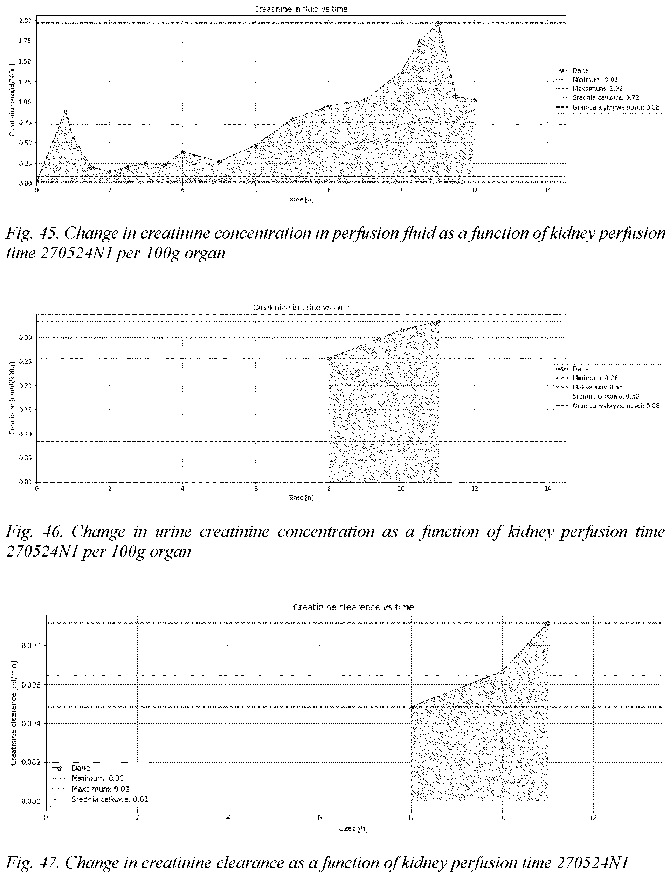

The creatinine concentration (See Fig. 45.) rises in the first 30 minutes of the process, this is most likely due to the eluting of residual blood from the organ. In the next 1.5 hours, the level drops practically to the limit of detection. After this time, it begins to rise gradually, reaching a maximum value at the 11th hour of the process. The increase of the parameter's concentration lower faster from 2nd to 5th hour than from 6th to 11th.

The concentration of creatinine in the urine (See Fig. 46.) increases during perfusion. This proves good organ functioning, as kidney removes excess creatinine from the perfusion fluid.

The creatinine clearance value (See Fig. 47.) rises during perfusion. This indicates that the organ is functioning normally as it excretes creatinine from the perfusion fluid.

In summary, a pig kidney subjected to significant ischaemic injury was successfully perfused. The kidney was subjected to ischaemia at physiological temperature (37°C). Based on the aforementioned conditions, the damage to the kidney prior to perfusion can be considered severe. The data obtained from the perfusion process allow the overall condition of the organ after perfusion to be determined as suited for transplantation. The kidney started working during the reperfusion process, by producing urine and filtering creatinine.

**Example III.1.3.5. Subnormothermic perfusion of isolated kidney 050624N1** - DCD model WIT: 21 min, CIT: 45 min

Kidney from a female weighing approximately 47 kg. The organ was harvested after 21 min of warm ischaemia, the kidney was stored 45 min under cold conditions: on ice or in a refrigerator. Kidney weight was assessed before perfusion at 115g and after perfusion at 119g. Perfusion was performed with perfusion fluid_02_II production method. The perfusion plan under subnormothermic conditions included:

(a) initial hypothermic perfusion for 2h
(b) controlled, linear increase in temperature from 4°C to 25°C over 60 minutes
(c) subnormothermic perfusion at 25°C for 8 h
(d) controlled, linear cooling of the body from 25°C to 4°C for 60 minutes
(e) reperfusion in the Langendorff model at 37°C and MAP 90 - 100 mmHg using Krebs-Henseleit solution enriched with albumin to simulate reperfusion in the recipient's body.

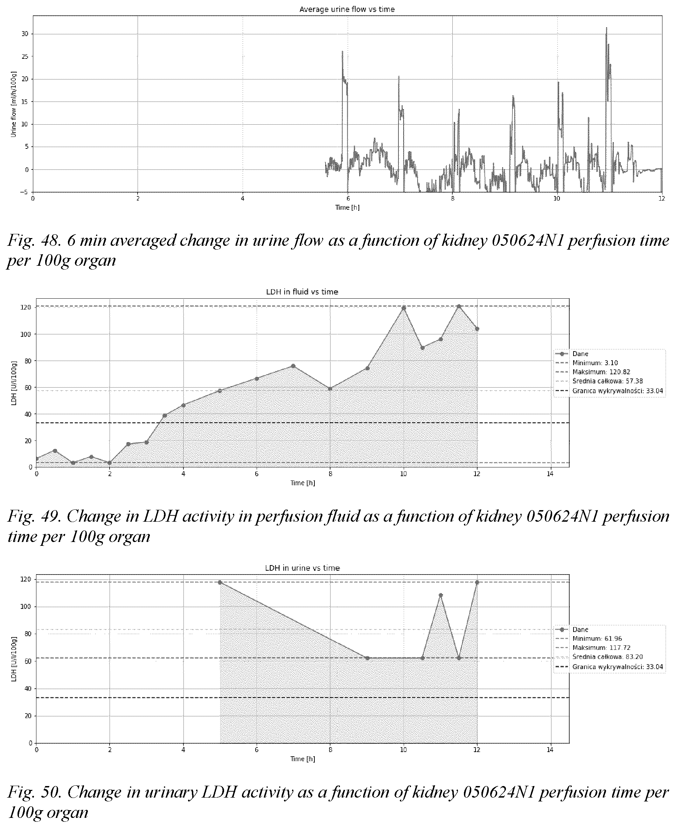

Urine production by the kidney (See Fig. 48). Started around the 5th hour of perfusion. Fig. 48. Urine flow is irregular, this is due to the sensitivity of the flow meter to any disturbance.

The LDH activity in the perfusion fluid (See Fig. 49.) increases during the process. In the first three hours, it maintains almost constantly below the limit of detection. At 3.5 hour, after the warming of the perfusion fluid is complete, the enzyme activity increases significantly. It's due to the higher rate of metabolic processes compared to their rate during hypothermia. A continuation of the increase is observed until the 7th hour of perfusion. In the last five hours of the process, the level of the parameter alternately increases and decreases.

The activity of LDH in urine (see Fig. 50.) reaches a high value of 120 U/l at the beginning of the process. In the following hours, an alternating increase and decrease in the level of the parameter is observed. Its value at the end of the process is also approximately 120 U/l.

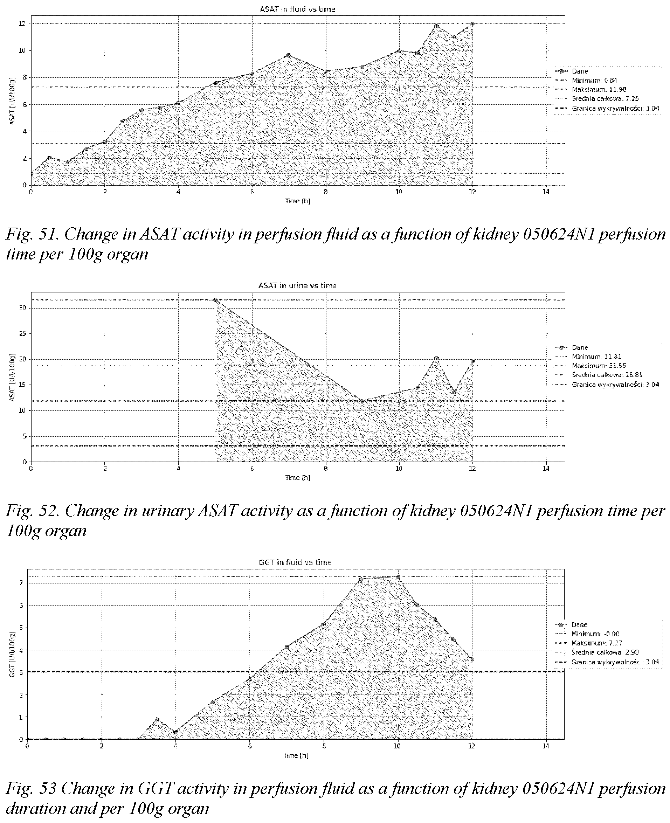

The ASAT activity in the fluid (see Fig. 51.) gradually increases during perfusion, starting at values below the limit of detection at the beginning and reaching values of about 12 U/l at the end of the experiment. The increase of level of the parameter is associated with heating of the perfusion fluid to 25°C. ASAT activity doesn't reach objectively high values.

Urinary ASAT activity (see Fig. 52.) reaches a high values of 36 U/l at the start of urine production. In the following hours, an alternating increase and decrease in the level of the parameter is observed. Its value at the end of the process approach values about 25 U/l.

GGT activity in the fluid (see Fig. 53.) during perfusion increases until the 9th hour of the process. At the beginning of the experiment, the parameter reaches values below the limit of detection, at the 7th hour of the process the level of the parameter starts to be significant. After the 9th hour of perfusion, a decrease in activity is observed. Values approach limit of detection at the end of the perfusion.

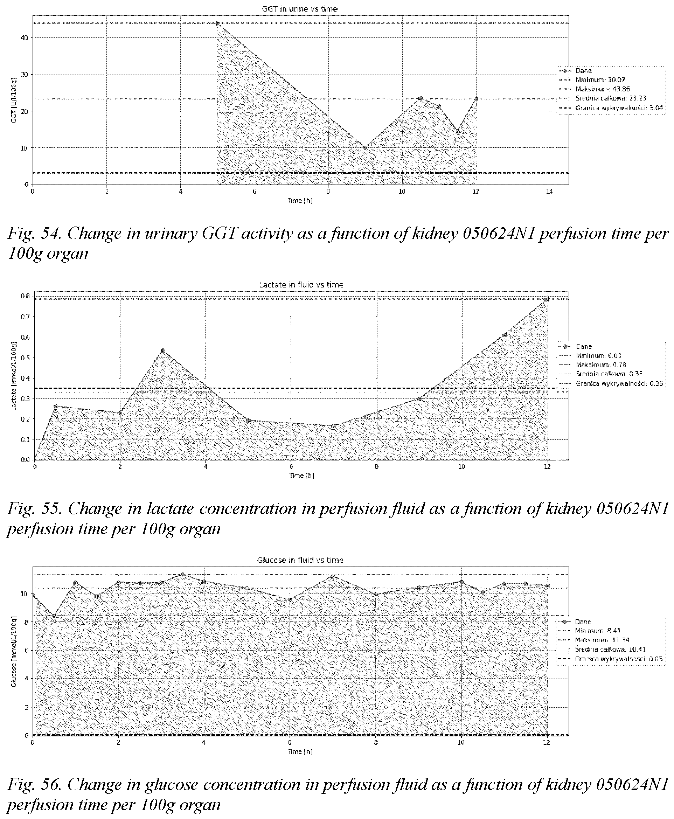

Urinary GGT activity (see Fig. 54.) is the highest at the start of urine production, it reaches value of 43 U/l. In the following hours, an alternating increase and decrease in the value of the parameter is observed. Its value at the end of the process is about 23 U/l.

The concentration of lactate in the perfusion fluid (See Fig. 55.) maintain below the limit of detection for most of the process duration, making these results irrelevant for drawing conclusions about the condition of the organ during perfusion.

The glucose concentration in the perfusion fluid (see Fig. 56.) oscillates around a constant level of 10 mmol/l during the process. Such situation may indicate reduced glucose consumption and, consequently, slower metabolic processes.

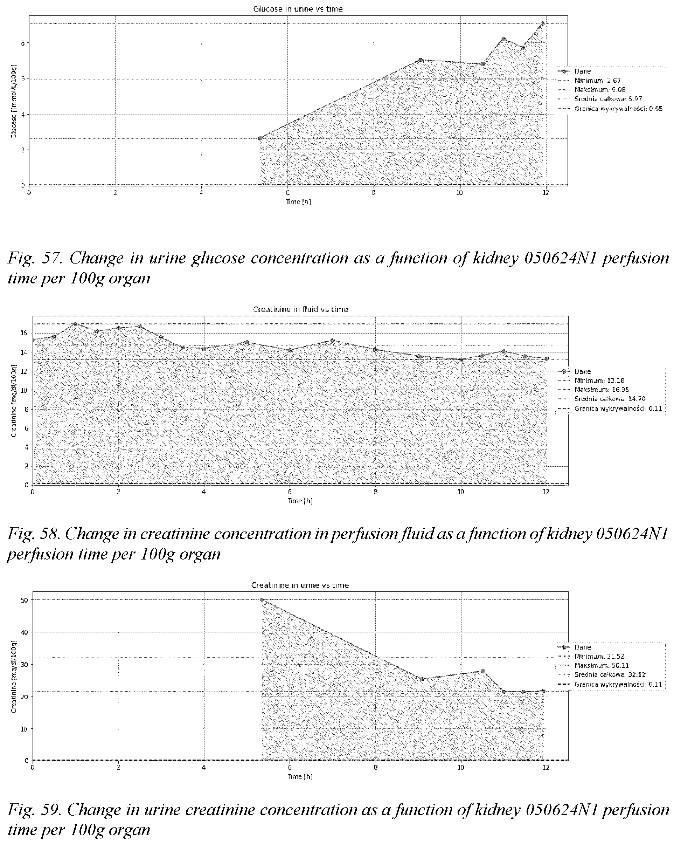

The urinary glucose concentration (See Fig. 57.) increases during perfusion time.

The creatinine concentration in the perfusion fluid (See Fig. 58.) decreases during the process. This indicates the excretion of creatinine in urine.

The urinary creatinine concentration (See Fig. 59.) decreases during perfusion. At the beginning of urine production, the parameter value is 50 mg/dl, which is a high concentration. At the end of the process, the creatinine level stabilises around a value of 21 mg/dl, which is a higher concentration than in the fluid. This indicates properly conduction of urine concentration process

In summary, a pig kidney subjected to significant ischaemic injury was successfully perfused. The kidney was initially subjected to ischaemia at physiological temperature (37° C), followed by further simple hypothermia. Based on the aforementioned conditions, the kidney damage prior to perfusion can be considered high. The data obtained from the perfusion process allow the overall condition of the organ after perfusion to be determined as suitable for transplantation. The kidney during perfusion produces urine and filters creatinine. In some cases, it can be observed that the creatinine concentration profile in the urine is higher than in the fluid which indicates that the organ is able to conduct a urine concentration process.

**Example III.1.3.6. Subnormothermic perfusion of isolated kidney 080624N1** - **DCD model WIT: 20 min, CIT: 22 min**

Kidney from a female weighing approximately 50 kg. The organ was harvested after 20 min of warm ischaemia, the kidney was stored 22 min under cold conditions: on ice or in a refrigerator. The weight of the kidney before perfusion was assessed at 193g and after perfusion at 230g. Perfusion was performed with perfusion fluid_02_II production method. The perfusion plan under subnormothermic conditions included:

(a) initial hypothermic perfusion for 2h
(b) controlled, linear increase in temperature from 4°C to 25°C over 60 minutes
(c) subnormothermic perfusion at 25°C for 8 h
(d) controlled, linear cooling of the body from 25°C to 4°C for 60 minutes
(e) reperfusion in the Langendorff model at 37°C and MAP 90 - 100 mmHg using Krebs-Henseleit solution enriched with albumin to simulate reperfusion in the recipient's body.

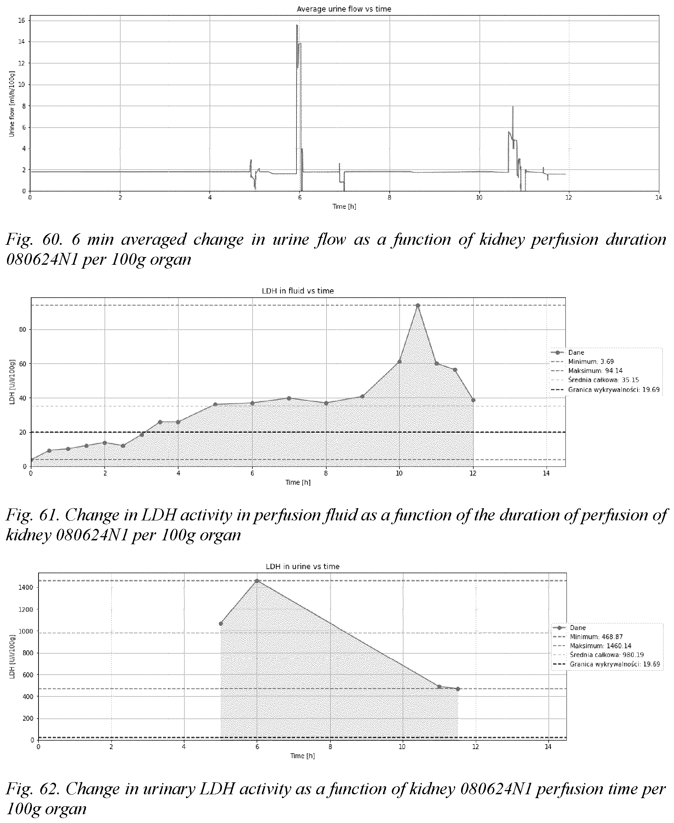

The production of urine by the kidney (See Fig. 60.) started around the 5th hour of perfusion. The urine flow curve is irregular, this is due to the sensitivity of the flow meter to any disturbance.

The LDH activity in the perfusion fluid (See Fig. 61.) increases during the process. In the first three hours, it maintains almost constantly below the limit of detection. At 3.5 hour, after finishing the warming of the perfusion fluid, the enzyme activity increases significantly due to the higher rate of metabolic processes compared to their rate during hypothermia. A continuous increase is observed until the 11th hour of perfusion. In the last two hours of the process, activity of the parameter decreases. The decrease in activity may be caused by cooling of the perfusion fluid in the last hour of the process.

The activity of LDH in the urine during perfusion (see Fig. 62.) is very high at the beginning, reaching values around 1,400 U/l. Thereafter, values of the parameter decreases rapidly, reaching around 500 U/l at the end of perfusion. The decrease in activity may be caused by the cooling perfusion fluid in the last hour of the process.

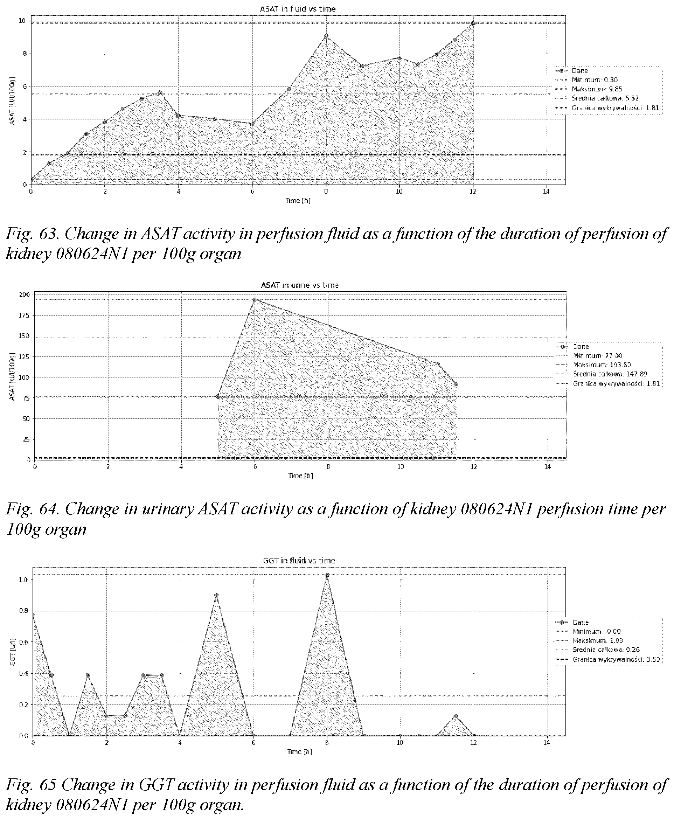

The ASAT activity in the perfusion fluid (see Fig. 63.) gradually increases during the first three hours of the process. Then, from the 4th hour onwards, a slight decrease in the activity of the parameter is observed. From the 6th hour of the process, the activity starts to increase again, ending on a value of 10 U/l. This is not an objectively high value.

The urinary ASAT activity (See Fig. 64.) during the perfusion time alternately increases and decreases. Initially, a large increase is noticeable between the 5th and 6th hour of the process, the parameter reach doubled an activity value. Then, in the last hour of the process, a decrease is observed, leading toredue of parameter value to around 100 U/l.

The GGT activity (see Fig. 65.) undergo considerably. Unfortunately, the concentration of the substance during the process was too low, even below the limit of detection.

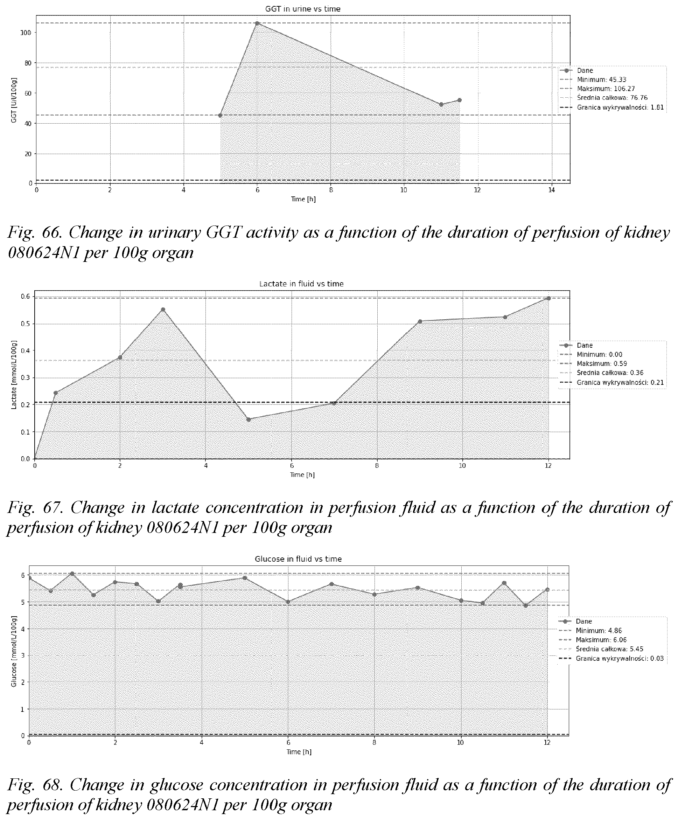

The urinary GGT activity (see Fig. 66.) during the perfusion time changes considerably. Initially, a large increase is noticeable between the 5th and 6th hour of the process, the parameter reach doubled activity value. Then, in the last hour of the process, a decrease is visible leading to a drop in the parameter value to around 50 U/l.

The lactate concentration (see Fig. 67.) maintain low during the process, even below the parameter's limit of detection.

The glucose concentration in the perfusion fluid (see Fig. 68.) oscillates around a constant value during the process, between 5 and 6 mmol/l. This observation may indicate reduced metabolic activity under subnormothermic conditions compared to normothermia.

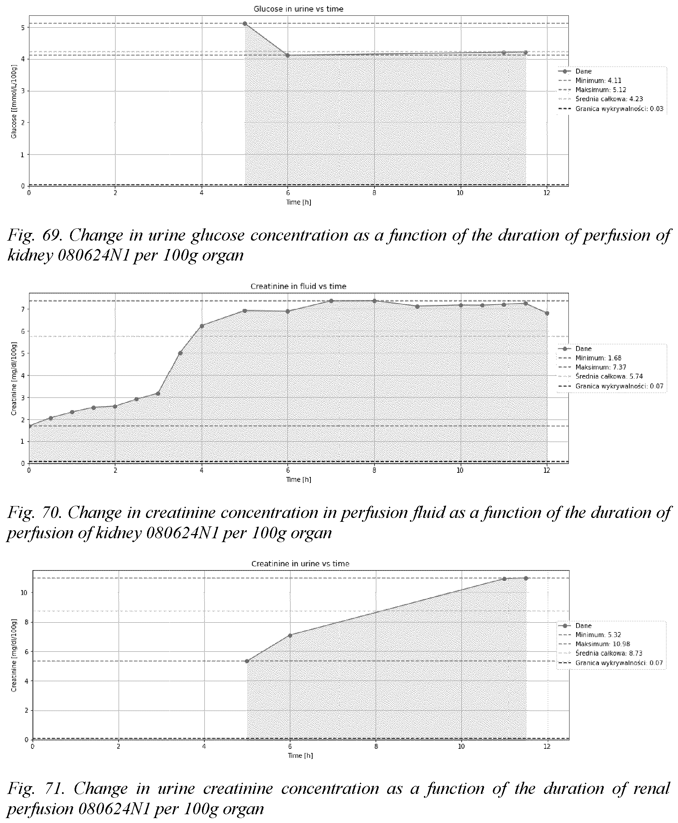

The urine glucose concentration (See Fig. 69.) has a constant value during perfusion. At the beginning of urine production concentration value slightly increases, but this is not a large bias from the rest of the values.

The concentration of creatinine in the perfusion fluid (See Fig. 70.) increases during the perfusion. Up to the 3rd hour of the process, a mild increase in the level of the parameter is observed. While heating, the increase accelerates due to the accelerating metabolic processes in the organ. From the 5th hour of the experiment the creatinine concentration oscillates around a value of 7 mg/dl, and this state is maintained until the end of the process.

The concentration of creatinine excreted in the urine (See Fig. 71.) increases during perfusion. This indicates good organ function as it removes excess creatinine from the perfusion fluid.

In summary, a porcine kidney subjected to significant ischaemic injury was successfully perfused. The kidney was exposed to ischaemia at a physiological temperature (37°C). Based on the aforementioned conditions, the damage to the kidney prior to perfusion can be considered severe. The data obtained from the perfusion process allow the overall condition of the organ after perfusion to be determined as fit for transplantation. The kidney started functioning during the reperfusion process by producing urine and filtering creatinine.

**Example III.1.3.7. Hypothermic perfusion of isolated kidney 030222N2** - **DCD model WIT: 7 min, CIT: 54 h 20 min**

Hypothermic perfusion is carried out in a model where the temperature is maintained at 5°C throughout the process.

Kidney from a female weighing approximately 40 kg. Organ harvesting followed a 7-min warm ischaemia, the kidney was stored 54 h 20 min under cold conditions: on ice or in a refrigerator. Perfusion was performed with MPS Belzer fluid. The perfusion plan under hypothermia included:

(a) hypothermic perfusion at 5°C for 24 hours
(b) reperfusion in the Langendorff model at 37°C and MAP 90 - 100 mmHg using Krebs-Henseleit solution enriched with albumin to simulate reperfusion in the recipient's body

Urine flow in hypothermic perfusion is negligible, metabolic processes are very slow. Biochemical measurements are carried out only for perfusion fluid.

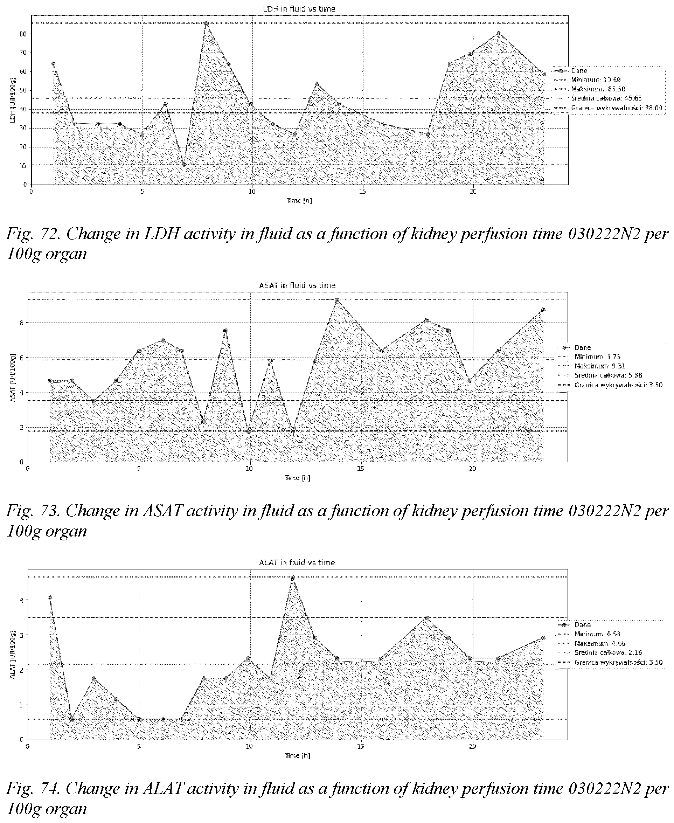

The LDH activity (see Fig. 72.) changes significantly, with the enzyme activity alternately rising and falling below the limit of detection. The parameter reaches its maximum value at the 8th hour of the process. It's worth noticing that hypothermic perfusions metabolic processes are slowed down, so enzyme activities will be lower than in perfusions at higher temperatures. Performed tests may have lower accuracy due to low concentration of biochemical marker.

The ASAT activity in the perfusion fluid (See Fig. 73.) maintains low during the process, slightly above or below the limit of detection. In the last hours of the process, stabilization of values can be observed.

The ALAT activity in the perfusion fluid (see Fig. 74.) during perfusion maintains practically below the limit of detection. This means that the results are not meaningful the parameter can't be used to draw conclusions when assessing the organ's general condition.

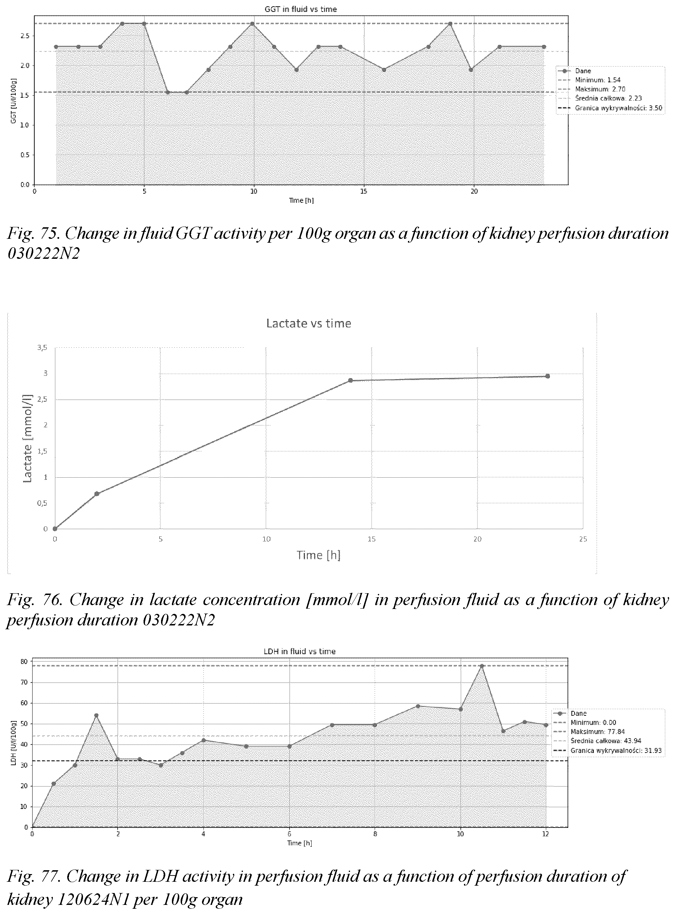

The GGT activity in the perfusion fluid (See Fig. 75.) during perfusion maintains practically below the limit of detection. This means that the results aren't meaningful and the parameter can't be used to draw conclusions when assessing the organ's general condition.

The lactate concentration (see Fig. 76.) increases during the perfusion time. Stabilization of values can be observed around the 13th hour of the process. The values of the parameter remain low.

Due to reduced metabolism, the kidney didn't produce urine.

**Example III.1.3.8. Hypothermic perfusion of kidney 120624N1** - **DCD model WIT: 30 min, CIT: 26 min**

Hypothermic perfusion is carried out in a model where the temperature is maintained at 4°C throughout the process.

Kidney from a female weighing approximately 51.6 kg. The organ was harvested after 30 min of warm ischaemia, the kidney was stored 26 min under cold conditions: on ice or in a refrigerator. The weight of the kidney before perfusion 119g and after perfusion 149g was assessed. Perfusion was performed with MPS Belzer fluid. The perfusion plan under hypothermia included:

(a) hypothermic perfusion at 4°C for 12 h
(b) implanting an organ back into the animal's body

Urine flow in hypothermic perfusion is negligible, metabolic processes are very slow. Biochemical measurements are carried out only for the perfusion fluid.

The LDH activity in the perfusion fluid (See Fig. 77.) increases during the process. In the first hour, it remains below the limit of detection. Continuous increase is observed until the 11th hour of perfusion. In the last hour of the process, the level of the parameter decreases.

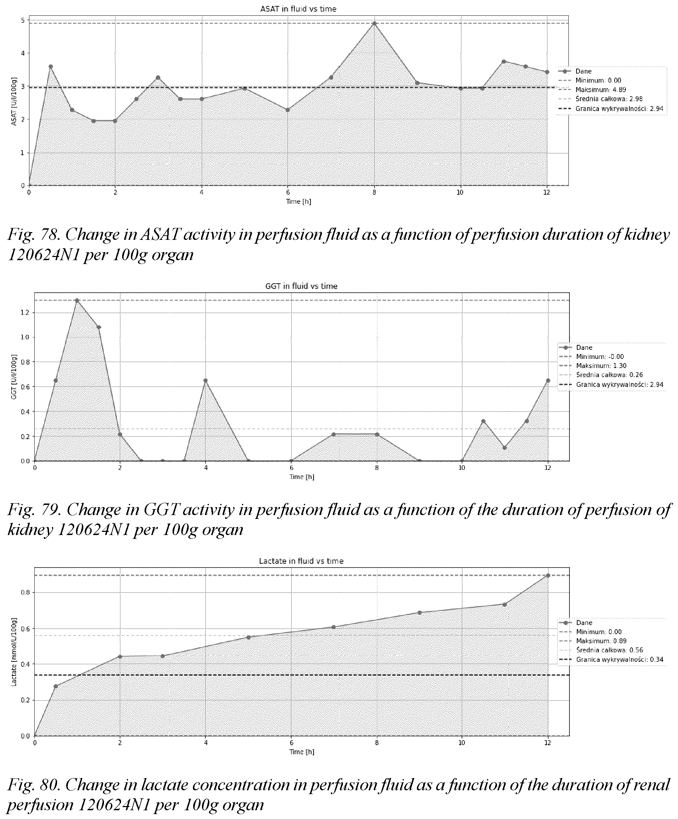

The ASAT activity in the perfusion fluid (See Fig. 78.) maintains low during the process, slightly above or below the limit of detection. In the last hour of the process, smaller fluctuations in values can be observed.

The GGT activity in the perfusion fluid (see Fig. 79.) maintains below the limit of detection during perfusion. This means that the results aren't meaningful and the parameter can't be used to draw conclusions when assessing the organ's general condition.

The lactate concentration (see Fig. 80.) increases steadily during perfusion. The value of the parameter is maintained at a low level.

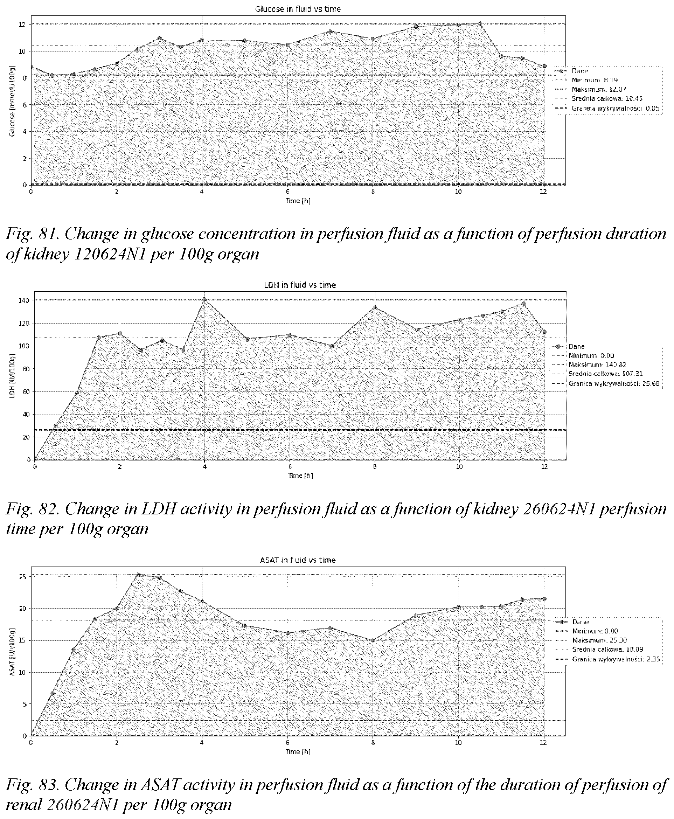

The glucose concentration in the perfusion fluid (see Fig. 81) initially decrease at the beginning of the process. Increase in the parameter value is observed after 1 hour of the perfusion. The value stabilises after the 3rd hour of the process between 10 and 12 mmol/l. In the last hour of the process, the concentration decreases.

Due to reduced metabolism, the kidney did not produce urine.

**Example HI. 1.3.9. Hypothermic perfusion of isolated kidney 260624N1** - **WIT model: 30 min, CIT: 29** min

Hypothermic perfusion is carried out in a model where the temperature is maintained at 4°C throughout the process.

Kidney from a female weighing approximately 50.5 kg. The organ was harvested after 30 min of warm ischaemia, the kidney was stored 29 min under cold conditions: on ice or in a refrigerator. The weight of the kidney before perfusion 155g and after perfusion 176g was assessed. Perfusion was performed with MPS Belzer fluid. The perfusion plan under hypothermia included:

(a) hypothermic perfusion at 4°C for 12 h
(b) implanting an organ back into the animal's body

LDH activity (see Fig. 82.) increases intensively during the first 1.5 hours of the process. Then a further increase is observed, less intense than at the start. Between 4th and 8th hour of the process, peaks of activity reaching values of 140 U/l can be observed.

ASAT activity (see Fig. 83.) increases intensively during the first 2.5 hours of the process. Thereafter, the level of the enzyme begins to decrease reaching a value of 15 U/l. In the last four hours of the process, the ASAT activity increases.

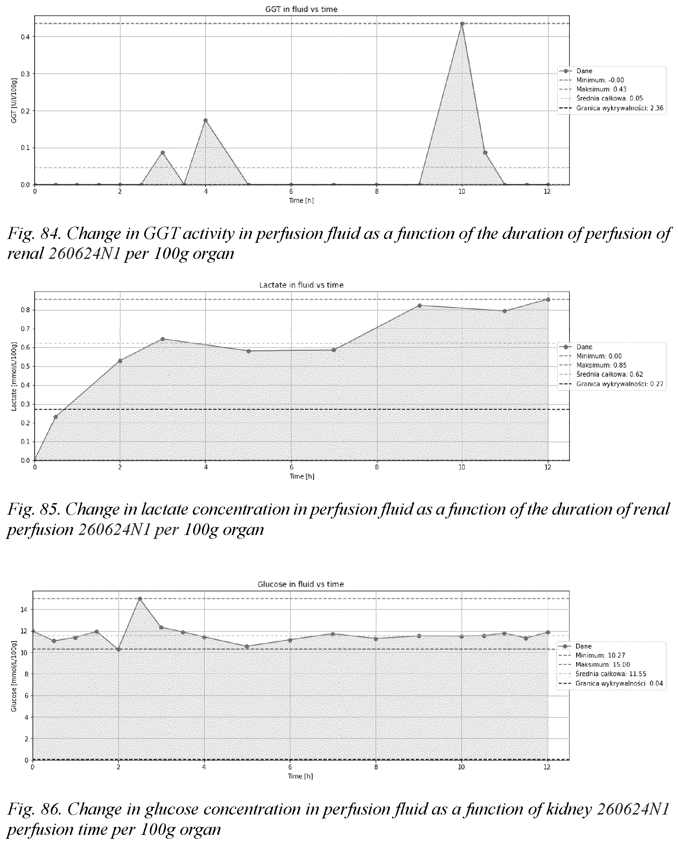

The GGT activity in the perfusion fluid (See Fig. 84.) remains below the limit of detection during perfusion. This means that the results aren't meaningful and the parameter can't be used to draw conclusions when assessing the organ's general condition

The lactate concentration (See Fig. 85.) increases during the perfusion time. Values stabilization can be observed between 4th and 7th hour of the process. The value of the parameter maintains low.

The glucose concentration in the perfusion fluid (see Fig. 86.) oscillates around a constant value between 10 and 12 mmol/l during the process. At 2.5 hour of the process, a maximum value of 15 mmol/l can be observed.

Due to reduced metabolism, the kidney did not produce urine.

## Example III.1.4. Proteomic-metabolomic evaluation of the kidney after ex vivo perfusion

Proteomics and metabolomics are key tools in systems biology, which focuses on the analysis of complex molecular interactions in specific biological systems. Proteomics involves understanding all proteins in a given sample, while metabolomics identifies all chemical compounds. In proteomic analysis, proteins can be separated, identified, quantified and studied in terms of their amino acid sequences, three-dimensional structures and interactions. Metabolomic analysis generates both quantitative and qualitative profiles of detected compounds, which can then be analysed in databases and compared with proteomic results.

Perfusion fluid_02_II production method and perfusion fluid_04_II production method, investigated in the experiment, aim to improve the process of organ perfusion for transplantation. Ensuring that the fluid works properly requires an accurate assessment of organ viability. Systems biology methods, including proteomics and metabolomics, have been chosen for their high sensitivity and minimal requirements for biological material, allowing comprehensive analysis of proteins and metabolites. These methods reveal known or novel molecules indicative of organ status.

Analysis of these molecules using specific databases allows their assignment to metabolic pathways, cellular localisations and signalling pathways such as apoptosis, regeneration or vascular damage. Identified proteins are semi-quantitatively described by their 'peak' intensity, providing both type and level information. These values can be ordered for comparison with control samples, tracking temporal changes and observing the effects of disease or medication. Selective analysis of proteins involved in specific metabolic processes or those unexpectedly appearing in samples is also possible. Advanced statistical methods, such as multivariate ANOVA and data mining, offer deeper insights and reveal hidden relationships.

## Example III.1.4.1 Methods:

The study included extracorporeal renal perfusion in the IKP model (minimum warm ischaemic time, i.e. less than 10 minutes) of the following cases:

- Case A: Perfusion fluid: perfusion fluid_02_II production method under subnormothermic conditions
- Case B: Perfusion fluid: Perfusion fluid_02_II production method under normothermic conditions
- Case C: Perfusion fluid: perfusion fluid_04_II production method under normothermic conditions
- Case D: Perfusion fluid: MPS Belzer fluid under hypothermic conditions

The hypothermic perfusion method used included perfusion with commercially used MPS Belzer fluid at a mean temperature of 6 deg C. The duration of perfusion was 24 hours. During perfusion, fluid samples were collected for proteomic and metabolomic studies at the following time points (calculated from the start of perfusion, in hours): 0.5h; 2h, 24h. After perfusion, reperfusion was performed with albumin-enriched Krebs-Henseleit buffer at 37 deg C and a mean arterial pressure of 90 - 100 mmHg .

The subnormothermic perfusion method used included perfusion using the study perfusion fluid_02_II production method comprising the following scheme:

- initial perfusion under hypothermia (4 - 12°C for 4h

- controlled, linear temperature increase to 25°C for 60 minutes

- subnormothermic perfusion at 25°C for 24 hours

- controlled, linear temperature gradient down to 5°C for 60 minutes

After perfusion, reperfusion was performed with albumin-enriched Krebs-Heneseleit buffer at 37 deg C and a mean arterial pressure of 90 - 100 mmHg

During perfusion, the fluid was oxygenated with a stream of 2.5 L/min of 100% oxygen entering the oxygenator.

During perfusion, fluid samples were collected for proteomic and metabolomic studies at the following time points (calculated from the start of perfusion, in hours): 1h; 3h; 4h; 4.5h; 9h; 29h; 30h.

The normothermic perfusion method used included perfusion with the perfusion fluid_04_ II production method under study comprising the following scheme:

- initial perfusion under hypothermia (4 - 12°C) for 3h

- controlled, linear temperature rise to 37°C for 90 minutes

- subnormothermic perfusion at 37°C for 24 hours

- controlled, linear temperature gradient down to 5°C for 90 minutes

After perfusion, reperfusion was performed with albumin-enriched Krebs-Heneseleit buffer at 37 °C and a mean arterial pressure of 90 - 100 mmHg

During perfusion, the fluid was oxygenated with a stream of 2.5 L/min of 100% oxygen entering the oxygenator.

During perfusion, fluid samples were collected for proteomic and metabolomic studies at the following time points (calculated from the start of perfusion, in hours): 0.5h; 2.5h; 4h; 5h; 9h; 28.5h; 30h.

A mass spectrometry system consisting of two mass spectrometers coupled to a nano-UHPLC (nanoElute - Bruker) was used for the analyses. The mass spectrometers are SolariX 2xR (FT-ICR) with ESI and MALDI ionisation sources and Compact (quadrupole with time-of-flight analyser) with ESI ionisation.

The first task was to standardise samples from different kidneys into specific groups based on the time of collection during perfusion. This standardisation allowed the assignment of periods to groups: Before perfusion (Control - sample taken before connecting the organ), shortly after the start **(Start** - sample taken up to 1h after the start of perfusion), during hypothermia **(Cold** - sample taken between 1h and 3h of running the process under hypothermic conditions), at the start of heating **(Heat A** - sample taken during the initial moments of linear fluid heating, up to 0.5h of running the process), during normothermia/subnormothermia **(Heat B, C** or **D,** respectively: after reaching the set temperature of subnormothermia, or normothermia; during subnormothermia, or normothermia; at the start of the cooling of the perfusion fluid) and after the final cooling before reperfusion **(End** - sample taken at the **end** of the perfusion process).

Results of proteomic and metabolomic analyses were sorted by group and kidney origin, allowing identification of trends, patterns and comparisons. Protein or metabolite levels were averaged for all kidneys and percentages were averaged for all time points. Student's t-test applied to each detected protein and metabolite determined statistical significance. Significant associations were analysed in bioinformatic databases (UniProt, KEGG, MetaboAnalyst).

Biomarkers were selected for processes such as apoptosis, matrix and vascular damage, tissue regeneration, ischaemia and inflammation, with 5-10 markers for each process. Renal-specific markers of glomerular function, tubular damage, endothelial dysfunction and acute or chronic renal injury were also selected. The selection criteria included the most commonly used markers, best illustrating each process, detected in our analysis and present in the majority of samples and kidneys.

### Example III.1.4.2. Results and discussion:

Apoptosis during perfusion: The study examined markers of apoptosis in different kidneys, revealing significant differences. High levels of cytochrome C, catalase and apoptosis-stimulating protein p53 2 (ASp53 p2) were found. The kidney from Case A showed high levels of cytochrome C, AVEN and ASp53 p2. Kidneys from Cases B and C had elevated levels of catalase and ASp53 p1, but zero cytochrome C. The kidney from Case D had high levels of cytochrome C, catalase and ASp53 p1.

Markers of apoptosis differed between time points. In Case A, high markers appeared at baseline (cytochrome C and TNFR), heat B (AVEN, TNFR, GAPDH), heat C (ASp53 p1, TNFR, HIF3 alpha) and heat D (TNFR, catalase, ASp53 p2, GAPDH). Case B showed a significant increase in heat A (GPADH and apoptosis inducing factor 1), heat B (ASp53 p1 and catalase) and heat C (catalase, ASp53 p1). Case C markers peaked in control (ASp53 p1, catalase), early (ASp53 p1, cytochrome oxidase, catalase, AVEN), in Heat A (ASp53 p1, GAPDH, apoptotic protease activating factor 1, cytochrome oxidase), in Heat B (catalase, PARP14) and at the end of the process (ASp53 p1 and 2, GAPDH, catalase).

Regeneration processes: High regeneration markers (oestrogen receptor, AKT1) were found in Case A, while CD28, CD3, oestrogen receptor beta and GLI2 were elevated in Case C. Case B and D had lower levels of these compounds. Case A had high levels of oestrogen receptor at baseline, CD3 in heat A, TGF beta1 in heat B and again CD3 in heat C.

Vascular status: Cases A and B had high levels of ICAM1, von Willebrand factor, osteopontin and stabilin 1, respectively. Case D had high levels of VCAM1, while Case C had the lowest levels of this marker.

Matrix damage: Case A showed high levels of matrix damage markers (ADAM1, collagenase 3, fibrinogen, interstitial collagenase, MMP9). The other cases maintained elevated levels of fibrinogen but lower levels of other markers.

<u>Oxidative stress</u>: Case B showed the highest enzymatic markers of oxidative stress, although the other cases also had high levels. Case A showed similar markers and myeloperoxidase, lactoperoxidase, catalase. Case B showed high levels of ceruloplasmin and catalase. Case D had the lowest levels of glutathione peroxidase 6, catalase and ceruloplasmin.

<u>Renal-specific markers</u>: Cases B, Ci D had elevated levels of alpha 2 macroglobulin. The highest peaks of ischaemia markers were in Case B (HIF1 alpha, HIF3 alpha). Inflammatory markers were highest in Case A (TNF, interleukin 6).

<u>Metabolite-protein interactions</u>: metabolomic analysis revealed key metabolic pathways active at different time points, such as sphingolipid transformation, steroid synthesis, amino acid metabolism and glutathione metabolism. Network analysis revealed interactions between proteins and metabolites, indicating significant changes in metabolic activity during perfusion and reperfusion.

**Example III.1.4.3. Markers to assess renal perfusion processes**

<u>Markers of apoptosis:</u>

The most reliable markers of apoptosis are GAPDH, catalase and the apoptosis stimulator p53, consistently appearing in all kidneys and most time points. These markers, which are associated with oxidative stress and tissue damage, were most elevated during Warming A, B and C periods, indicating a correlation between apoptosis and organ warming.

<u>Markers of vascular health:</u>

VEGF A and B, together with von Willebrand factor, are crucial for monitoring blood vessel health. VEGF A promotes the growth of new blood vessels in low-oxygen conditions, while VEGF B maintains newly formed vessels. Their presence in perfusion fluid suggests hypoxia or vascular damage, with the highest levels observed at Control, Onset and Heat C.

<u>Damage and rebuilding of the matrix:</u>

ADAM metalloproteinases are key indicators of matrix damage and remodelling. These markers, which facilitate cell adhesion and the action of cytokines and growth factors, were most elevated during periods: Control, Heat B, C and D, highlighting damage at the tissue level and attempts at repair.

<u>Regeneration markers:</u>

T cell surface glycoproteins CD3, 8 and 28 are potential markers of regeneration, reflecting the renewal of T cell populations in the vicinity of damaged tissue. The highest levels were detected at Heat A, suggesting repair efforts after surgery, hypoxia and hypothermia.

<u>Small-molecule markers:</u>

Urobilin indicates normal renal filtration, while tiglycarnitine and glutamic acid levels signal renal damage and metabolic efficiency, respectively. High hypoxanthine levels in all kidneys are due to the presence of allopurinol, which inhibits xanthine oxidase, the enzyme that converts hypoxanthine to uric acid.

<u>Markers of oxidative stress:</u>

Catalase, glutathione peroxidases and glutathione are effective markers of oxidative stress. Catalase levels increase with perfusion time, while glutathione peroxidases change inversely to catalase. Changes in glutathione levels indicate tissue antioxidant requirements and levels of oxidative stress.

<u>Markers of hypoxia:</u>

Hypoxia-inducible factors 1 and 3 (HIF 1 alpha and HIF3 alpha) are reliable markers of hypoxia. HIF 1 alpha levels peak at baseline and endpoints, correlating with changes in oxygen uptake. HIF3 alpha, a negative regulator of the adaptive response to hypoxia, shows elevated levels in Case B

**Example III.1.4.5 Specific results for individual kidneys:**

Each Case showed unique marker profiles at different time points, highlighting differences in apoptosis, regeneration, vascular status, matrix damage, oxidative stress and kidney-specific markers. Case C: Best overall condition:
Case C showed the best overall condition, with minimal markers of apoptosis, high markers of regeneration, low markers of vascular and matrix damage and stable levels of oxidative stress and ischaemia. Levels of urobilin and reduced glutathione were consistently high, indicating normal renal function, which was further supported by stable urine production during perfusion.

Descriptions of kidneys based on perfusion analysis

**The case of A:**

- Perfusion protocol: subnormothermia, perfusion fluid_02_II production method

- Key findings/conclusions: High levels of apoptosis markers (cytochrome C, GAPDH, p53) with additional high inflammatory markers (TNF, interleukin 6). The lower temperature may have slowed down metabolism, reducing the impact of oxidative stress. No urobilin was detected, indicating renal damage.

**The case of B:**

- Perfusion protocol: normothermia, perfusion fluid_02_II production method

- Key results/conclusions: High markers of oxidative stress. Initially elevated levels of antioxidant enzymes, but reduced glutathione levels indicate depletion due to oxidative stress. High levels of long-term damage markers.

**Case C:**

- Perfusion protocol: normothermia, perfusion fluid_04_II production method.

- Key results/conclusions: Best overall condition with minimal markers of apoptosis and high markers of regeneration. Low levels of markers of vascular and matrix damage. Stable levels of oxidative stress and ischaemia markers. High levels of urobilin indicate normal renal function. Steady urine production during perfusion.

**The case of D:**

- Perfusion protocol: hypothermia, MPS Belzer fluid

- Key findings/conclusions: Low markers of apoptosis during perfusion, high markers of vascular and glomerular function. Elevated markers of nephropathy and ischaemia. During reperfusion, significant increase in markers of apoptosis and inflammation, probably due to reperfusion injury and oxygen return, leading to oxidative stress and cell damage.

**Example III.2: Perfusion of porcine liver ex vivo**

In an ex vivo porcine liver perfusion study, the liver was harvested from a 40 kg female pig and the liver perfusion process was performed. The study was performed using an organ perfusion device, the subject of patent application no. P.449924. During the process, fluid was sampled regularly for the significance of key markers: general cellular damage (LDH - lactate dehydrogenase) and liver enzymes (AST - aspartate aminotransferase, GGT -gamma glutamyltranspeptidase, ALT - alanine aminotransferase. In the case of fluid and bile, lactate concentration was additionally measured.

Physicochemical parameters of the fluid such as pH and oxygenation were measured with FTC sensors (online).

Drains, sensors and other disposable components were connected to the perfusion device. After that, device was filled with 1000 ml of perfusion fluid_02_II production method. During the perfusion another 1000 ml of perfusion fluid_02_II production method was added. The device was turned on to calibrate the sensors and check the system for leaks. Once the proper functioning of the system was verified, cooling of the fluid inside the device was started. Once hypothermic conditions were reached (10°C), the flow rate was reduced to 100 ml/min. Under these conditions, the system is ready for organ connection. The perfusion plan under normothermic conditions included:

- initial hypothermic perfusion for 1h

- controlled, linear temperature increase from 10°C to 37°C over 1 hour

- normothermic perfusion at 37°C for 2.5 h

During each stage samples of perfusion fluid and bile were collected for biochemical parameters determining the organ's condition, samples for proteomic and metabolomic studies and a biopsy.

Before connecting to the ex vivo organ perfusion device on the side table, the liver was cannulated and washed of blood with UW fluid (3 L). The condition of the liver was macroscopically assessed as correct. Organ weight before perfusion: 820g. Weight of organ after perfusion: 1272g (weight increase of 55%). The organ was then connected to the machine and perfusion of the organ was carried out for 4.5 hours. Perfusion was conducted through two inlet vessels (portal vein and hepatic artery). During the process, glucose, verapamil and insulin were continuously administered. Calcium bicarbonate 8.4% was also administered to equilibrate the pH.

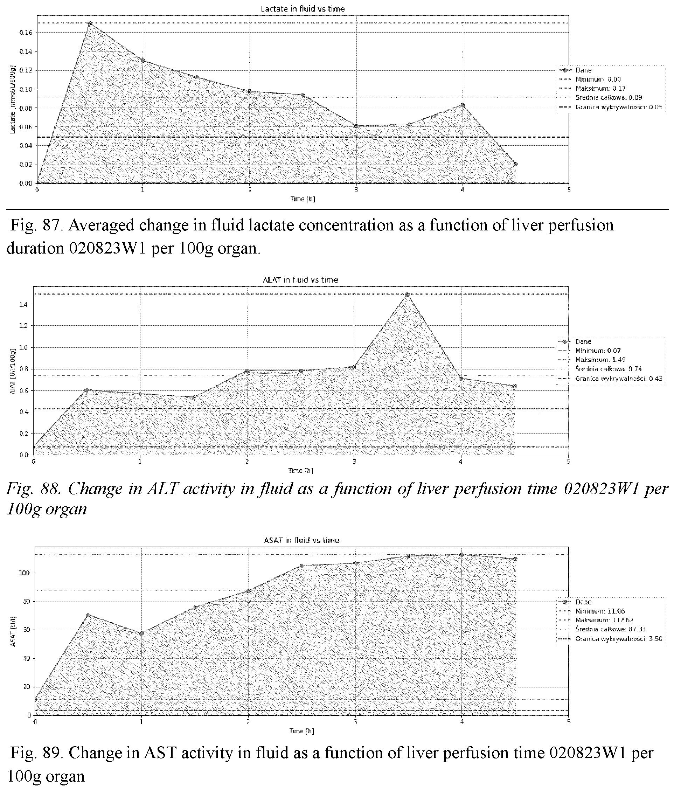

Biochemical analysis shows that lactate levels (see Fig.87.) successively decreased during the process, indicating adequate oxygenation of the entire organ during perfusion. This is also confirmed by the fact of increased flow through the blood vessels during the normothermic period and, consequently, a higher oxygen uptake by the liver.

The change of ALT activity (See Fig.88.) during the process is small. It increases slightly in the duration of the process. The large peak in ALT level at 3.5 hour of perfusion can be considered as an error of the enzyme test. Enzyme level returns to a much lower level in the next measurement.

AST activity (See Fig.89.) increase during perfusion. A more intense increase is observed during the switch from hypothermia to normothermia, which is a typical phenomenon due to accelerating metabolic processes. During the last two hours of perfusion, a plateau in AST levels is observed.

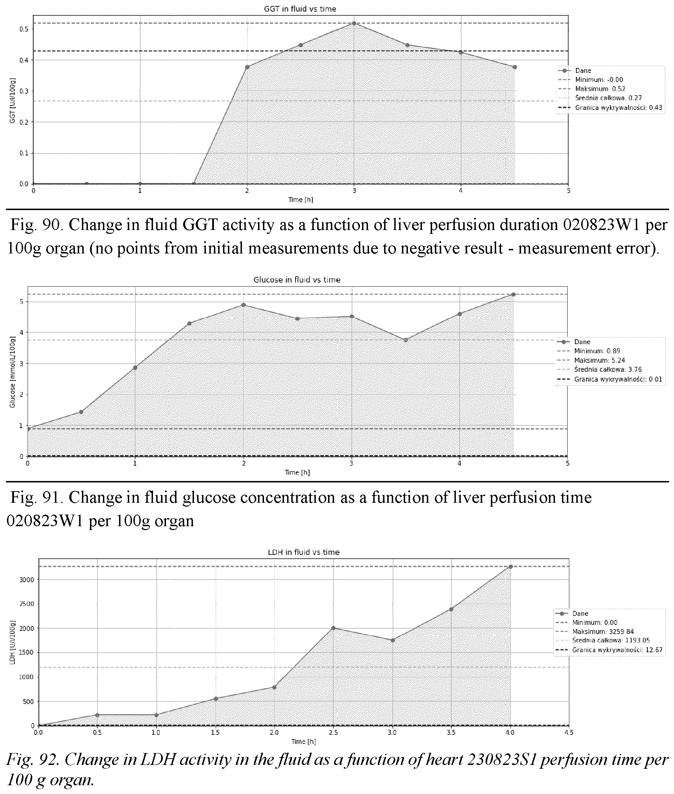

The course of the GGT activity curve (See Fig.90.) varies. For the first hours of perfusion, it was not possible to detect GGT in the perfusion fluid. Concentration of the substance was so low that the enzyme test didn't work properly. Subsequently, the enzyme concentration increased, above the limit of detection. It maintained at a slightly higher level.

Glucose (See Fig.91.) during liver perfusion was continuously dosed during the normothermic period, due to the high consumption of this substance by the liver. The graph shows that after a period of hypothermia and warming of the perfusion fluid (the first two hours of perfusion), where the concentration of the component was increasing, the glucose level starts to decrease. This situation indicates that the organ is carrying out its metabolic processes correctly due to adequate glucose uptake.

In summary, the study involved perfusion of an 820g pig liver harvested in the Donation after Brain Dead (DBD) model. In this model, the blood is washed out of the animal's vascular bed before the organ is physically harvested, which keeps warm ischaemia time to a minimum. After flushing, the organ was transferred to a special package and kept in a hypothermic environment. After 8.5h of cold ischaemia time, organ perfusion was initiated as described in section 3.2. Perfusion proceeded as intended. The organ perfusion device allowed perfusion of both inlet vessels, namely the portal vein and the hepatic artery. Throughout the process, flow in both vessels was stable and increased as intended. After half an hour of normothermic perfusion, bile was observed in the cannula tied to the bile duct. During the process, the liver produced approximately 3.5 ml of bile. However, this result could have been much higher, as the system was not prepared for bile transport. After perfusion, the gallbladder was opened and contained additional 15-20 ml of bile.

During the process, liver enzymes activities are within acceptable ranges. A decreasing concentration of lactate during the process was also observed.

**Histopathological evaluation of the liver:**

A total of 8 specimens were evaluated: 3 thick-needle biopsies (BG) and 5 liver sections (HP) were taken. The sections were toyed with haematoxylin and eosin Microscopic evaluation indicated that all liver preparations evaluated had preserved bead structure and architecture. The structures of the portal spaces (veins, arteries, bile ducts) and central veins were preserved and normal. The assessment of the lesion was modelled on the scheme used in liver transplantation for the assessment of reperfusion/ischaemic damage (DOI: 10.1002/lt.2407). This scheme assesses:

- the presence of inflammatory infiltrates from neutrophils
- occurrence of apoptotic bodies
- occurrence of hepatocyte necrosis

Based on the above parameters, the so-called degree of injury is assessed: IRI=ischemia/reperfusion injury

0=no signs of damage, 1=moderate severity, 2=medium severity and
3=severe severity of damage

In addition, steatosis was assessed: fine- and large-droplet

A % score (used in the assessment of NASH-non-alcoholic steatohepatitis. NASH CRN histology scoring system) pinnacle degeneration of hepatocytes (hydatid degeneration)- analogous to steatosis, a percentage breakdown was used

other: in two liver cases, acidophilic aggregations in the cytoplasm reminiscent of metamitochondria or Mallory bodies were clearly visible

The gallbladder wall was autolytically altered. The mucosa of the gallbladder was unreadable due to these changes.

The IRI, used to assess reperfusion/non-ischaemic damage to the transplanted liver, correlates with the risk of developing graft complications.

Severe damage severity was shown to occur in patients with PNF (primary non function) or EGD (early graft dysfunction), which was associated with a higher risk of retransplantation within 90 days, compared to the medium and low severity groups.

The liver material assessed showed that 1 case showed severe damage (IRI 3), the others: 1 case showed no change, 2 cases low damage (IRI 1), 4 cases medium damage (IRI 2). Given the results, the vast majority of liver biopsies do not show signs of high severity of damage, which may have an impact on limiting graft complications, and reducing the risk of retransplantation.

## Example III.3: Perfusion of porcine heart ex vivo

The study involved a heart harvest procedure from a female pig, followed by a heart perfusion process. The study was performed using an organ perfusion device that is the subject of patent application No. P.449924.

Drains, sensors and other disposable components were connected to the perfusion device. After that, device was filled with 1250 ml of perfusion fluid_04_II production method. The device was started up to calibrate the sensors and check the system for leaks. Once the proper functioning of the system had been verified, heating of the fluid inside the device proceeded. Once normothermic conditions were reached (38°C), the flow rate was reduced to 400 ml/min. Under these conditions, the system is ready for organ connection. The experiment involved perfusion at 37-39°C and a MAP of 100 - 120 mmHg of perfusion fluid_04_II production method for 4 hours. During perfusion, samples of the preparation were taken to examine biochemical parameters that determine the organ's condition.

A pig heart collection from a female was performed. **WIT: 0 s CIT: 1 h.** On the side table, the heart was cannulated and washed of blood with UW fluid (1 L). The organ was then connected to the machine and perfusion was carried out for 4 h.

LDH activity (See Fig. 92.) increases during perfusion. For the first two hours of the process, activity increases gradually. During following hours activity level incerases more rapidly. Enzyme activity increase is caused by overall organ condition. Macroscopic organ evaluation shows that heart is upswelling intensely after 2nd hour of the perfusion. LDH activity reaches high level which confirms decreasing efficiency of organ functioning.

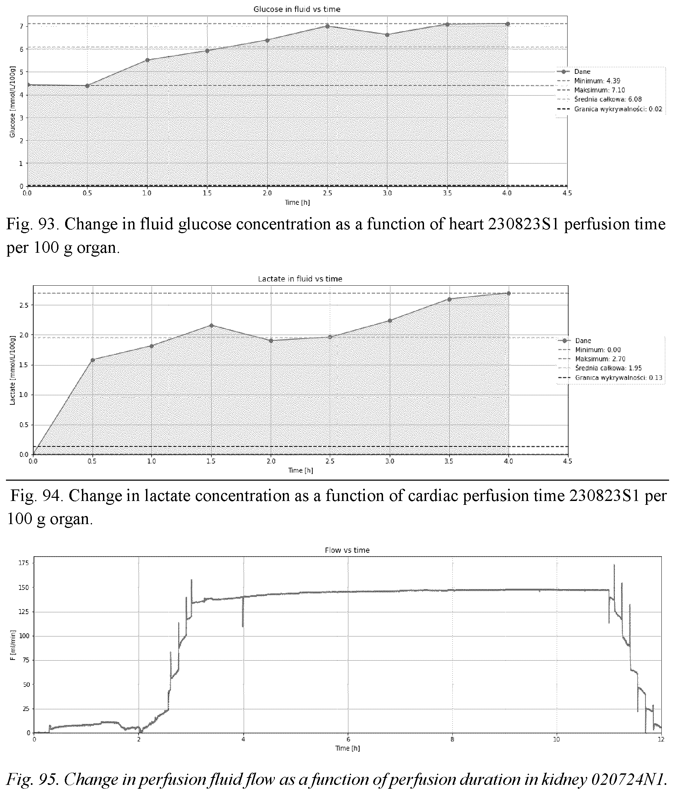

The glucose concentration (See Fig.93.) was decreasing during the process. Perfusion was carried out at a constant temperature of approximately 38°C.

Lactate concentration (See Fig. 94.) increases during the perfusion.

The study involved perfusion of an isolated pig heart taken in a DBD model. Prior to perfusion, the heart was washed with PlegiStore cardioplegic solution. Perfusion was performed under normothermic conditions with perfusion fluid_04_II production method. The process was carried out in a prototype perfusion device equipped with a set dedicated to heart perfusion. Such a set allows monitoring of cardiac parameters and control of cardiac function. ECG measurement was performed using innovative cardiac electrodes from Heart Sense on an integrated OEM board from MedLab. Pacing was done via the Osypka PACE 101 H. After 20 seconds of perfusion, the heart started to work. After 20 minutes, defibrillation was performed. After defibrillation, the heart regained a normal intrinsic rhythm. During the first two hours of perfusion, the heart macroscopically appeared normal. Perfusion parameters (flow and resistance) also indicated systematic reconditioning of the organ under ex vivo perfusion conditions during the first hours of perfusion. Throughout the perfusion process, oxygen consumption remained at a constant, satisfactorily high level.

**Example IV. Perfusion of porcine kidney ex vivo with subsequent autotransplantation**

As part of the experiment, a study was performed on an animal - the Polish White Hog. The aim of the experiment was to determine the effectiveness of kidney perfusion using perfusion fluid_02_II production method after induction of significant ischaemic damage - DCD WIT 30 damage model (collection after cardiac arrest and 30 minutes of warm ischaemia time). The tests were performed using an organ perfusion device that is the subject of patent application no. P.449924. A kidney (left) was taken from the animal, which was stored under controlled conditions for 12h and then transplanted into the same animal with subsequent removal of the other kidney (right). The uptake of normal function by the transplanted kidney was assessed by a 14-day follow-up with daily assessment of the animals' general health and blood and urine analyses (assessment of renal parameters). After euthanasia, a detailed histopathological evaluation was performed to determine the status of the organ 14 days after transplantation.

The experiment consisted of **five main stages:**

1. Organ retrieval after induction of warm ischaemia

2. Organ presentation by machine perfusion under subnormothermic conditions

3. Transplantation of an organ into an animal after preservation

4. Observation of the animal for a period of 14 days

5. Collection of kidney for histopathological examination with subsequent killing of the animal

**In stage 1,** after anaesthetising the animal and preparing the surgical field, the procedure was started. After tracing the arterial and venous vessels of the left kidney, intravenous heparin (approximately 100 IU/kg body weight, 5,000 IU total) was administered, followed by the process of stopping circulation in the kidney by splicing and ligating these vessels. From the moment the circulation was stopped in the vessels, the warm ischaemia time (WIT) was started to be measured, which was a scheduled 30 minutes. The warm ischaemia time was terminated when blood was started to be washed out of the vascular bed with cold Belzer UW fluid enriched with added heparin (5000 IU/L). The organ was cannulated (arterial vessel, venous vessel and ureter) and flushed with the previously prepared 500 ml of heparin-enriched Belzer UW fluid. The organ was then lavaged with 200 ml of cold Ringer's fluid. While the blood was being washed out of the placenta, the patency of the vessels and the tightness of the cannula were checked, and the organ thus dissected was then transferred to the Organ Bag of the previously prepared organ perfusion device filled with perfusion fluid_02_II production method. The abdominal shells were then closed and a dressing applied, thus completing the procedure. After the procedure, the animal was transported to an individual cage where the recovery process took place.

**Step 2** Preparing the organ perfusion device. Drains, sensors and other disposable components were connected to the perfusion device. After that, device was filled with 1000 ml of perfusion fluid_02_II production method. The device was turned on to calibrate the sensors and check the system for leaks. Once the proper functioning of the system was verified, the fluid inside the device was started to cool down. Once hypothermic conditions were reached (4-12°C), the flow rate was reduced to 50 ml/min. Under these conditions, the system is ready for organ connection.

The subnormothermic perfusion plan included:

- initial hypothermic perfusion for 2h

- controlled, linear temperature increase to 25°C for 60 minutes

- subnormothermic perfusion at 25°C for 8 h

- controlled, linear cooling of the body from 25°C to 4°C for 60 minutes

After the preparatory steps, the oxygen supply to the oxygenator was switched on, and the renal artery cannula was connected to the inlet port of the Organ Bag (a disposable organ pack containing the inlet and outlet ports of the perfusion fluid) initiating the perfusion process. The perfusion process lasted 12h. The end of the perfusion time is when the arterial cannula is disconnected from the Organ Bag inlet port. After perfusion, the vascular bed of the organ was rinsed with 1000 ml of cold Belzer UW fluid to remove residual perfusion fluid_02_II production method. The organ was subsequently rinsed with 200 ml of cold Ringer's fluid. A biopsy was performed and the organ was transferred to the operating theatre, where the vascular preparation of the perfused kidney began.

**Stage 3** begins the start of the transplant procedure. First, the abdominal shell was opened where it had been closed during the previous procedure. The right kidney and its renal artery, renal vein and ureter were then retrieved. A nephrectomy was performed and vessel emergence proceeded. After vessel emergence, the preparation of the vessels of the perfused kidney proceeded and vascular anastomoses were started. The end of the anastomosis time is considered to be the moment when the arterial and venous clamp opened initiating reperfusion of the organ with blood. After reperfusion, 120 ml of 20% r-r mannitol (approximately 2.5 ml/kg body weight) was administered intravenously to increase diuresis. Also, 40 mg papaverine was administered around the arterial and venous anastomoses to improve blood flow through the organ. Subsequently, the process of anastomosing the ureter to the bladder wall was started. During the process, the ureter was catheterised with a double J stent (DJ) catheter. Once the anastomosis of the ureter was completed, placement of the bowel in the abdominal cavity proceeded. Peritoneal closure was then commenced. Immediately after peritoneal closure, 50 mg of furosemide was administered intravenously. The final process was to complete the closure of the abdominal shells and apply a dressing, thus completing the procedure. After the procedure, the animal was transported to an individual cage, where it underwent the recovery process.

**Stage 4** Postoperative observation of the animal was carried out in an individual cage adjacent to the cages of other pigs. Post-operative day (POD) counting started from the day of organ implantation as day '0'.

**Stage 5.** After a 14-day observation period, euthanasia was done in order to do dissection. The procedure included: general anaesthesia of the animal, opening the abdominal cavity, finding and dissecting the transplanted kidney, euthanasia of the animal.

**Perfusion of the kidney**

A pig kidney was harvested from a female pig weighing approximately 47 kg. **_WIT: 30 minutes_**. Kidney weight before perfusion: 200g. Kidney weight after perfusion: 211g (weight increase of 6%).

For the first 30 minutes of perfusion, the mean flow values (See Figs. 95 - 96.) were close to zero, and for the next 1.5 hours of perfusion they maintained at a similar level of 25 ml/min. From the second hour, the values started to increase significantly, stabilising around the fourth hour of perfusion at around 140 ml/min. Values began to decrease again from the 11th hour of perfusion. Changes in perfusion fluid flow are closely related to changes in fluid temperature and pressure pulsations. The fluid flow starts to increase after the second hour of perfusion, at the start of the temperature rise, which was completed after one hour.

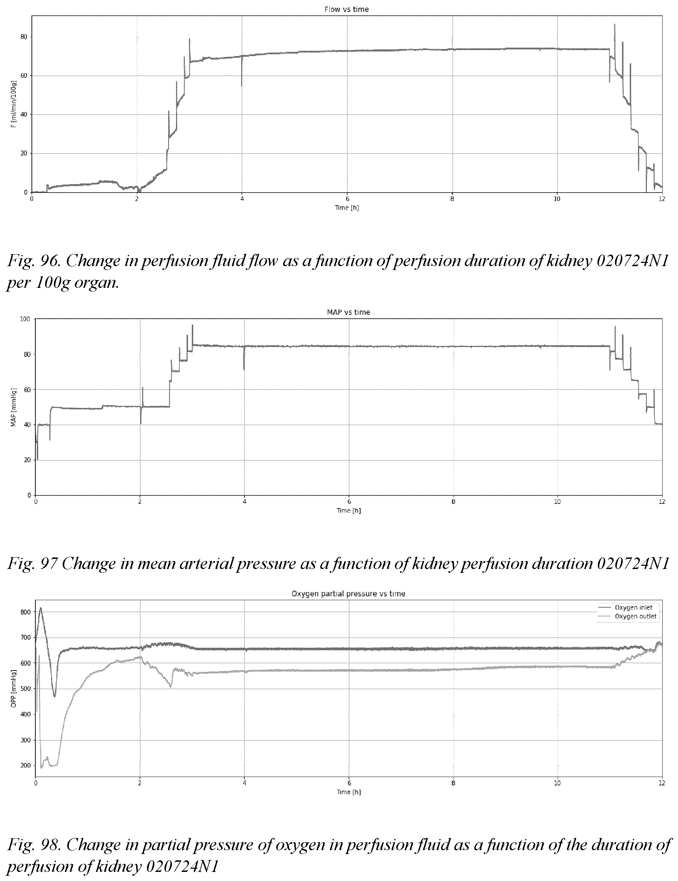

The mean arterial flow pressure (See Fig. 97.) increased from the start of perfusion, stabilising at around 50 mmHg after about 30 minutes. After about 2.5 hours, the pressure began to rise, reaching values between 80 and 90 mmHg. The pressure remained at this level until 11th hour, from which it began to gradually decrease, reaching a level of 40 mmHg.

The mean partial pressure of oxygen in the fluid (See Fig. 98.) is higher at the inlet than at the outlet of the organ, indicating oxygen consumption by the kidney.

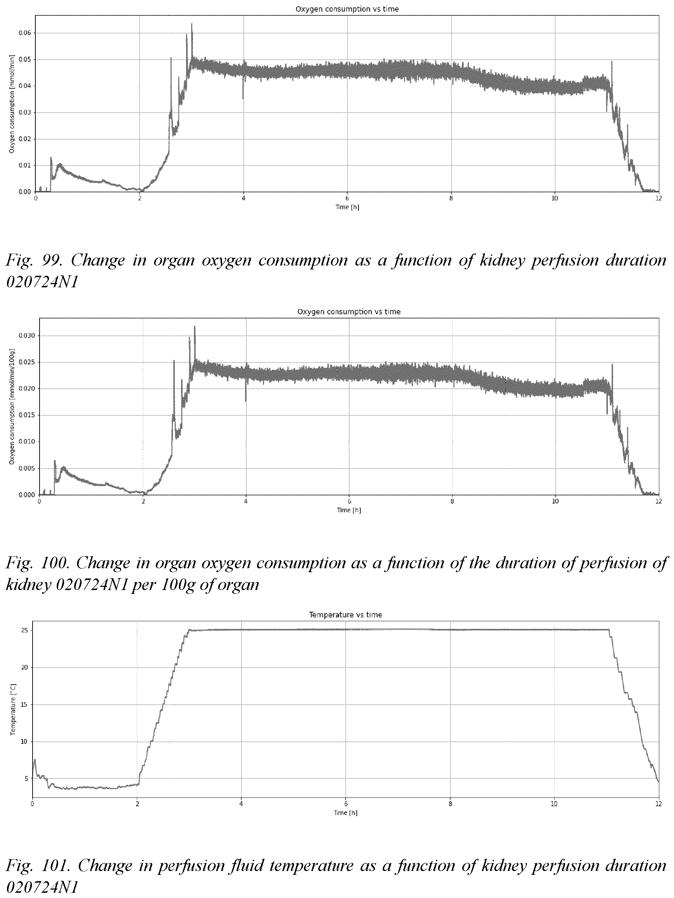

The average oxygen consumption of the organ (See Fig. 99 - 100.) began to increase significantly two hours after the start of perfusion, stabilising after a further hour. The increase in oxygen consumption is related to the warming of perfusion fluid. Temperature is reaching 25°C after 3 hours of perfusion, at which time oxygen consumption stabilised. A decrease in oxygen consumption occurred after 11 hours of perfusion, at the start of cooling perfusion fluid.

The temperature of the perfusion fluid (See Fig. 101.) is related to the perfusion protocol. For the first two hours, the process was conducted under hypothermic conditions with a temperature of about 4°C. In the second hour, heating was initiated, raising the fluid temperature about 15 degrees in 30 minutes. For the next half hour, the temperature was raised to 25 degrees, reaching subnormothermic conditions, maintained for a further eight hours. Cooling of the fluid began at the 11th hour of perfusion, reaching a temperature of about 5 St. Celsius after one hour.

The kidney started producing urine around the fourth hour of perfusion, after subnormothermic conditions had been reached.

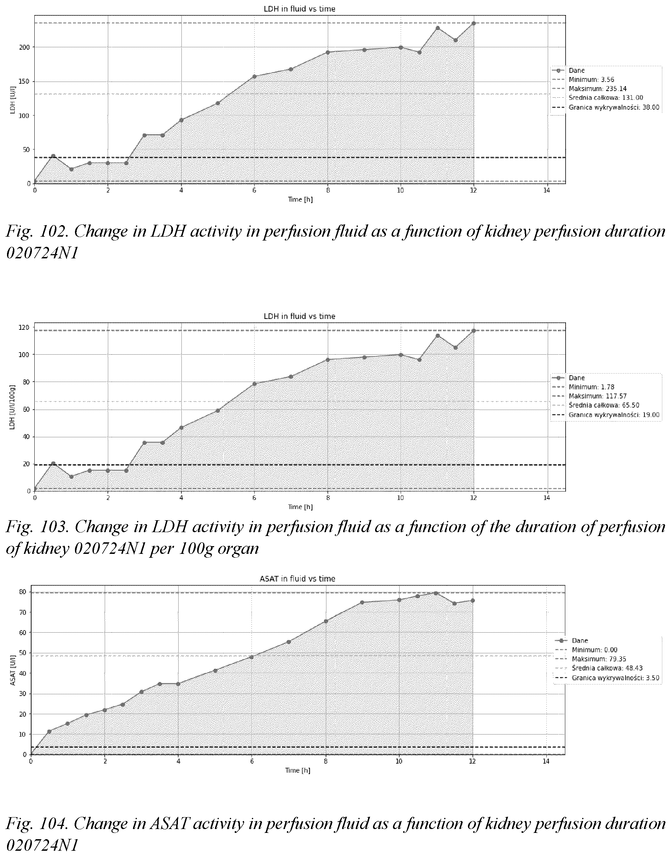

An increase in LDH activity can be observed (See Fig. 102- 103.) from the 4th to the 10th hour of perfusion. From the beginning to 2.5 hour of process, the values are below the detection limit. The maximum enzyme activity was measured at the 12th hour of perfusion. The values presented can be considered satisfactory and not indicative of severe cellular damage. At the same time, an increase in the activity of the enzyme during perfusion time is evident. In the second hour of perfusion, the temperature of the fluid began to rise. The switch from hypothermic to subnormothermic conditions was made. From the 11th hour of perfusion, the temperature was reduced to reach hypothermic conditions after one hour. During warming, an increase in enzyme activity is normal, as metabolic processes accelerate at higher temperatures. Highest enzyme activity was measured in hypothermia .

There is a noticeable increase in aspartate aminotransferase (ASAT) activity (see Fig. 104 - 105.) from the beginning of perfusion until its 10th hour. Maximum activity was measured at the 11th hour. All values of the enzyme activity, with the exception of that measured at the start of perfusion (point 0), are above the detection limit.

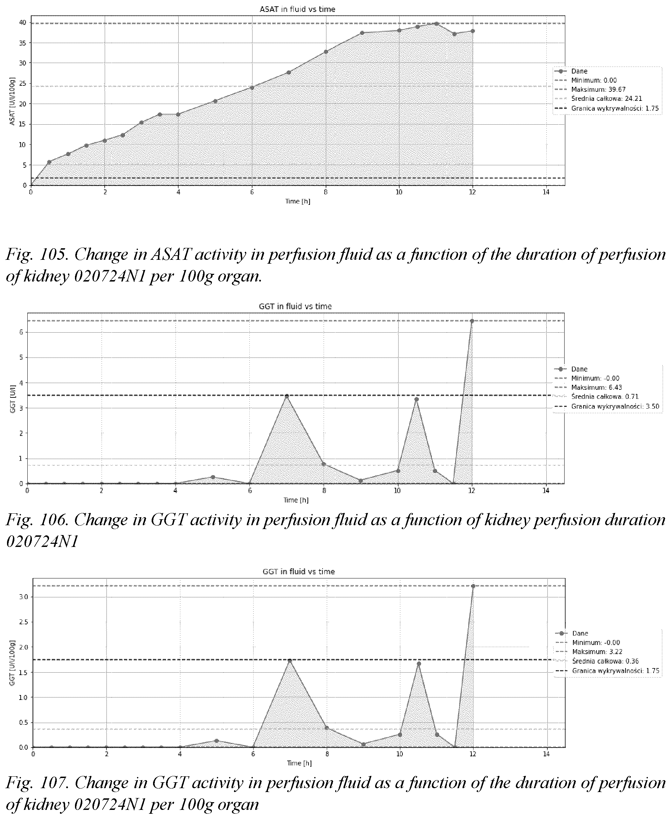

The change in gamma-glutamyltransferase (GGT) activity (See Figs. 106 - 107.) during perfusion time did not provide significant information on the state of the perfused organ. Only one value, measured at the 12th hour of perfusion, is above the detection limit.

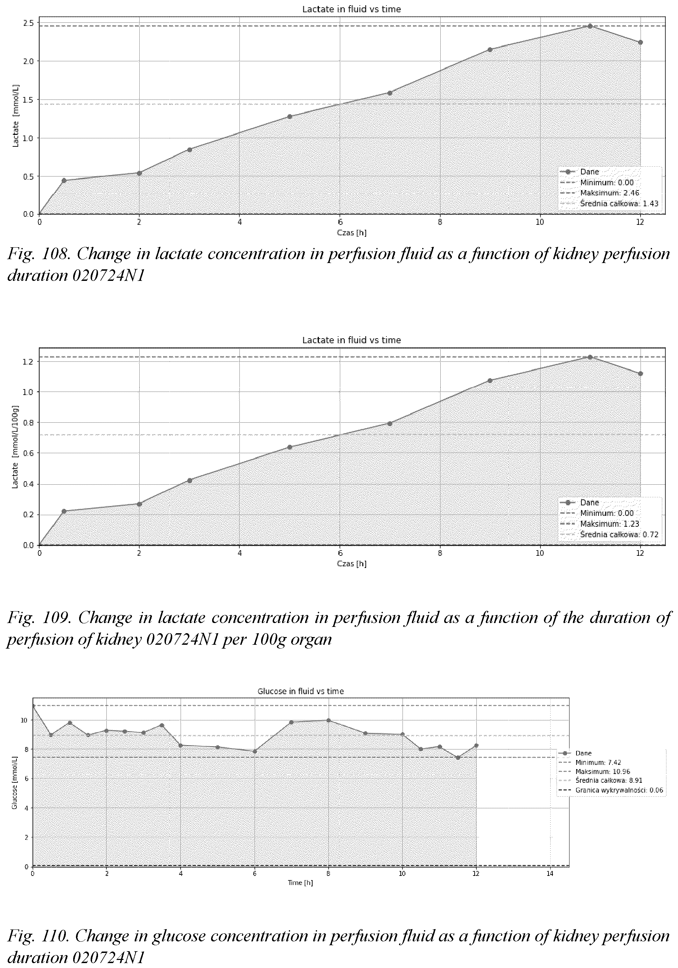

An increase in lactate concentration in the perfusion fluid can be seen (See Figs. 108 - 109.) with the duration of perfusion. The maximum concentration was measured at the 11th hour of perfusion, after 8 hours of subnormothermic perfusion, when cooling began.

The change in glucose concentration (See Fig. 110 - 111.) during the process is quite irregular. The high glucose level successively decreases from the start of the warming of the perfusion fluid (2nd hour), it rises relatively strongly at the end of the subnormothermic time. The organ has consumed glucose, which confirms the correctness of the metabolic processes running.

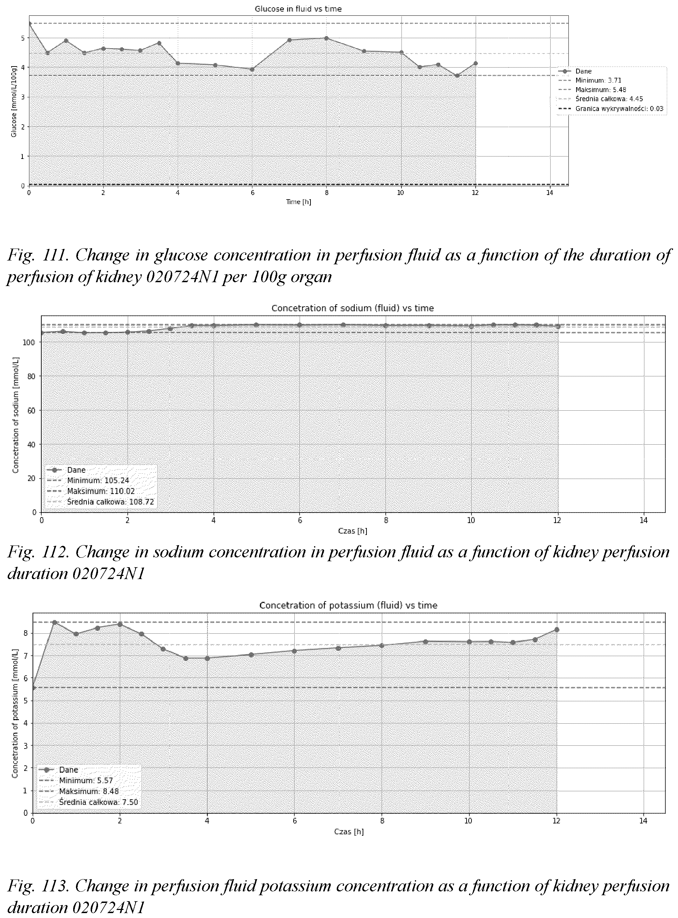

The sodium concentration in the perfusion fluid (See Fig. 112.) remains constant throughout the perfusion.

The concentration of potassium in the perfusion fluid (See Fig. 113.) varies from the start of perfusion until its 4th hour, from which it begins to rise gently. From 8th until 11th hour the values are at a similar level. For the last hour of perfusion a slight increase in concentration is noticeable. The maximum value was measured half an hour after the start of perfusion. Minimum value was measured at the start of perfusion, which means an increase from minimum to maximum in just 0.5 hours, at the same time the percentage increase is about 70%.

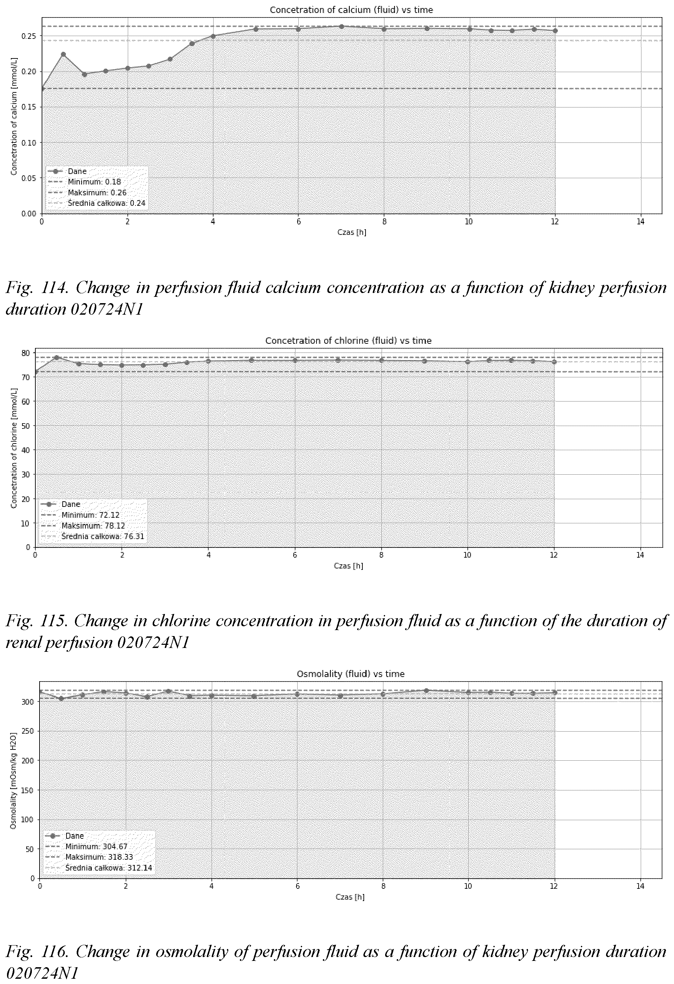

The calcium concentration in the perfusion fluid (See Fig. 114.) increases during the first 30 minutes of perfusion. Then the value falls slightly above the initial value and begins to gradually increase. Stabilization of values occur after five hours of perfusion and maintain until the end of perfusion.

The concentration of chlorine in the perfusion fluid (See Fig. 115.) remains constant throughout the perfusion and is not affected by time or fluid temperature. The difference between minimum and maximum value is low.

The osmolality of the perfusion fluid (See Fig. 116.) does not change significantly during the perfusion and remains at physiological levels. The difference between minimum and maximum value is low.

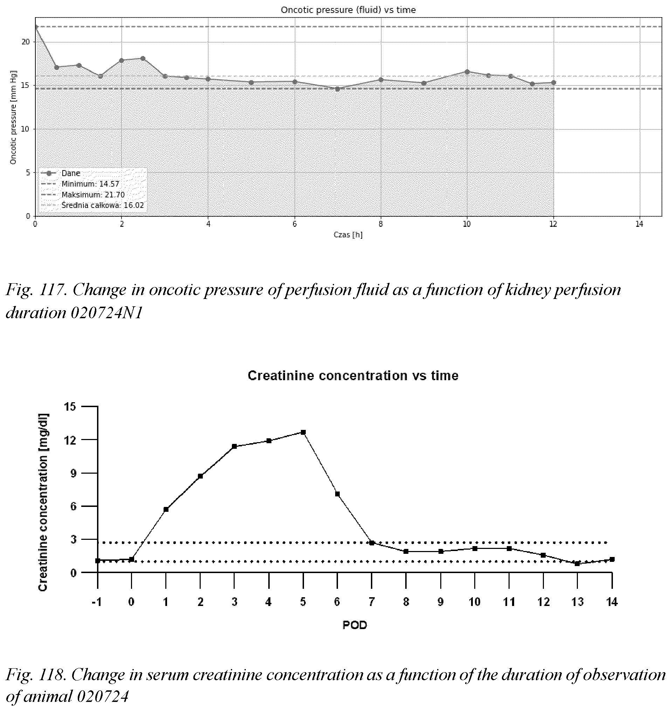

The oncotic pressure of the perfusion fluid (See Fig. 117.) fluctuates over time, reaching its highest value at the start of perfusion and its minimum value after 7 hours. From the results, there is no direct effect of temperature changes on the value of this parameter.

**2. Change in creatinine and blood urea nitrogen as a function of duration of postoperative observation**

During the observation, the change in the values of two basic biochemical parameters of organ functioning were measured daily: serum creatinine and urea nitrogen.

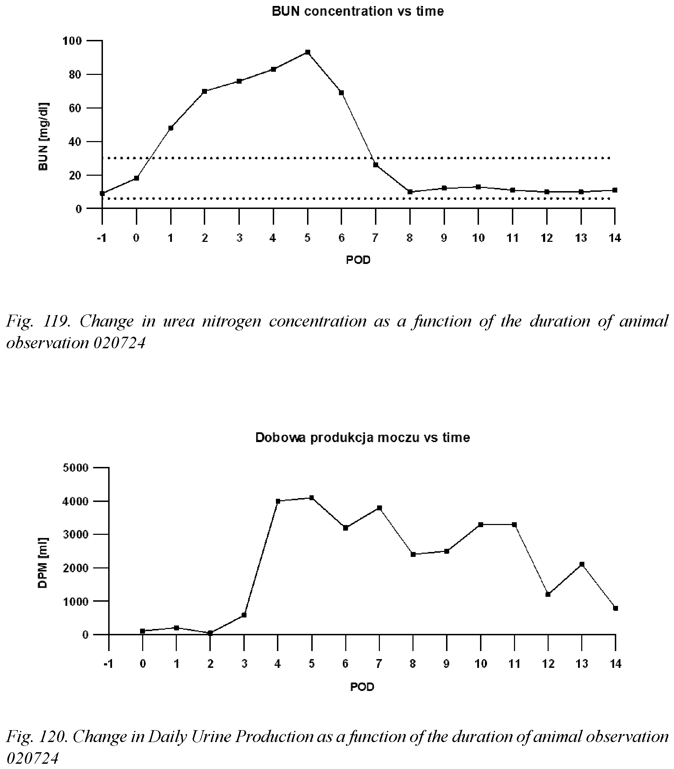

The maximum value of serum creatinine concentration was 12.7 mg/dl and was reached on the fifth postoperative day. The maximum value of urea nitrogen concentration was 93 mg/dl and was also reached on the fifth postoperative day. Both parameters (See Figs. 118 - 120.) returned to rangebound values on the eighth postoperative day. Diuresis was already observed immediately during the operation. It reached rangebound volume on the fourth postoperative day.

The animal survived the presumed observation period and the renal function parameters returned to within normal ranges during the observation period, which was considered a success of the test performed and confirmed the efficacy of perfusion with the perfusion fluid_02_II production method.

**Claims**

1.  A perfusion fluid in the form of an oil-in-water PFC nanoemulsion including:

    the disperse phase, which includes:

    - an oxygen carrier, preferably in an amount of 100.00-800.00 g/L, more preferably in an amount of 100-400 g/L, which is one or more compounds selected from:
    perfluorooctyl bromide, perfluorodecyl bromide; perfluoropentane, perfluorohexane, perfluoroheptane, perfluorotributylamine, perfluorodecalin perfluorooctane, perfluoromethylcyclohexylpiperidine, perfluorodichlorooctane, perfluorobutylcyclohexane, perfluoro-15-crown ether (PFCE), perfluorocyclohexane, preferably perfluorooctyl bromide, perfluorodecyl bromide; perfluoropentane, perfluorohexane, perfluoroheptane, perfluorotributylamine, perfluorodecalin

    the water phase, which includes:

- a surfactant which is either the main surfactant or at least one main surfactant and at least one cosurfactant, wherein:

a main surfactant, preferably in an amount of 23.80-40.08 g/L, more preferably in an amount of 23.80-34.00 g/L, which is Pluronic F68 (Kolliphor P188) or Pluronic F108 or bovine serum albumin (BSA) or human serum albumin (HSA) or recombinant human serum albumin or derivatives thereof, diblock/triblock/multiblock pluronics, such as, for example pluronics (condensation polymers of polypropylene glycols with polyoxyethylene glycols) and derivatives thereof, perfluorinated copolymers of the hydrocarbon-fluorocarbon type, gelatin and derivatives thereof, preferably Pluronic F68 (Kolliphor P188) or Pluronic F108 7 or bovine serum albumin (BSA) or human serum albumin (HSA) or recombinant human serum albumin or derivatives thereof

a cosurfactant, preferably in an amount of 0.35-0.60 g/L, more preferably in an amount of 0.35-0.50 g/L, which is: -perfluorinated: (1H, 1H, 2H, 2H-Perfluorooctyl)phosphocholine or (1H, 1H, 2H, 2H-Perfluorooctyl)-β-D-maltopyranoside or modified lipids: 1,2-dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or the sodium salt of N-(carbonyl-methoxy polyethyleneglycol-2000)-1,2-distearoyl-sn-glycero-3-phosphoethanolamine, perfluorinated phospholipids (e.g. phosphatidylethanolamines, phosphatidylserine, phosphatidylinositol, PEGylated phospholipids, sphingomyelins, glycerophosphocholine), perfluorinated glycolipids, preferably (1H, 1H, 2H, 2H-Perfluorooctyl)phosphocholine or (1H, 1H, 2H, 2H-Perfluorooctyl)-β-D-maltopyranoside or modified lipids: 1,2-dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or N-(carbonyl-methoxy polyethylene glycol-2000)-1,2-distearoyl-sn-glycero-3-phosphoethanolamine sodium salt

- an oncotic agent, preferably in an amount of 20.00-40.00 g/L, more preferably in an amount of 40.00 g/L, which is one oncotic agent selected from the group comprising of bovine serum albumin (BSA) or human serum albumin (HSA) or PEG 35 kDa or PEG 20 kDa or dextran 40 kDa or succinylated gelatin or hydroxyethylated starch or recombinant human albumin or derivatives thereof, dextran 30 kDa, dextran 50 kDa or derivatives thereof, gelatin derivatives, cyclodextrins, preferably bovine serum albumin (BSA) or human serum albumin (HSA) or PEG 35 kDa or PEG 20 kDa or dextran 40 kDa or succinylated gelatin or hydroxyethylated starch

- a main osmotic agent, preferably in an amount of 10.00-100.00 mM, most preferably in an amount of 21.40-50.00 nM, which is mannitol or raffinose or sucrose or glucose or citrate chelates or salts of lactobionic acid or gluconic acid, preferably mannitol,

- the main components of the buffer are one or more compounds selected from the

- D-glucose · $H_2O$ or fructose or sucrose or dextran, preferably D-glucose · $H_2O$ preferably in an amount of 5.00-20.00 mM, most preferably in an amount of 10.00 mM;
- sodium salt of DL-β-hydroxybutyric acid, preferably in an amount of 0.10-0.50 mM, most preferably in an amount of 0.20 mM,
- glutathione or vitamin C or vitamin E or deferoxamine or N-acetylcysteine or catalase or peroxidase, preferably glutathione, preferably in an amount of 1.50-6.00 mM, most preferably in an amount of 3.00 mM, and
- allopurinol or adenine, adenosine or phosphates or hormones: insulin, steroids i.e. dexamethasone, preferably allopurinol, preferably in an amount of 0.50-3.00 mM most preferably in an amount of 1.00 mM,

- buffer components constitute $Na^+$, $K^+$, $Mg^{2+}$, $Ca^{2+}$, $Cl^-$ salts, preferably selected from the group comprising chloride, sulphate, phosphate, lactobionate, citrate, gluconate, preferably containing $Na^+$, $K^+$, $Mg^{2+}$ $Ca^{2+}$, $Cl^-$ ions, preferably one or more of NaCl, preferably in an amount of 20,00-100.00 mM, most preferably in an amount of 60.00 mM, KCl, preferably in an amount of 2.00-10.00 mM, most preferably in an amount of 4.60 mM, $CaCl_2$ preferably in an amount of 0.30-2.00 mM, most preferably in an amount of 0.60 mM, $MgCl_2 \cdot 6H_2O$, preferably in an amount of 0.20-2.00 mM, most preferably in an amount of 0.40 mM;

- buffer components maintaining the correct pH, preferably one or more compounds selected from the group $Na_2HPO_4$, $NaH_2PO_4$, bicarbonate buffers consisting of a mixture of $NaHCO_3$ and $H_2CO_3$, phosphate buffers consisting of a mixture of $KH_2PO_4$ and $K_2HPO_4$, citrate buffers consisting of a mixture of $Na_3C_6H_5O_7 \cdot 2H_2O$ and $C_6H_8O_7 \cdot H_2O$, a histidine buffers consisting of a mixture of histidine-HCl and L-histidine, preferably $Na_2HPO_4$ and/or preferably $Na_2HPO_4$, preferably $Na_2HPO_4$ in an amount of 6.00-15.00 mM, most preferably in an amount of 8.00 mM and NaH$_2$PO$_4$ , preferably in an amount of 0.50-3.00 mM, most preferably in an amount of 1.5 mM;

wherein the perfusion fluid advantageously exhibits osmotic pressures in the range of: 280-400 mOsm/kgH$_2$O, pH in the range of 7.1-7.6, oncotic pressure in the range of 15-36 mmHg, concentration of the main ions: sodium in the range of 80-200 mM, potassium in the range of 1-15 mM, calcium in the range of 0.1-2.5 mM and chlorine in the range of 50-170 mM; furthermore, the average diameter of the dispersed phase nanoparticles does not exceed 300 nm, preferably is below 220 nm, most preferably is below 130 nm.

2. The perfusion fluid according to claim 1, **characterized in that** the aqueous phase further comprises amino acids including preferably one or more amino acids selected from the group of: taurine preferably in an amount of 5.00-15.00 mM, most preferably in an amount of 10.00 mM, L-arginine preferably in an amount of 0.05-0.20 mM, most preferably in an amount of 0.10 mM, L-tryptophan preferably in an amount of 1,00-3.00 mM, most preferably 2.00 mM, L-glutamine preferably in an amount of 0.10-1.00, most preferably in an amount of 0.50 mM, L-isoleucine preferably in an amount of 0.50-10.00 mM, most preferably in an amount of 1.52-4.19 mM, L-leucine preferably in an amount of 1.00-10.00 mM, most preferably in an amount of 3.05-6.86 mM, L-lysine preferably in an amount of 1.00-10.00 mM, most preferably in an amount of 2,05-4.79 mM, L-methionine preferably in an amount of 0.50-10.00 mM, most preferably in an amount of 1.34-3.35 mM, L-threonine preferably in an amount of 0.50-10.00 mM, most preferably in an amount of 1,69-3.78 mM, L-valine preferably in an amount of 0.50-10.00 mM, most preferably in an amount of 2.56-5.55, L-histidine preferably in an amount of 0.10-10.00 mM, most preferably in an amount of 0.64-2,26 mM, L-alanine preferably in an amount of 1.00-20.00 mM, most preferably in an amount of 5.61-12.35 mM, L-glycine preferably in an amount of 1.00-20.00 mM, most preferably in an amount of 6.66-17,32 mM, L-aspartic acid preferably in an amount of 0.50-10.00 mM most preferably in an amount of 1.88-4.51 mM, L-proline preferably in an amount of 0.50-10.00 mM most preferably in an amount of 2.17-5,21 mM, L-serine preferably in an amount of 0.10-10.00 mM, most preferably in an amount of 0.95-2.38 mM, L-tyrosine preferably in an amount of 0.01-1.00 mM, most preferably in an amount of 0.06-0.28 mM.

3. The perfusion fluid according to claim 1 or 2, **characterised in that** the oncotic agent is bovine/human albumin or succinylated gelatin, preferably bovine/human albumin, in an amount of: 20-40 g/L, most preferably in an amount of 40 g/L.

4. The perfusion fluid according to any of claims 1-3, **characterised in that** the buffer is mannitol, D-glucose · H$_2$O, glutathione, allopurinol, DL-β-hydroxybutyric acid sodium salt, L-arginine, taurine, L-tryptophan, L-glutamine, L-isoleucine, L-leucine, L-lysine, L-methionine, L-threonine, L-valine, L-histidine, L-alanine, L-glycine, L-aspartic acid, L-proline, L-serine, L-tyrosine, and Na$_2$HPO$_4$, NaH$_2$PO$_4$, NaCl, KCl, CaCl$_2$, MgCl$_2$ ·6H$_2$O and more preferably mannitol, D-glucose · H$_2$ O, glutathione, allopurinol, DL-β-hydroxybutyric acid sodium salt, L-arginine, taurine, L-tryptophan, L-glutamine, and Na$_2$HPO$_4$, NaH$_2$PO$_4$, NaCl, KCl, CaCl$_2$, MgCl$_2$ · 6H$_2$ O.

5. The perfusion fluid according to any of the claims 1-4, **characterised in that** it contains perfluorooctyl bromide (PFOB) in an amount of 187.21 g/L; perfluorodecyl bromide (PFDB) in an amount of 5.79 g/L; Pluronic F68 (Kolliphor P188) in an amount of 27.20 g/L; (1H, 1H, 2H, 2H-Perfluorooctyl)phosphocholine in an amount of 4.00 g/L; bovine albumin in an amount of 40.00 g/L; L-arginine in an amount of 0.10 mM; allopurinol in an amount of 1.00 mM; mannitol in an amount of 50.00 mM; taurine in an amount of 10.00 mM; L-tryptophan in an amount of 2.00 mM; D-glucose · H$_2$ O in an amount of 10.00 mM; NaCl in an amount of 60.00 mM; KCl in an amount of 4.60 mM; Na$_2$HPO$_4$ in an amount of 8.00 mM; NaH$_2$PO$_4$ in an amount of 1.50 mM; CaCl$_2$ in an amount of 0.60 mM; MgCl$_2$ · 6H$_2$ O in an amount of 0.40 mM; L-glutamine in an amount of 0.50 mM; glutathione in an amount of 3.00 mM; DL-β-hydroxybutyric acid sodium salt in an amount of 0.20 mM; NaOH to adjust a pH in the range of 6.8-7.4.

6. The perfusion fluid according to any of claims 1-4, **characterised in that** it contains perfluorooctyl bromide (PFOB) in an amount of 374.42 g/L; perfluorodecyl bromide (PFDB) in an amount of 11.58 g/L; Pluronic F68 (Kolliphor P188) in an amount of 34.00 g/L; (1H, 1H, 2H, 2H-Perfluorooctyl)phosphocholine in an amount of 5.00 g/L; bovine albumin in an amount of 40.00 g/L; L-arginine in an amount of 0.10 mM; allopurinol in an amount of 1.00 mM; mannitol in an amount of 21.40 mM; taurine in an amount of 10.00 mM; L-tryptophan in an amount of 2.00 mM; D-glucose · H$_2$ O in an amount of 10.00 mM; NaCl in an amount of 60.00 mM; KCl in an amount of 4.60 mM; Na$_2$HPO$_4$ in an amount of 8.00 mM; NaH$_2$PO$_4$ in an amount of 1.50 mM; CaCl$_2$ in an amount of 0.60 mM; MgCl$_2$ · 6H$_2$ O in an amount of 0.40 mM; L-glutamine in an amount of 0.50 mM; glutathione in an amount of 3.00 mM; DL-β-hydroxybutyric acid sodium salt in an amount of 0.20 mM; NaOH to adjust a pH in the range of 6.8-7.4.

7. A method for producing a perfusion fluid in the form of a PFC oil-in-water nonoemulsion, comprising the steps of

1. preparation of the components of the primary perfusion fluid including

1a. preparation of the solution of surfactant or surfactants,
1b. preparation of perfluorinated phase,
1c. preparation of the buffer

2. mechanical homogenization of the components prepared in step 1 to obtain the primary perfusion fluid;
3. microfluidization of the primary perfusion fluid obtained in step 2 to obtain the perfusion fluid, wherein:

in step 1a. the solution of surfactant or surfactants constitutes:

ultrapure water and
the main surfactant or at least one main surfactant and at least one cosurfactant wherein:
the main surfactant is Pluronic F68 (Kolliphor P188) or Pluronic F108 or bovine serum albumin (BSA) or human serum albumin (HSA) or recombinant human serum albumin or derivatives thereof, diblock/-triblock/multiblock pluronics, such as e.g. pluronics (condensation polymers of polypropylene glycols with polyoxyethylene glycols) and derivatives thereof, perfluorinated copolymers of the hydrocarbon-fluorocarbon type, gelatin and derivatives thereof, preferably Pluronic F68 (Kolliphor P188) or Pluronic F108 or bovine serum albumin (BSA) or human serum albumin (HSA) or recombinant human serum albumin or derivatives thereof
cosurfactant constitutes: (1H, 1H, 2H, 2H-Perfluorooctyl)phosphocholine or (1H, 1H, 2H, 2H-Perfluorooctyl)-β-D-maltopyranoside or modified lipids: 1,2-dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or the sodium salt of N-(carbonyl-methoxy polyethyleneglycol-2000)-1,2-distearoyl-sn-glycero-3-phosphoethanolamine, perfluorinated phospholipids (e.g. phosphatidylethanolamines, phosphatidylserine, phosphatidylinositol, PEGylated phospholipids, sphingomyelins, glycerophosphocholine), perfluorinated glycolipids, preferably (1H, 1H, 2H, 2H-Perfluorooctyl)phosphocholine or (1H, 1H, 2H, 2H-Perfluorooctyl)-β-D-maltopyranoside or modified lipids: 1,2-dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or N-(carbonyl-methoxy polyethylene glycol-2000)-1,2-distearoyl-sn-glycero-3-phosphoethanolamine sodium salt

in step 1b. the perfluorinated phase is one or more compounds selected from
perfluorooctyl bromide, perfluorodecyl bromide; perfluoropentane, perfluorohexane, perfluoroheptane, perfluorotributylamine, perfluorodecalin perfluorooctane, perfluoromethylcyclohexylpiperidine, perfluorodichlorooctane, perfluorobutylcyclohexane, perfluoro-15-crown ether (PFCE), perfluorocyclohexane, preferably perfluorooctyl bromide, perfluorodecyl bromide; perfluoropentane, perfluorohexane, perfluoroheptane, perfluorotributylamine, perfluorodecalin
in step 1c. the solid component of the buffer is:

a. an oncotic agent which is one oncotic agent selected from the group comprising of bovine serum albumin (BSA) or human serum albumin (HSA) or PEG 35 kDa or PEG 20 kDa or dextran 40 kDa or succinylated gelatin or hydroxyethylated starch or recombinant human albumin or derivatives thereof, dextran 30 kDa, , dextran 50 kDa, or derivatives thereof, gelatin derivatives, cyclodextrins, preferably bovine serum albumin (BSA) or human serum albumin (HSA) or PEG 35 kDa or PEG 20 kDa or dextran 40 kDa or succinylated gelatin or hydroxyethylated starch;
b. the main osmotic agent, which is mannitol or raffinose or sucrose or glucose or citrate chelates or salts of lactobionic acid or gluconic acid, preferably mannitol;
c. the main component of a buffer which is one or more compounds such as

- D-glucose · $H_2O$ or fructose or sucrose or dextran, preferably D-glucose · $H_2O$,
- DL-β-hydroxybutyric acid sodium salt,
- glutathione or vitamin C or vitamin E or deferoxamine or N-acetylcysteine or catalase or peroxidase, preferably glutathione,
- allopurinol or adenine or adenosine or phosphates or hormones preferably insulin or steroids preferably dexamethasone, most preferably allopurinol;

d. buffer components constitute $Na^+$, $K^+$, $Mg^{2+}$, $Ca^{2+}$, $Cl^-$ salts, preferably selected from the group consisting of chloride, sulphate, phosphate, lactobionate, citrate, gluconate, preferably containing $Na^+$, $K^+$, $Mg^{2+}$, $Ca^{2+}$, $Cl^-$ ions, preferably one or more, preferably all, of NaCl, KCl, $CaCl_2$, $MgCl_2 \cdot 6H_2O$;
e. buffering components maintaining the correct pH preferably one or more compounds selected from the group $Na_2HPO_4$, $NaH_2PO_4$, buffers such as bicarbonate consisting of a mixture of $NaHCO_3$ and $H_2CO_3$, phosphate consisting of a mixture of $KH_2PO_4$ and $K_2HPO_4$, citrate consisting of a mixture of $Na_3C_6H_5O_7 \cdot 2H_2O$ and $C_6H_8O_7 \cdot H_2O$, histidine consisting of a mixture of histidine-HCl and L-histidine, preferably $Na_2HPO_4$ and/or $Na_2HPO_4$
f. possibly other constituents that are amino acids including preferably one or more amino acids selected from the group taurine, L-arginine, L-tryptophan, L-glutamine, L-isoleucine, L-leucine, L-lysine, L-methionine, L-threonine, L-valine, L-histidine, L-alanine, L-glycine, L-aspartic acid, L-proline, L-serine, L-tyrosine.

8. The method according to claim 7, **characterised in that**

in **step 3,** the primary perfusion fluid obtained in step **(2)** is pumped at a pressure of 2065 bar through a polycrystalline diamond Y-type microfluidizer chamber with an inner diameter of 75 μm microchannels and cooled using a water bath so that the temperature of the fluid leaving the microfluidizer chamber does not exceed 30 °C; and/or
in **step 3,** the microfluidization of the primary perfusion fluid obtained in step **2** is carried out in cycles of 1-15 passes of the pre-emulsion obtained in step **2** through the microfluidizer chamber, preferably 3-9 passes.

9. A method for producing a perfusion fluid in the form of an oil-in-water PFC nonoemulsion, comprising the steps of

**1.** preparation of the initial perfusion fluid components including

**I.** preparation of the pre-emulsion components comprising

**Ia.** preparation of the solution of surfactant or surfactants
**Ib.** preparation of the perfluorinated phase

**II.** mechanical homogenization of the components prepared in step **I** to obtain a primary pre-emulsion
**III.** microfluidization of the primary pre-emulsion obtained in step **II** to obtain the pre-emulsion,
**IV.** the preparation of the buffer comprising

**IVa.** preparation of buffer components
**IVR.** pH adjustment
**IVQ.** quality control of the buffer obtained

**2.** mixing the pre-emulsion obtained in step **III** with the buffer obtained in step **IV** to obtain the primary perfusion fluid, wherein the components being preferably mixed in a ratio of pre-emulsion to buffer of 1: 1 to 1:2**;**
**3.** microfluidization of the primary perfusion fluid obtained in step **2** to obtain the perfusion fluid;
whereby:

in step **1a.** the solution of surfactant or surfactants constitutes:

ultrapure water and
the main surfactant or at least one main surfactant and at least one cosurfactant with:
the main surfactant is Pluronic F68 (Kolliphor P188) or Pluronic F108 or bovine serum albumin (BSA) or human serum albumin (HSA) or recombinant human serum albumin or derivatives thereof, diblock/-triblock/multiblock pluronics, such as e.g. pluronics (condensation polymers of polypropylene glycols with polyoxyethylene glycols) and derivatives thereof, perfluorinated copolymers of the hydrocarbon-fluorocarbon type, gelatin and derivatives thereof, preferably Pluronic F68 (Kolliphor P188) or Pluronic F108 or bovine serum albumin (BSA) or human serum albumin (HSA) or recombinant human serum albumin or derivatives thereof
cosurfactant constitutes: (1H, 1H, 2H, 2H-Perfluorooctyl)phosphocholine or (1H, 1H, 2H, 2H-Perfluorooctyl)-β-D-maltopyranoside or modified lipids: 1,2-dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or the sodium salt of N-(carbonyl-methoxy polyethyleneglycol-2000)-1,2-distearoyl-sn-glycero-3-phosphoethanolamine, perfluorinated phospholipids (e.g. phosphatidylethanolamines, phosphatidylserine, phosphatidylinositol, PEGylated phospholipids, sphingomyelins, glycerophosphocholine), perfluorinated glycolipids, preferably (1H, 1H, 2H, 2H-Perfluorooctyl)phosphocholine or (1H, 1H, 2H, 2H-Perfluorooctyl)-β-D-maltopyranoside or modified lipids: 1,2-dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or N-(carbonyl-methoxy polyethylene glycol-2000)-1,2-distearoyl-sn-glycero-3-phosphoethanolamine sodium salt

in step 1b. the perfluorinated phase constitutes of one or more compounds selected from perfluorooctyl bromide, perfluorodecyl bromide; perfluoropentane, perfluorohexane, perfluoroheptane, perfluorotributylamine, perfluorodecalin perfluorooctane, perfluoromethylcyclohexylpiperidine, perfluorodichlorooctane, perfluorobutyl cyclohexane, perfluoro-15-crown ether (PFCE), perfluorocyclohexane, preferably perfluorooctyl bromide, perfluorodecyl bromide; perfluoropentane, perfluorohexane, perfluoroheptane, perfluorotributylamine, perfluorodecalin
in step **1c.** the solid component of the buffer is:

a. an oncotic agent, which is one oncotic agent selected from the group comprising of bovine serum albumin (BSA) or human serum albumin (HSA) or PEG 35 kDa or PEG 20 kDa or dextran 40 kDa or succinylated gelatin or hydroxyethylated starch or recombinant human albumin or derivatives thereof, dextran 30 kDa, dextran 50 kDa or derivatives thereof, gelatin derivatives, cyclodextrins, preferably bovine serum albumin (BSA) or human serum albumin (HSA) or PEG 35 kDa or PEG 20 kDa or dextran 40 kDa or succinylated gelatin or hydroxyethylated starch;
b. the main osmotic agent, which is mannitol or raffinose or sucrose or glucose or citrate chelates or salts of lactobionic acid or gluconic acid, preferably mannitol;
c. the main component of the buffer, which is one or more compounds selected from the

- D-glucose · $H_2O$ or fructose or sucrose or dextran, preferably D-glucose · $H_2O$,
- DL-β-hydroxybutyric acid sodium salt,
- glutathione or vitamin C or vitamin E or deferoxamine or N-acetylcysteine or catalase or peroxidase, preferably glutathione, and
- allopurinol or adenine or adenosine or phosphates or hormones preferably insulin or steroids preferably dexamethasone, most preferably allopurinol;

d. buffer components constitute $Na^+$, $K^+$, $Mg^{2+}$, $Ca^{2+}$, $Cl^-$ salts, preferably selected from the group comprising of chloride, sulphate, phosphate, lactobionate, citrate, gluconate, preferably containing $Na^+$, $K^+$, $Mg^{2+}$, $Ca^{2+}$, $Cl^-$ ions, preferably one or more, preferably all, of NaCl, KCl, $CaCl_2$, $MgCl_2$ · $6H_2O$,;
e. buffer components that maintain the correct pH: preferably one or more compounds selected from the group $Na_2HPO_4$, $NaH_2PO_4$, buffers such as bicarbonate consisting of a mixture of $NaHCO_3$ and $H_2CO_3$, phosphate consisting of a mixture of $KH_2PO_4$ and $K_2HPO_4$, citrate consisting of a mixture of $Na_3C_6H_5O_7·2H_2O$ and $C_6H_8O_7·H_2O$, histidine consisting of a mixture of histidine-HCl and L-histidine, preferably $Na_2HPO_4$ and/or $Na_2HPO_4$
f. optionally other constituents that are amino acids including preferably one or more amino acids selected from the group of taurine, L-arginine, L-tryptophan, L-glutamine, L-isoleucine, L-leucine, L-lysine, L-methionine, L-threonine, L-valine, L-histidine, L-alanine, L-glycine, L-aspartic acid, L-proline, L-serine, L-tyrosine.

**10.** The method according to claim 9, **characterised in that**

in **stage III,** preferably the primary pre-emulsion obtained in stage **(II)** is pumped at a pressure of 2065 bar through a polycrystalline diamond Y-type microfluidizer chamber with an inner diameter of 75 μm microchannels and cooled by means of a water bath so that the temperature of the fluid leaving the microfluidizer chamber does not exceed 30 °C.
and in **step 3.** preferably the primary perfusion fluid obtained in step **(2)** is pumped at a pressure of 2065 bar through a polycrystalline diamond Y-type microfluidizer chamber with an inner diameter of 75 μm microchannels and cooled by means of a water bath so that the temperature of the fluid leaving the microfluidizer chamber does not exceed 30 °C;

and/or

in **stage III.** microfluidization of the primary pre-emulsion obtained in stage **II** to obtain the pre-emulsion is carried out in cycles of 1-10 passings of the PFC primary pre-emulsion through the microfluidizer chamber, most preferably 8 passings

wherein

in **step 3,** microfluidization of the primary perfusion fluid obtained in step **2** is carried out in cycles of 1-10 passes of the pre-emulsion obtained in step **2** through the microfluidizer chamber, most preferably 1-5 passes.

11. The method for producing according to any of claims 7-10, **characterised in that** the oncotic agent is bovine/human albumin or succinylated gelatin, and preferably bovine/human albumin, in an amount of: 20-40 g/L, preferably in an amount of 40 g/L.

12. The method for producing according to any of claims 7-11, **characterised in that** the buffer is mannitol, D-glucose $\cdot$ $H_2O$, glutathione, allopurinol, DL-$\beta$-hydroxybutyric acid sodium salt, L-arginine, taurine, L-tryptophan, L-glutamine, L-isoleucine, L-leucine, L-lysine, L-methionine, L-threonine, L-valine, L-histidine, L-alanine, L-glycine, L-aspartic acid, L-proline, L-serine, L-tyrosine, and $Na_2HPO_4$, $NaH_2PO_4$, NaCl, KCl, $CaCl_2$, $MgCl_2$ $\cdot$ $6H_2O$ and more preferably mannitol, D-glucose $\cdot$ $H_2O$, glutathione, allopurinol, DL-$\beta$-hydroxybutyric acid sodium salt, L-arginine, taurine, L-tryptophan, L-glutamine, and $Na_2HPO_4$, $NaH_2PO_4$, NaCl, KCl, $CaCl_2$ $MgCl_2$ $\cdot$ $6H_2O$.

13. The method for producing according to any of claims 7-12, **characterised in that** the primary surfactant is: Pluronic F68 (Kolliphor P188) or Pluronic F108 or Pluronic F127 or recombinant bovine/human albumin, in an amount of: 23.80-40.08 g/L, preferably 23.80-34.00 g/L, and the cosurfactant is: -perfluorinated: (1H, 1H, 2H, 2H-Perfluorooctyl) phosphocholine or (1H, 1H, 2H, 2H-Perfluorooctyl)-$\beta$-D-Maltopyranoside or modified lipid: 1,2-Dioleoyl-sn-glycero-3-phospho-L-serine sodium salt or 1,2-dipalmitoyl-sn-glycero-3-phosphate sodium salt or 1,2-dimyristoyl-sn-glycero-3-phospho-rac-glycerol sodium salt or N-(carbonyl-methoxy polyethylene glycol-2000)-1,2-distearoyl-sn-glycero-3-phosphoethanolamine sodium salt, in amounts: 0.35-0.60 g/L, preferably 0.35-0.50 g/L.

14. The method for producing according to any one of claims 7 to 13, **characterised in that** steps **1** to **3 of** the method are carried out under sterile/sterile conditions and/or step **3** shall be followed by step **4** involving sterilization of the perfusion fluid obtained in step 3

to obtain sterile perfusion fluid,

wherein sterilization of the perfusion fluid obtained in step **3** is carried out by sterilization filtration, gamma radiation, sterilization by ethylene oxide, preferably by sterilizing filtration,

alternatively, after steps **1-3** or after step **4,** packaging of the resulting sterile perfusion fluid is carried out.

15. The perfusion fluid obtained by the method according to any of claims 7-14, preferably having a qualitative and quantitative composition and physicochemical properties as disclosed in claims 1-6.

16. The perfusion fluid according to claims 1-6 and 15 for use in ex vivo machine perfusion of organs for transplantation, preferably donations of organs after brain death - DBD and donations taken from donors after irreversible cardiac death - DCD or donors aged 60 years and over and donors aged 50-59 years with at least two of the following risk factors: hypertension, death due to stroke, blood creatinine level at the time of death above 1.5 mg/dL - ECD, for better storage and reconditioning of the organ before transplantation.

17. The perfusion fluid according to claims 1-6 and 15 for use in ex vivo machine perfusion of organs, preferably including kidney, liver, heart.

18. The perfusion fluid according to claims 1-6 and 15 for use in ex vivo machine perfusion of organs, preferably including subnormothermic and normothermic conditions, in particular hybrid conditions containing periods of hypothermic perfusion, periods of controlled linear temperature increase or decrease and periods of subnormothermic or normothermic perfusion over the whole application range of 4-37 °C and a MAP range of 30-120 mmHg.

1. preparation of the initial perfusion fluid components
   1a. preparation of the surfactant(s) solution
   1b. preparation of the perfluorinated phase
   1c. preparation of the buffer

⇩

2. mechanical homogenization of the components prepared in step (1) to obtain the primary perfusion fluid

⇩

3. microfluidization of the primary perfusion fluid obtained in step (2) to obtain the perfusion fluid

⇩

4. (preferably) sterilizing filtration of the perfusion fluid obtained in step (3)

⇩

5. (preferably) confectioning the sterile perfusion fluid obtained in step (4)
Advantageously - 5Q. quality control of the packaged sterile perfusion fluid obtained in step (4)

*Fig. 1. Block diagram of perfusion fluid production - **Scheme I***

**1.** preparation of the primary perfusion fluid components

> **I.** Preparation of pre-emulsion ingredients
> **Ia.** preparation of the surfactant(s) solution
> **Ib.** preparation of perfluorinated phase

⇩

> **II.** Mechanical homogenization of the ingredients prepared in step **(I)** to obtain a primary pre-emulsion

⇩

> **III.** microfluidization of the primary pre-emulsion obtained in step **(II)** to obtain the pre-emulsion

> **IV.** preparation of the buffer
> **IVa.** preparation of buffer components
> **IVR.** pH adjustment
> **IVQ.** quality control of the buffer obtained

⇩

**2.** mixing the pre-emulsion obtained in step **(III)** with the buffer obtained in step **(IV)** to obtain the primary perfusion fluid
Beneficially **2R.** regulation of pH and osmolality and ion concentration

⇩

**3.** microfluidization of the primary perfusion fluid obtained in step **(2)** to obtain the perfusion fluid

⇩

**4.** (preferably) sterilizing filtration of the perfusion fluid obtained in step **(3)**

⇩

**5.** (preferably) packaging of the sterile perfusion fluid obtained in step **(4)**
Advantageously - **5Q**. Advantageous quality control of the packaged sterile perfusion fluid obtained in step **(4)**.

*Fig. 2. Block diagram of perfusion fluid production - **Scheme II***

*Fig. 3. Time diagram of SEVKP (subnormothermic ex vivo perfusion of the kidney)*

*Fig. 4. Time diagram of NEVKP (normothermic ex vivo perfusion of the kidney)*

*Fig. 5. Change in LDH activity in fluid per 100g of organ as a function of the duration of renal perfusion 260122N2*

Fig. 6. Change in urinary LDH activity per 100g organ as a function of kidney perfusion duration 260122N2 .

Fig. 7. Change in ASAT activity in fluid per 100g organ as a function of kidney perfusion duration 260122N2

Fig. 8. Change in urinary ASAT activity per 100g organ as a function of the duration of renal perfusion 260122N2

*Fig. 9. Change in ALAT activity in fluid per 100g organ as a function of the duration of renal perfusion 260122N2.*

*Fig. 10. Change in urinary ALAT activity per 100g organ as a function of the duration of renal perfusion 260122N2*

*Fig. 11. Change in fluid GGT activity per 100g organ as a function of kidney 260122N2 perfusion duration.*

Fig. 12. Change in urinary GGT activity per 100g organ as a function of kidney perfusion duration 260122N2

Fig. 13. Change in creatinine concentration in fluid per 100g organ as a function of kidney perfusion duration 260122N2

Fig. 14. Change in urinary creatinine concentration per 100g organ as a function of the duration of renal perfusion 260122N2

*Fig. 15. Averaged change in urine flow as a function of kidney 070223N2 perfusion time per 100 g organ.*

*Fig. 16. Change in LDH activity in perfusion fluid as a function of kidney 070223N2 perfusion time.*

*Fig. 17. Change in AST activity in perfusion fluid as a function of kidney 070223N2 perfusion time per 100 g organ.*

*Fig. 18. Change in creatinine concentration in perfusion fluid as a function of kidney 070223N2 perfusion time.*

*Fig. 19. Change in urinary creatinine concentration as a function of kidney 070223N2 perfusion time.*

*Fig. 20. Change in urinary LDH activity as a function of kidney 070223N2 perfusion time per 100 g organ.*

*Fig. 21. Change in urinary AST activity as a function of kidney 070223N2 perfusion time per 100 g organ.*

*Fig. 22. Change in urinary creatinine clearance as a function of renal perfusion duration 070223N2.*

*Fig. 23. Change in glucose concentration as a function of kidney 070223N2 perfusion time.*

*Fig. 24. Averaged (from 6 min) change in urine flow as a function of kidney perfusion time 270223N1 per 100g organ*

*Fig. 25. Change in LDH activity in fluid per 100g organ as a function of kidney perfusion duration 270223N1*

*Fig. 26. Change in AST activity in fluid per 100g organ as a function of kidney perfusion duration 270223N1*

*Fig. 27. Change in fluid GGT activity per 100g organ as a function of kidney perfusion duration 270223N1*

Fig. 28. Change in fluid creatinine concentration per 100g organ as a function of kidney perfusion duration 270223N1

Fig. 29. Change in urine creatinine concentration per 100g organ as a function of kidney

perfusion                                    duration                                    270223N1

Fig. 30. Change in urinary LDH activity per 100g organ as a function of kidney perfusion duration 270223N1.

Fig. 31. Change in urinary AST activity per 100g organ as a function of kidney perfusion duration 270223N1.

Fig. 32. Change in urinary GGT activity per 100g organ as a function of kidney perfusion duration 270223N1.

*Fig. 33. Change in creatinine clearance as a function of renal perfusion time 270223N1.*

*Fig. 34. Change in glucose concentration as a function of kidney perfusion time 270223N1.*

*Fig. 35. 6 min averaged change in urine flow as a function of kidney perfusion time 270524N1 per 100g organ*

Fig. 36. Change in LDH activity in perfusion fluid as a function of kidney perfusion duration 270524N1 per 100g organ

Fig. 37. Change in urinary LDH activity as a function of kidney perfusion time 270524N1 per 100g organ.

Fig. 38. Change in ASAT activity in perfusion fluid as a function of perfusion duration of kidney 270524N1 per 100g organ

Fig. 39. Change in urinary ASAT activity as a function of kidney 270524N1 perfusion time per 100g organ

Fig. 40. Change in GGT activity in perfusion fluid as a function of the duration of renal perfusion 270524N1 per 100g organ

Fig. 41. Change in urinary GGT activity as a function of the duration of renal perfusion 270524N1 per 100g organ

Fig. 42. Change in lactate concentration in perfusion fluid as a function of the duration of perfusion of kidney 270524N1 per 100g organ

Fig. 43. Change in glucose concentration in perfusion fluid as a function of kidney perfusion time 270524N1 per 100g organ

Fig. 44. Change in urine glucose concentration as a function of kidney perfusion time 270524N1 per 100g organ

*Fig. 45. Change in creatinine concentration in perfusion fluid as a function of kidney perfusion time 270524N1 per 100g organ*

*Fig. 46. Change in urine creatinine concentration as a function of kidney perfusion time 270524N1 per 100g organ*

*Fig. 47. Change in creatinine clearance as a function of kidney perfusion time 270524N1*

Fig. 48. 6 min averaged change in urine flow as a function of kidney 050624N1 perfusion time per 100g organ

Fig. 49. Change in LDH activity in perfusion fluid as a function of kidney 050624N1 perfusion time per 100g organ

Fig. 50. Change in urinary LDH activity as a function of kidney 050624N1 perfusion time per 100g organ

*Fig. 51. Change in ASAT activity in perfusion fluid as a function of kidney 050624N1 perfusion time per 100g organ*

*Fig. 52. Change in urinary ASAT activity as a function of kidney 050624N1 perfusion time per 100g organ*

*Fig. 53 Change in GGT activity in perfusion fluid as a function of kidney 050624N1 perfusion duration and per 100g organ*

Fig. 54. Change in urinary GGT activity as a function of kidney 050624N1 perfusion time per 100g organ

Fig. 55. Change in lactate concentration in perfusion fluid as a function of kidney 050624N1 perfusion time per 100g organ

Fig. 56. Change in glucose concentration in perfusion fluid as a function of kidney 050624N1 perfusion time per 100g organ

Fig. 57. Change in urine glucose concentration as a function of kidney 050624N1 perfusion time per 100g organ

Fig. 58. Change in creatinine concentration in perfusion fluid as a function of kidney 050624N1 perfusion time per 100g organ

Fig. 59. Change in urine creatinine concentration as a function of kidney 050624N1 perfusion time per 100g organ

Fig. 60. 6 min averaged change in urine flow as a function of kidney perfusion duration 080624N1 per 100g organ

Fig. 61. Change in LDH activity in perfusion fluid as a function of the duration of perfusion of kidney 080624N1 per 100g organ

Fig. 62. Change in urinary LDH activity as a function of kidney 080624N1 perfusion time per 100g organ

*Fig. 63. Change in ASAT activity in perfusion fluid as a function of the duration of perfusion of kidney 080624N1 per 100g organ*

*Fig. 64. Change in urinary ASAT activity as a function of kidney 080624N1 perfusion time per 100g organ*

*Fig. 65 Change in GGT activity in perfusion fluid as a function of the duration of perfusion of kidney 080624N1 per 100g organ.*

*Fig. 66. Change in urinary GGT activity as a function of the duration of perfusion of kidney 080624N1 per 100g organ*

*Fig. 67. Change in lactate concentration in perfusion fluid as a function of the duration of perfusion of kidney 080624N1 per 100g organ*

*Fig. 68. Change in glucose concentration in perfusion fluid as a function of the duration of perfusion of kidney 080624N1 per 100g organ*

*Fig. 69. Change in urine glucose concentration as a function of the duration of perfusion of kidney 080624N1 per 100g organ*

*Fig. 70. Change in creatinine concentration in perfusion fluid as a function of the duration of perfusion of kidney 080624N1 per 100g organ*

*Fig. 71. Change in urine creatinine concentration as a function of the duration of renal perfusion 080624N1 per 100g organ*

*Fig. 72. Change in LDH activity in fluid as a function of kidney perfusion time 030222N2 per 100g organ*

*Fig. 73. Change in ASAT activity in fluid as a function of kidney perfusion time 030222N2 per 100g organ*

*Fig. 74. Change in ALAT activity in fluid as a function of kidney perfusion time 030222N2 per 100g organ*

*Fig. 75. Change in fluid GGT activity per 100g organ as a function of kidney perfusion duration 030222N2*

*Fig. 76. Change in lactate concentration [mmol/l] in perfusion fluid as a function of kidney perfusion duration 030222N2*

*Fig. 77. Change in LDH activity in perfusion fluid as a function of perfusion duration of kidney 120624N1 per 100g organ*

*Fig. 78. Change in ASAT activity in perfusion fluid as a function of perfusion duration of kidney 120624N1 per 100g organ*

*Fig. 79. Change in GGT activity in perfusion fluid as a function of the duration of perfusion of kidney 120624N1 per 100g organ*

*Fig. 80. Change in lactate concentration in perfusion fluid as a function of the duration of renal perfusion 120624N1 per 100g organ*

*Fig. 81. Change in glucose concentration in perfusion fluid as a function of perfusion duration of kidney 120624N1 per 100g organ*

*Fig. 82. Change in LDH activity in perfusion fluid as a function of kidney 260624N1 perfusion time per 100g organ*

*Fig. 83. Change in ASAT activity in perfusion fluid as a function of the duration of perfusion of renal 260624N1 per 100g organ*

Fig. 84. Change in GGT activity in perfusion fluid as a function of the duration of perfusion of renal 260624N1 per 100g organ

Fig. 85. Change in lactate concentration in perfusion fluid as a function of the duration of renal perfusion 260624N1 per 100g organ

Fig. 86. Change in glucose concentration in perfusion fluid as a function of kidney 260624N1 perfusion time per 100g organ

Fig. 87. Averaged change in fluid lactate concentration as a function of liver perfusion duration 020823W1 per 100g organ.

*Fig. 88. Change in ALT activity in fluid as a function of liver perfusion time 020823W1 per 100g organ*

Fig. 89. Change in AST activity in fluid as a function of liver perfusion time 020823W1 per 100g organ

Fig. 90. Change in fluid GGT activity as a function of liver perfusion duration 020823W1 per 100g organ (no points from initial measurements due to negative result - measurement error).

Fig. 91. Change in fluid glucose concentration as a function of liver perfusion time 020823W1 per 100g organ

*Fig. 92. Change in LDH activity in the fluid as a function of heart 230823S1 perfusion time per 100 g organ.*

Fig. 93. Change in fluid glucose concentration as a function of heart 230823S1 perfusion time per 100 g organ.

Fig. 94. Change in lactate concentration as a function of cardiac perfusion time 230823S1 per 100 g organ.

*Fig. 95. Change in perfusion fluid flow as a function of perfusion duration in kidney 020724N1.*

*Fig. 96. Change in perfusion fluid flow as a function of perfusion duration of kidney 020724N1 per 100g organ.*

*Fig. 97 Change in mean arterial pressure as a function of kidney perfusion duration 020724N1*

*Fig. 98. Change in partial pressure of oxygen in perfusion fluid as a function of the duration of perfusion of kidney 020724N1*

Fig. 99. Change in organ oxygen consumption as a function of kidney perfusion duration 020724N1

Fig. 100. Change in organ oxygen consumption as a function of the duration of perfusion of kidney 020724N1 per 100g of organ

Fig. 101. Change in perfusion fluid temperature as a function of kidney perfusion duration 020724N1

Fig. 102. Change in LDH activity in perfusion fluid as a function of kidney perfusion duration 020724N1

Fig. 103. Change in LDH activity in perfusion fluid as a function of the duration of perfusion of kidney 020724N1 per 100g organ

Fig. 104. Change in ASAT activity in perfusion fluid as a function of kidney perfusion duration 020724N1

Fig. 105. Change in ASAT activity in perfusion fluid as a function of the duration of perfusion of kidney 020724N1 per 100g organ.

Fig. 106. Change in GGT activity in perfusion fluid as a function of kidney perfusion duration 020724N1

Fig. 107. Change in GGT activity in perfusion fluid as a function of the duration of perfusion of kidney 020724N1 per 100g organ

Fig. 108. Change in lactate concentration in perfusion fluid as a function of kidney perfusion duration 020724N1

Fig. 109. Change in lactate concentration in perfusion fluid as a function of the duration of perfusion of kidney 020724N1 per 100g organ

Fig. 110. Change in glucose concentration in perfusion fluid as a function of kidney perfusion duration 020724N1

*Fig. 111. Change in glucose concentration in perfusion fluid as a function of the duration of perfusion of kidney 020724N1 per 100g organ*

*Fig. 112. Change in sodium concentration in perfusion fluid as a function of kidney perfusion duration 020724N1*

*Fig. 113. Change in perfusion fluid potassium concentration as a function of kidney perfusion duration 020724N1*

*Fig. 114. Change in perfusion fluid calcium concentration as a function of kidney perfusion duration 020724N1*

*Fig. 115. Change in chlorine concentration in perfusion fluid as a function of the duration of renal perfusion 020724N1*

*Fig. 116. Change in osmolality of perfusion fluid as a function of kidney perfusion duration 020724N1*

*Fig. 117. Change in oncotic pressure of perfusion fluid as a function of kidney perfusion duration 020724N1*

*Fig. 118. Change in serum creatinine concentration as a function of the duration of observation of animal 020724*

**BUN concentration vs time**

Fig. 119. Change in urea nitrogen concentration as a function of the duration of animal observation 020724

**Dobowa produkcja moczu vs time**

Fig. 120. Change in Daily Urine Production as a function of the duration of animal observation 020724

Europäisches Patentamt

European Patent Office

Office européen des brevets

# EUROPEAN SEARCH REPORT

**Application Number**

EP 24 46 1635

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | FELKER T E ET AL: "A COMPARISON OF LIPOPROTEIN SECRETION, BILE PRODUCTION AND HEPATIC MORPHOLOGY IN ISOLATED RAT LIVERS PERFUSED WITH A PERFLUOROCARBON EMULSION OR RAT ERYTHROCYTES", HEPATOLOGY, 71ST ANNU MEET AM ASSOC STUDY LIVER DIS (AASLD) . 2020-11-13 / 2020-11-17 . VIRTUAL, N/A . ABST 215, vol. 14, no. 2, 1 August 1991 (1991-08-01), pages 340-351, XP000590872, ISSN: 0270-9139, DOI: 10.1016/0270-9139(91)91425-Z title; * abstract * * introduction; page 340 * * ch. "RESULTS"; page 342; table 1 * * page 343, column 2, last paragraph * * page 344; figure 2 * * footnote 5; page 349 * ----- | 1-18 | INV. A01N1/122 A01N1/126 A01P1/00 A61K9/00 A61K9/107 A61K47/26 A61K49/00 C12N5/00 |
| Y | WO 2007/139827 A2 (ALLIANCE PHAMACEUTICAL CORP [US]; WEERS JEFFREY [US] ET AL.) 6 December 2007 (2007-12-06) * claims 1-20 * * page 6, line 1 - line 11 * * page 8, line 9 - line 21 * * page 30, line 14 - page 31, line 20 * ----- -/-- | 1-18 | TECHNICAL FIELDS SEARCHED (IPC) A01N A61K A01P C12N |

The present search report has been drawn up for all claims

| Place of search | Date of completion of the search | Examiner |
|---|---|---|
| Munich | 2 June 2025 | Größl, Sylvester |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 93/02653 A1 (SEGEL LEIGH D [US]) 18 February 1993 (1993-02-18) * claims 1-22, 27 * * page 3, line 34 - page 4, line 2 * * page 7, line 9 - line 13 * * page 10, line 34 - line 37 * * page 10, line 4 - page 11, line 10; table 1 * ----- | 1-18 | |
| Y | WO 2015/138999 A1 (GEN HOSPITAL CORP [US]) 17 September 2015 (2015-09-17) * page 15, line 17 - line 30 * * page 16; tables 1-2 * * page 36, line 9 - line 30 * ----- | 1-18 | |
| Y | DIRKS B ET AL: "Fluorocarbon perfusion medium applied to the isolated rat brain", JOURNAL OF PHARMACOLOGICAL METHODS, ELSEVIER SCIENCE PUBLISHING, NEW YORK, NY, US, vol. 4, no. 2, 1 September 1980 (1980-09-01), pages 95-108, XP025522001, ISSN: 0160-5402, DOI: 10.1016/0160-5402(80)90029-7 [retrieved on 1980-09-01] title; * abstract * * ch. "Perfusion Media"; page 96 - page 97 * * page 105; table 3 * * ch. "DISCUSSION"; page 105 - page 106 * * page 106; table 4 * ----- -/-- | 1-18 | TECHNICAL FIELDS SEARCHED (IPC) |

The present search report has been drawn up for all claims

| Place of search | Date of completion of the search | Examiner |
|---|---|---|
| Munich | 2 June 2025 | Größl, Sylvester |

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | HAE WON KIM ET AL: "Artificial Oxygen Carriers as Red Blood Cell Substitutes: A Selected Review and Current Status", ARTIFICIAL ORGANS, BLACKWELL SCIENTIFIC PUBLICATIONS, INC., BOSTON, US, vol. 28, no. 9, 20 August 2004 (2004-08-20), pages 813-828, XP071482762, ISSN: 0160-564X, DOI: 10.1111/J.1525-1594.2004.07345.X title; * abstract * * ch. "Perfluorocarbon based oxygen carriers (PFBOCs)"; page 819; table 3 * ----- | 1-18 | |
| Y | EP 0 231 091 A1 (CHILDRENS HOSP MEDICAL CENTER [US]; HEMAGEN PFC [US]) 5 August 1987 (1987-08-05) * claims 1-2, 6-10, 14-16 * * page 9, line 17 - page 10, line 24 * * page 10 - page 18; examples 1-14 * ----- | 1-18 | TECHNICAL FIELDS SEARCHED (IPC) |

The present search report has been drawn up for all claims

| Place of search | Date of completion of the search | Examiner |
|---|---|---|
| Munich | 2 June 2025 | Größl, Sylvester |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 46 1635

This annex lists the patent family members relating to the patent documents cited in the above-mentioned European search report.
The members are as contained in the European Patent Office EDP file on
The European Patent Office is in no way liable for these particulars which are merely given for the purpose of information.

02-06-2025

| Patent document cited in search report | | | Publication date | Patent family member(s) | | | Publication date |
|---|---|---|---|---|---|---|---|
| WO | 2007139827 | A2 | 06-12-2007 | AU | 2007267997 | A1 | 06-12-2007 |
| | | | | CA | 2655140 | A1 | 06-12-2007 |
| | | | | CN | 101448485 | A | 03-06-2009 |
| | | | | EP | 2034959 | A2 | 18-03-2009 |
| | | | | HK | 1133185 | A1 | 19-03-2010 |
| | | | | JP | 5205369 | B2 | 05-06-2013 |
| | | | | JP | 2009538311 | A | 05-11-2009 |
| | | | | KR | 20090023620 | A | 05-03-2009 |
| | | | | US | 2010298445 | A1 | 25-11-2010 |
| | | | | WO | 2007139827 | A2 | 06-12-2007 |
| WO | 9302653 | A1 | 18-02-1993 | AU | 2441592 | A | 02-03-1993 |
| | | | | EP | 0663821 | A1 | 26-07-1995 |
| | | | | US | 5374624 | A | 20-12-1994 |
| | | | | WO | 9302653 | A1 | 18-02-1993 |
| WO | 2015138999 | A1 | 17-09-2015 | AU | 2015229085 | A1 | 29-09-2016 |
| | | | | CA | 2942714 | A1 | 17-09-2015 |
| | | | | CN | 106455541 | A | 22-02-2017 |
| | | | | CN | 117694335 | A | 15-03-2024 |
| | | | | EP | 3116311 | A1 | 18-01-2017 |
| | | | | ES | 2841142 | T3 | 07-07-2021 |
| | | | | JP | 6829078 | B2 | 10-02-2021 |
| | | | | JP | 7075446 | B2 | 25-05-2022 |
| | | | | JP | 2017512492 | A | 25-05-2017 |
| | | | | JP | 2020188780 | A | 26-11-2020 |
| | | | | US | 2017015963 | A1 | 19-01-2017 |
| | | | | US | 2023002710 | A1 | 05-01-2023 |
| | | | | US | 2024124813 | A1 | 18-04-2024 |
| | | | | WO | 2015138999 | A1 | 17-09-2015 |
| EP | 0231091 | A1 | 05-08-1987 | AU | 598226 | B2 | 21-06-1990 |
| | | | | CA | 1337969 | C | 23-01-1996 |
| | | | | CN | 87100492 | A | 19-08-1987 |
| | | | | DE | 3785054 | T2 | 08-07-1993 |
| | | | | EP | 0231091 | A1 | 05-08-1987 |
| | | | | ES | 2054658 | T3 | 16-08-1994 |
| | | | | IE | 59175 | B1 | 26-01-1994 |
| | | | | IL | 81356 | A | 16-09-1991 |
| | | | | JP | H0822815 | B2 | 06-03-1996 |
| | | | | JP | S62228010 | A | 06-10-1987 |
| | | | | KR | 870006923 | A | 13-08-1987 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2024046999 A1
- WO 200877641 A1
- US 11406722 B2
- KR 20230124148
- WO 2020209752 A1
- WO 201356246 A1
- CN 106176601 A
- WO 2010121082 A

- WO 2015147705 A
- WO 2007139827 A2
- WO 2007105978 A
- CN 1286081 A
- US 5684050 A
- US 4987154 A
- JP 94062402 B

**Non-patent literature cited in the description**

- *Statistics*, 2023, https://www.poltransplant.org.pl/statystyka_2023.html
- Committee on A Fairer and More Equitable, Cost-Effective, and Transparent System of Donor Organ Procurement, Allocation, and Distribution. Realizing the Promise of Equity in the Organ Transplantation System.. National Academies Press, 2022, vol. 26364
- **HIMMELFARB, J.** ; **VANHOLDER, R.** ; **MEHROTRA, R.** ; **TONELLI, M.** The current and future landscape of dialysis.. *Nat. Rev. Nephrol*, 2020, vol. 16, 573-585
- **RAO, P. S.** ; **OJO, A**. The Alphabet Soup of Kidney Transplantation: SCD, DCD, ECD-Fundamentals for the Practicing Nephrologist.. *Clin. J. Am. Soc. Nephrol.*, 2009, vol. 4, 1827
- **WESSON, L. G.** ; **COLBERG, J. E.,** ; **DEGUZMAN, A.** ; **ELSASSER, W.** ; **DUNN, S.** Extracellular fluid of the kidney preserved by the Collins technique.. *Transplantation*, 1979, vol. 27, 380-383

- **JOHANNES JÄGERS et al.** Artificial oxygen carriers in organ preservation: Dose dependency in a rat model of ex-vivo normothermic kidney perfusion. *Artificial Organs*, 2022, vol. 46, 1783-1793, https://onlinelibrary.wiley.com/doi/full/10.1111/aor.14264
- **HOSGOOD, SARAH A.** ; **NICHOLSON, MICHAEL L.** The Role of Perfluorocarbon in Organ Preservation. *Transplantation*, 2010, vol. 89 (10), 1169-1175, https://journals.lww.com/transplantjournal/fulltext/2010/0527 0/the_role_of_perfluorocarbon_in_organ_preservation.1.aspx
- **VON HORN C** ; **MINOR T.** Improved approach for normothermic machine perfusion of cold stored kidney grafts.. *Am J Transl Res*, 15 June 2018, vol. 10 (6), 1921-1929